(19) Europäisches Patentamt
European Patent Office
Office européen des brevets

(11) EP 0 632 329 B1

(12) EUROPÄISCHE PATENTSCHRIFT

(45) Veröffentlichungstag und Bekanntmachung des Hinweises auf die Patenterteilung:
**03.12.1997 Patentblatt 1997/49**

(51) Int Cl.[6]: **G03F 7/031**, C07C 271/20, C08F 2/50, C08B 37/16, C08F 8/30, G02B 1/04

(21) Anmeldenummer: **94810380.9**

(22) Anmeldetag: **24.06.1994**

(54) **Funktionalisierte Photoinitiatoren, Macromere daraus und deren Verwendung**

Functionalised photoinitiator, macromeres and their use

Photoinitiateur fonctionnalisé, macromères et leur usage

(84) Benannte Vertragsstaaten:
**AT BE CH DE DK ES FR GB GR IE IT LI LU NL PT SE**

(30) Priorität: **02.07.1993 CH 2006/93**

(43) Veröffentlichungstag der Anmeldung:
**04.01.1995 Patentblatt 1995/01**

(73) Patentinhaber: **Novartis AG
4058 Basel (CH)**

(72) Erfinder:
- **Chabrecek, Peter, Dr.
CH-4055 Basel (CH)**
- **Lohmann, Dieter, Dr.
CH-4142 Münchenstein (CH)**

(56) Entgegenhaltungen:
**EP-A- 0 281 941**

- **PATENT ABSTRACTS OF JAPAN vol. 013, no. 556 (C-664) 11. Dezember 1989 & JP-A-01 230 603 (MITSUBISHI RAYON CO LTD) 14. September 1989**
- **CHEMICAL ABSTRACTS, vol. 89, no. 22, 27. November 1978, Columbus, Ohio, US; abstract no. 180747, MASLYUK, A. F. ET AL 'Synthesis of oligourethanes capable of polymerization and properties of polyurethanes based on them'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 632 329 B1

Printed by Jouve, 75001 PARIS (FR)

## Beschreibung

Die vorliegende Erfindung betrifft mit organischen Diisocyanaten funktionalisierte Hydroxylgruppen enthaltende Acetophenone; Oligomere und Polymere, an die solche funktionalisierte Acetophenone gebunden sind; polymerisierbare Photoinitiatoren; über einen bifunktionellen Diisocyanat-Linker ethylenisch ungesättigte Acetophenone; sowie deren Verwendung als Photoinitiatoren; beschichtete Materialien; und die Verwendung der funktionalisierten Acetophenone zur Modifizierung von Oberflächen.

Die Verbindungen vom Alkylphenontyp oder Hydroxyalkylphenontyp mit dem Strukturelement der Formel (A)

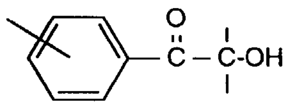

(A)

sind hervorragende Photoinitiatoren für die strahleninduzierte Polymerisation von ethylenisch ungesättigten monomeren, oligomeren oder polymeren Verbindungen. Als besonderer Nachteil werden in vielen Fällen eine Verfärbung (Vergilbung) der gebildeten Polymere und die toxischen Eigenschaften der entstehenden niedermolekularen Fragmente empfunden, die die Gebrauchseigenschaften der so hergestellten Polymere vermindern können. Zur Vermeidung dieses Nachteils und anderer Nachteile solcher monomerer Photoinitiatoren wird in der EP-A-0 281 941 vorgeschlagen, Photoinitiatoren am Phenylkern so zu modifizieren, dass die Photolyseprodukte im resultierenden Polymerverband fest eingebunden werden. Ganz allgemein werden für diesen Zweck auch Isocyanatgruppen als funktionelle Gruppen erwähnt, die über eine Spacergruppe, zum Beispiel eine lineare Alkylengruppe, an den Phenylkern gebunden ist. Die Herstellung solcher Verbindungen bereitet jedoch erhebliche synthetische Probleme, da bei der Umsetzung von linearen Diisocyanaten mit Hydroxylgruppen enthaltenden Verbindungen die Bildung von Diaddukten nicht vermieden werden kann und sogar überwiegt. Aus der JP(A) 1-230603 sind Umsetzungsprodukte eines Photoinitiators enthaltend ein Strukturelement der oben angegebenen Formel (A) mit zwei verschiedenen Diisocyanaten bekannt, die aufgrund der gewählten Herstellungsbedingungen nicht einheitlich sind, sondern jeweils ein Verbindungsgemisch darstellen, welches die Monoisocyanatoverbindung nur in geringem Umfang enthält. Chemical Abstracts 89:180747 (1994) offenbart oligomere Polyurethane, die durch Reaktion eines Polyalkylenglykols mit einem Gemisch aus (i) dem Umsetzungsprodukt von Benzoin und einem Diisocyanat und (ii) einem ethylenisch ungesättigten Isocyanat erhältlich sind.

Es besteht ein Bedarf an einfach herstellbaren funktionellen Photoinitiatoren mit Strukturelementen der Formel (A), die in hoher Reinheit erhältlich sind und sich durch eine hohe Reaktivität und Lagerstabilität auszeichnen, die an geeignete Oligomere oder Polymere zur Herstellung von makromeren Photoinitiatoren mit hohen Wirksamkeiten angelagert werden können, die zur Modifizierung von Oberflächen, besonders Kunststoffoberflächen, durch photoinduzierte Pfropfpolymerisationen geeignet sind, und die auch für biologisch verträgliche Materialien, insbesondere im biomedizinischen Bereich, wie z.B. für Kontaktlinsen, verwendet werden können. Es wurde gefunden, dass man dieses Ziel erreichen kann, wenn man zur Einführung von Isocyanatgruppen Diisocyanate verwendet, die unterschiedlich reaktive Isocyanatgruppen enthalten, mit im Phenylkern der Formel (A) gebundenen funktionellen Gruppen umsetzt, oder die Hydroxygruppe im Strukturelement (A) mit Diisocyanaten umsetzt, und so durch eine hohe Regioselektivität die Bildung von Isomeren und anderen Nebenprodukten unterdrückt.

Ein Gegenstand der Erfindung sind Verbindungen der Formel

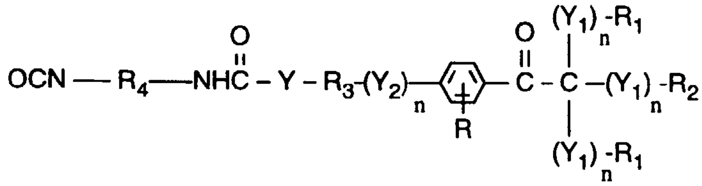

(I)

in reiner Form, worin Y O, NH oder $NR_6$ bedeutet; $Y_1$ O darstellt; $Y_2$ für -O-, -O-(O)C-, -C(O)-O- oder -O-C(O)-O- steht; die n unabhängig voneinander für 0 oder 1 stehen; R H, $C_1$-$C_{12}$-Alkyl, $C_1$-$C_{12}$-Alkoxy oder $C_1$-$C_{12}$-AlkylNH- darstellt; die $R_1$ und $R_2$ unabhängig voneinander H, lineares oder verzweigtes $C_1$-$C_8$-Alkyl, $C_1$-$C_8$-Hydroxyalkyl oder $C_6$-$C_{10}$-Aryl darstellen, oder zwei Gruppen $R_1$-$(Y_1)_n$- zusammen $-(CH_2)_x-$ bedeuten, oder die Gruppen $R_1$-$(Y_1)_n$- und $R_2$-$(Y_1)_n$- zusammen einen Rest der Formel

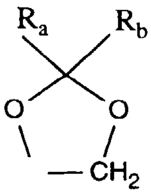

bilden; $R_3$ eine direkte Bindung oder lineares oder verzweigtes $C_1$-$C_8$-Alkylen darstellt, das unsubstituiert oder mit -OH substituiert ist und/oder gegebenenfalls mit ein oder mehreren Gruppen -O-, -O-C(O)- oder -O-C(O)-O- unterbrochen ist; $R_4$ verzweigtes $C_3$-$C_{18}$-Alkylen, mit $C_1$-$C_4$-Alkyl oder $C_1$-$C_4$-Alkoxy substituiertes $C_6$-$C_{10}$-Arylen, unsubstituiertes oder mit $C_1$-$C_4$-Alkyl oder $C_1$-$C_4$-Alkoxy substituiertes $C_7$-$C_{18}$-Aralkylen, mit $C_1$-$C_4$-Alkyl oder $C_1$-$C_4$-Alkoxy substituiertes $C_3$-$C_8$-Cycloalkylen, unsubstituiertes oder mit $C_1$-$C_4$-Alkyl oder $C_1$-$C_4$-Alkoxy substituiertes $C_3$-$C_8$-Cycloalkylen-$C_yH_{2y}$- oder mit $C_1$-$C_4$-Alkyl oder $C_1$-$C_4$-Alkoxy substituiertes -$C_yH_{2y}$-($C_3$-$C_8$-Cycloalkylen)-$C_yH_{2y}$-bedeutet; $R_6$ für lineares oder verzweigtes $C_1$-$C_6$-Alkyl steht; x ganze Zahlen von 3 bis 5 bedeutet; y ganze Zahlen von 1 bis 6 bedeutet; $R_a$ und $R_b$ unabhängig voneinander H, $C_1$-$C_8$-Alkyl, $C_3$-$C_8$-Cycloalkyl, Benzyl oder Phenyl darstellen; mit den Massgaben, dass n in den Gruppen -$(Y_1)_n$-$R_1$ für 0 steht, wenn $R_2$ H bedeutet; dass höchstens zwei $Y_1$ der -$(Y_1)_n$-Gruppen O bedeuten sowie n in den anderen -$(Y_1)_n$-Gruppen für 0 steht; dass n in der Gruppe -$(Y_2)_n$- für 0 steht, wenn $R_3$ eine direkte Bindung bedeutet; und das der Rest $R_4$ den beiden NCO-Gruppen eine unterschiedliche Reaktivität verleiht.

In einer bevorzugten Ausführungsform steht Y für O.

$R_6$ als Alkyl kann zum Beispiel Methyl, Ethyl, n- oder i-Propyl, n-, i- oder t-Butyl, Pentyl oder Hexyl sein. Bevorzugt stellt $R_6$ Methyl dar.

Die Gruppe R enthält als Alkyl, Alkoxy oder AlkylNH- bevorzugt 1 bis 6 und besonders bevorzugt 1 bis 4 C-Atome. Einige Beispiele sind Methyl, Ethyl, n- oder i-Propyl, n-, i-oder t-Butyl, Pentyl, Hexyl, Octyl, Decyl, Dodecyl, Methoxy, Ethoxy, Propoxy, Butoxy, und MethylNH-. Insbesondere bevorzugt steht R für H.

$R_1$ ist als Alkyl bevorzugt linear und enthält bevorzugt 1 bis 4 C-Atome. Einige Beispiele sind Methyl, Ethyl, n- oder i-Propyl, n-, i- oder t-Butyl, Pentyl, Hexyl, Heptyl und Octyl. Besonders bevorzugt handelt es sich bei $R_1$ um Methyl oder Ethyl. $R_1$ kann als Aryl zum Beispiel Naphthyl und besonders Phenyl bedeuten. Wenn beide Gruppen $R_1$-$(Y_1)_n$- für -$(CH_2)_x$- stehen, ist x bevorzugt 4 und besonders bevorzugt 5. $R_1$ ist als Hydroxyalkyl bevorzugt linear und enthält bevorzugt 1 bis 4 C-Atome. Einige Beispiele sind Hydroxymethyl und 2-Hydroxyeth-1-yl.

Für $R_2$ gelten die gleichen Bevorzugungen wie für $R_1$. Besonders bevorzugt steht $R_2$ für H, Methyl oder Ethyl.

$R_a$ und $R_b$ bedeuten bevorzugt unabhängig voneinander H oder $C_1$-$C_4$-Alkyl, zum Beispiel Methyl oder Ethyl.

In einer bevorzugten Untergruppe bedeutet $R_1$ bevorzugt Ethyl und besonders bevorzugt Methyl oder die beiden Gruppen $R_1$-$(Y_1)_n$- zusammen Pentamethylen, steht n in der Gruppe -$(Y_1)_n$-$R_2$ bevorzugt für 0, stellt $R_2$ bevorzugt Methyl, Hydroxmethyl oder H dar und steht R für H.

In einer anderen bevorzugten Ausführungsform stehen in der Gruppe -$(Y_1)_n$-$R_2$ $Y_1$ für O, n für 1 und $R_2$ für H. Insbesondere steht in diesem Fall n in den Gruppen $R_1$-$(Y_1)_n$- für 0.

$R_3$ enthält als Alkylen bevorzugt 1 bis 6 und besonders bevorzugt 1 bis 4 C-Atome und bevorzugt ist das Alkylen linear. Einige Beispiele sind Methylen, Ethylen, 1,2- oder 1,3-Propylen, 1,2-, 1,3- oder 1,4-Butylen, Pentylen, Hexylen, Heptylen und Octylen. Bevorzugt sind Methylen, Ethylen, 1,3-Propylen und 1,4-Butylen. Ganz besonders bevorzugt stellt $R_3$ Ethylen dar; oder eine direkte Bindung, wobei n in der Gruppe -$(Y_2)_n$- für 0 steht.

Bei $R_3$ als mit Hydroxy substituiertem Alkylen kann es sich zum Beispiel insbesondere um 2-Hydroxy-1,3-propylen oder auch um 2-Hydroxy-1,3- oder-1,4-butylen handeln. Mit -O- unterbrochenes und gegebenenfalls mit -OH substituiertes Alkylen ist zum Beispiel $-CH_2CH_2-O-CH_2CH_2-$, $-CH_2CH_2-O-CH_2CH_2-O-CH_2CH_2-$, $-CH_2CH_2-O-CH_2CH_2-O-CH_2CH_2-O-CH_2CH_2-$, $[-CH(CH_3)CH_2-O-CH(CH_3)CH_2-]$, $-CH(CH_3)CH_2-O-CH_2CH_2-$, $-CH(C_2H_5)CH_2-O-CH_2CH_2-$, $[-CH(C_2H_5)CH_2-O-CH(C_2H_5)CH_2-]$ oder $-CH_2CH_2CH_2CH_2-O-CH_2CH_2CH_2CH_2-$ und $-CH_2CH(OH)CH_2CH(OH)CH_2-O-CH_2CH_2-$. Mit -O-C(O)- oder -C(O)-O- unterbrochenes Alkylen ist zum Beispiel $-CH_2CH_2-C(O)-O-CH_2-$ oder $-CH_2CH_2-O-C(O)-CH_2-$. Mit -O-C(O)-O- unterbrochenes Alkylen ist zum Beispiel $-CH_2CH_2-O-C(O)-O-CH_2CH_2-$ oder $-CH_2CH_2-O-C(O)-O-CH_2-$.

Bei den Substituenten $C_1$-$C_4$-Alkyl und $C_1$-$C_4$-Alkoxy handelt es sich vorzugsweise um Methyl, Ethyl, Methoxy oder Ethoxy.

$R_4$ enthält als verzweigtes Alkylen bevorzugt 3 bis 14 und besonders bevorzugt 4 bis 10 C-Atome. Beispiele für Alkylen sind 1,2-Propylen, 2-Methyl- oder 2,2-Dimethyl- 1,3-propylen, 1,2-, 1,3- und 2,3-Butylen, 2-Methyl- oder 2,3-Dimethyl-1,4-butylen, 1,2-, 1,3-oder 1,4-Pentylen, 2-Methyl- oder 3-Methyl- oder 4-Methyl- oder 2,3-Dimethyl- oder 2,4-Dimethyl oder 3,4-Dimethyl- oder 2,3,4-Trimethyl oder 2,2,3-Trimethyl- 2,2,4-Trimethyl- oder 2,2,3,3-Tetramethyl- oder 2,2,3,4-Tetramethyl- 1,5-pentylen, 1,2-,1 1,3-, 1,4- oder 1,5-Hexylen, 2-Methyl- oder 3-Methyl oder 4-Methyl- oder 2,2-Dimethyl- oder 3,3-Dimethyl- oder 2,3-Dimethyl- oder 2,4-Dimethyl- oder 3,4-Dimethyl- oder 2,2,3-Trimethyl-oder 2,2,4-Trimethyl- oder 2,2,5-Trimethyl- oder 2,3,4-Trimethyl- oder 2,2,4,5-Tetramethyl-1,6-hexylen, 1,2-, 1,3-, 1,4- 1,5

oder 1,6-Heptylen, 2-Methyl- oder 3-Methyl- oder 4-Methyl- oder 5-Methyl- oder 2,2-Dimethyl- oder 3,3-Dimethyl- oder 2,3-Dimethyl- oder 2,4-Dimethyl- oder 3,4-Dimethyl- oder 2,2,3-Trimethyl- oder 2,2,4-Trimethyl- oder 2,2,5-Trimethyl- oder 2,2,6-Trimethyl- oder 2,3,4-Trimethyl- oder 2,4,5-Trimethyl- oder 2,4,6-Trimethyl- oder 2,2,4,5-Tetramethyl-1,7-heptylen, 1,2-, 1,3-, 1,4- 1,5- 1,6- oder 1,7-Octylen, 2-Methyl- oder 3-Methyl- oder 4-Methyl- oder 5-Methyl- oder 6-Methyl- oder 7-Methyl- oder 2,2-Dimethyl- oder 3,3-Dimethyl- oder 2,3-Dimethyl- oder 2,4-Dimethyl-oder 3,4-Dimethyl- oder 2,6-Dimethyl- oder 2,7-Dimethyl- oder 2,2,4-Trimethyl- oder 2,2,5-Trimethyl- oder 2,2,6-Trimethyl- oder 2,2,5,6-Tetramethyl- 1,8-Octylen, 1,2-, 1,3-, 1,4- 1,5- 1,6-, 1,7- oder 1,8-Nonylen, 2-Methyl- oder 3-Methyl- oder 4-Methyl- oder 5-Methyl- oder 6-Methyl- oder 7-Methyl- oder 8-Methyl oder 2,2-Dimethyl- oder 3,3-Dimethyl- oder 2,3-Dimethyl- oder 2,4-Dimethyl- oder 3,4-Dimethyl- oder 2,6-Dimethyl- oder 2,7-Dimethyl- oder 2,8-Dimethyl- oder 2,2,4-Trimethyl- oder 2,2,5-Trimethyl- oder 2,2,6-Trimethyl- oder 2,2,7-Trimethyl- oder 2,2,8-Trimethyl-nonylen, 1,2-, 1,3-, 1,4-1,5-1,6-, 1,7-, 1,8- oder 1,9-Decylen, 2-Methyl- oder 3-Methyl- oder 4-Methyl- oder 5-Methyl- oder 6-Methyl- oder 7-Methyl- oder 8-Methyl- oder 9-Methyl- oder 2,2-Dimethyl-oder 3,3-Dimethyl- oder 2,3-Dimethyl- oder 2,4-Dimethyl- oder 3,4-Dimethyl- oder 2,6-Dimethyl- oder 2,7-Dimethyl-, 2,8-Dimethyl- oder 2,9-Dimethyl-1,10-decylen, 1,2-, 1,3-, 1,4-1,5- 1,6-, 1,7-, 1,8-, 1,9- oder 1,10-Undecylen, 2-Methyl- oder 3-Methyl- oder 4-Methyl- oder 5-Methyl- oder 6-Methyl-oder 7-Methyl- oder 8-Methyl- oder 9-Methyl- oder 10-Methyl-1,11-undecylen, 1,4- 1,5- 1,6-, 1,7-, 1,8-, 1,9-, 1,10- oder 1,11-Dodecylen.

Einige bevorzugte verzweigte Alkylenreste sind 2,2-Dimethyl-1,4-butylen, 2,2-Dimethyl-1,5-pentylen, 2,2,3- oder 2,2,4-trimethyl-1,5-pentylen, 2,2-Dimethyl-1,6-hexylen, 2,2,3-oder 2,2,4- oder 2,2,5-Trimethyl-1,6-hexylen, 1,6-hexylen, 2,2-Dimethyl- 1,7-heptylen, 2,2,3- oder 2,2,4- oder 2,2,5- oder 2,2,6-Trimethyl-1,7-heptylen, 2,2-Dimethyl-1,8-octylen, 2,2,3- oder 2,2,4- oder 2,2,5- oder 2,2,6- oder 2,2,7-Trimethyl-1,8-octylen.

Wenn $R_4$ Arylen ist, handelt es sich bevorzugt um einen Naphthylenrest und besonders bevorzugt um einen Phenylenrest. Das Arylen ist substituiert, und vorzugsweise befindet sich ein Substituent in Orthostellung zu einer Isocyanatgruppe. Beispiele für substituiertes Arylen sind 1-Methyl-2,4-phenylen, 1,5-Dimethyl-2,4-phenylen, 1-Methoxy-2,4-phenylen und 1-Methyl-2,7-naphthylen.

$R_4$ als Aralkylen ist bevorzugt Naphthylalkylen und besonders bevorzugt Phenylalkylen. Die Alkylengruppe im Aralkylen enthält bevorzugt 1 bis 12, besonders bevorzugt 1 bis 6 und insbesondere bevorzugt 1 bis 4 C-Atome. Ganz besonders bevorzugt stellt die Alkylengruppe im Aralkylen Methylen oder Ethylen dar. Einige Beispiele sind 1,3- oder 1,4-Benzylen, Naphth-2-yl-7-methylen, 6-Methyl-1,3- oder 1,4-benzylen, 6-Methoxy-1,3-oder 1,4-benzylen.

Wenn $R_4$ Cycloalkylen ist, handelt es sich bevorzugt um $C_5$- oder $C_6$-Cycloalkylen, das mit Methyl substituiert ist. Einige Beispiele sind 4-Methyl-1,3-Cyclopentylen, 4-Methyl-1,3-Cyclohexylen, 4,4-Dimethyl-1,3-Cyclohexylen, 3-Methyl- oder 3,3-Dimethyl-1,4-Cyclohexylen, 3,5-Dimethyl-1,3-Cyclohexylen, 2,4-Dimethyl-1,4-Cyclohexylen.

Wenn $R_4$ Cycloalkylen-$C_yH_{2y}$- bedeutet, handelt es sich bevorzugt um Cyclopentylen-$C_yH_{2y}$- und besonders um Cyclohexylen-$C_yH_{2y}$-, das unsubstituiert oder mit vorzugsweise 1 bis 3 $C_1$-$C_4$-Alkyl, besonders bevorzugt Methyl, substituiert ist. In der Gruppe -$C_yH_{2y}$- steht y bevorzugt für ganze Zahlen von 1 bis 4. Bevorzugter stellt die Gruppe -$C_yH_{2y}$- Ethylen und besonders bevorzugt Methylen dar. Einige Beispiele sind Cyclopent-1-yl-3-methylen, 3-Methyl-cyclopent-1-yl-3-methylen, 3,4-Dimethylcyclopent- 1-yl-3-methylen, 3,4,4-Trimethyl-cyclopent-1-yl-3-methylen, Cyclohex-1-yl-3- oder -4-methylen, 3- oder 4- oder 5-Methyl-cyclohex-1-yl-3- oder -4-methylen, 3,4- oder 3,5-Dimethyl-cyclohex-1-yl-3- oder -4-methylen, 3,4,5- oder 3,4,4- oder 3,5,5-Trimethyl-cyclohex1-yl-3- oder -4-methylen.

Wenn $R_4$ -$C_yH_{2y}$-Cycloalkylen-$C_yH_{2y}$- bedeutet, handelt es sich bevorzugt um -$C_yH_{2y}$-Cyclopentylen-$C_yH_{2y}$- und besonders um -$C_yH_{2y}$-$C_yH_{2y}$-Cyclohexylen-$C_yH_{2y}$-, das mit vorzugsweise 1 bis 3 $C_1$-$C_4$-Alkyl, besonders bevorzugt Methyl, substituiert ist.

In der Gruppe -$C_yH_{2y}$- steht y bevorzugt für ganze Zahlen von 1 bis 4. Bevorzugter stellen die Gruppen -$C_yH_{2y}$- Ethylen und besonders bevorzugt Methylen dar. Einige Beispiele sind 3-Methyl-cyclopentan -1,3-dimethylen, 3,4-Dimethyl-cyclopentan-1,3-dimethylen, 3,4,4-Trimethyl-cyclopentan-1,3-dimethylen, 3- oder 4- oder 5-Methyl-cyclohexan-1,3-oder -1,4-dimethylen, 3,4- oder 3,5-Dimethyl-cyclohexan-1,3- oder -1,4-dimethylen, 3,4,5- oder 3,4,4- oder 3,5,5-Trimethyl-cyclohexan-1,3- oder -1,4-dimethylen.

Eine bevorzugte Untergruppe von Verbindungen der Formel I sind solche, worin in den Gruppen $R_1$-$(Y_1)_n$- n für 0 steht, Y, $Y_2$ und $Y_1$ in der Gruppe $R_2$-$(Y_1)_n$- je O bedeuten, n in der Gruppe $R_2$-$(Y_1)_n$- für 0 oder 1 steht, $R_1$ $C_1$-$C_4$-Alkyl oder Phenyl bedeuten oder die Gruppen $R_1$-$(Y_1)_n$- zusammen Tetramethylen oder Pentamethylen darstellen, $R_2$ $C_1$-$C_4$-Alkyl oder H darstellt, R Wasserstoff bedeutet, n in der Gruppe -$(Y_2)$-$_n$ für 0 oder 1 steht, $R_3$ lineares oder verzweigtes $C_2$-$C_4$-Alkylen darstellt oder eine direkte Bindung bedeutet, wobei n in der Gruppe -$(Y_2)$-$_n$ für 0 steht, $R_4$ verzweigtes $C_5$-$C_{10}$-Alkylen, mit 1 bis 3 Methylgruppen substituiertes Phenylen, Benzylen oder mit 1 bis 3 Methylgruppen substituiertes Benzylen, mit 1 bis 3 Methylgruppen substituiertes Cyclohexylen, Cyclohexyl-$C_yH_{2y}$- oder mit 1 bis 3 Methylgruppen substituiertes Cyclohexyl-$C_yH_{2y}$- oder -$C_yH_{2y}$-Cyclohexyl-$C_yH_{2y}$- bedeutet, und y für 1 oder 2 steht.

Eine besonders bevorzugte Untergruppe von Verbindungen der Formel I sind solche, worin in den Gruppen $R_1$-$(Y_1)_n$- und -$(Y_2)$-$_n$ n für 0 steht, Y, $Y_2$ und $Y_1$ in der Gruppe $R_2$-$(Y_1)_n$- je O bedeuten, n in der Gruppe $R_2$-$(Y_1)_n$- für 0 oder 1 steht, $R_1$ Methyl oder Phenyl bedeutet oder die Gruppen $R_1$-$(Y_1)_n$- zusammen Pentamethylen darstellen, $R_2$ Methyl oder H darstellt, R Wasserstoff bedeutet, n in der Gruppe -$(Y_2)$-$_n$ für 1 steht und $R_3$ Ethylen darstellt oder n in

der Gruppe $-(Y_2)-_n$ für 0 steht und $R_3$ eine direkte Bindung bedeutet, und $R_4$ verzweigtes $C_6$-$C_{10}$-Alkylen, mit 1 bis 3 Methylgruppen substituiertes Phenylen, Benzylen oder mit 1 bis 3 Methylgruppen substituiertes Benzylen, mit 1 bis 3 Methylgruppen substituiertes Cyclohexylen, Cyclohexyl-$CH_2$- oder mit 1 bis 3 Methylgruppen substituiertes Cyclohexyl-$CH_2$- bedeutet.

Bei der Gruppe $R_4$ handelt es sich insbesondere um solche, die die Reaktivität der OCN-Gruppe vermindern, was im wesentlichen durch eine sterische Hinderung an mindestens einem benachbarten C-Atom erreicht wird. Bevorzugt ist $R_4$ daher in $\alpha$- oder besonders $\beta$-Stellung zur OCN-Gruppe verzweigtes Alkylen, oder ein in mindestens einer $\alpha$-Stellung wie definiert substituierter cyclischer Kohlenwasserstoffrest.

Einige Beispiele für besonders bevorzugte Verbindungen sind

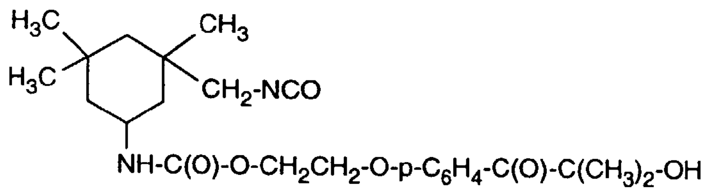

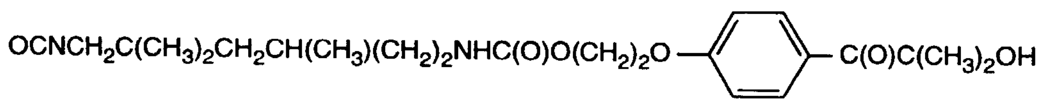

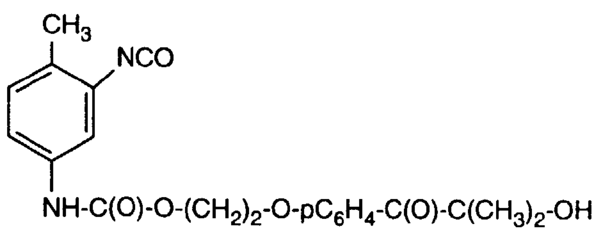

Die Verbindungen der Formel I können in an sich bekannter Weise durch die Umsetzung von Diisocyanaten mit den entsprechenden H-aciden Photoinitiatoren hergestellt werden. Die Verbindungen werden in hohen Ausbeuten und Reinheiten erhalten, selbst wenn im Photoinitiator gleichzeitig zwei verschieden reaktive H-acide Gruppen zugegen sind, zum Beispiel 2 OH-Gruppen. Besonders vorteilhaft ist es, Diisocyanate mit Isocyanatgruppen unterschiedlicher Reaktivität zu verwenden, weil hiermit die Bildung von Isomeren und Diaddukten weitgehend unterdrückt werden kann. Die unterschiedliche Reaktivität kann zum Beispiel wie zuvor beschrieben durch eine sterische Hinderung bewirkt werden. Die unterschiedliche Reaktivität kann auch durch eine Verkappung einer Isocyanatgruppe im Diisocyanat erzielt werden, zum Beispiel mit Carbonsäuren oder Hydroxylamin.

Ein weiterer Gegenstand der Erfindung ist ein Verfahren zur Herstellung der Verbindungen der Formel I, das dadurch gekennzeichnet ist, dass man eine Verbindung der Formel II

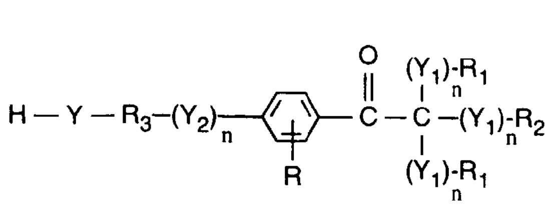

(II),

worin Y, $Y_1$, $Y_2$, R, $R_1$, $R_2$, $R_3$ und n die zuvor angegebenen Bedeutungen haben, in einem inerten organischen Lösungsmittel mit einem Diisocyanat der Formel III oder einem solchen gegebenenfalls monoverkappten Diisocyanat,

$$OCN-R_4-NCO \qquad (III),$$

worin $R_4$ die zuvor angegebenen Bedeutungen hat, bei einer Temperatur von bis zu 40°C, bevorzugt bei Raumtemperatur, umsetzt.

Verkappungsmittel sind aus der Urethanchemie bekannt. Es kann sich zum Beispiel um Phenole (Kresol, Xylenol), Lactame (ε-Caprolactam), Oxime (Acetoxim, Benzophenonoxim), H-aktive Methylenverbindungen (Diethylmalonat, Ethylacetoacetat), Pyrazole oder Benztriazole handeln. Verkappungsmittel sind zum Beispiel von Z. W. Wicks, Jr. in Pro-gress in Organic Coatings, 9 (1981), Seiten 3-28 beschrieben.

Die Verbindungen der Formel II sind bekannte Photoinitiatoren vom Hydroxyalkylphenontyp und in der Literatur beschrieben [siehe zum Beispiel H. F. Gruber, Prog. Polym. SCI., Vol. 17, Seiten 953 bis 1044 (1992), Pergamon Press Ltd.]. Bei den Isocyanaten handelt es sich um aus der Polyurethanchemie wohlbekannte Verbindungen.

Geeignete inerte Lösungsmittel sind aprotische vorzugsweise polare Lösungsmittel wie zum Beispiel Kohlenwasserstoffe (Petrolether, Methylcyclohexan, Benzol, Toluol, Xylol), Halogenkohlenwasserstoffe (Chloroform, Methylenchlorid, Trichlorethan, Tetrachlorethan, Chlorbenzol), Ether (Diethylether, Dibutylether, Ethylenglykoldimethylether, Diethylenglykoldimethylether, Tetrahydrofuran, Dioxan), Ketone (Aceton, Dibutylketon, Methyl-isobutylketon), Cabonsäureester und Lactone (Essigsäureethylester, Butyrolacton, Valerolacton), alkylierte Carbonsäureamide (N,N-Dimethylacetamid, N-Methylpyrrolidon), Nitile (Acetonitil), Sulfone und Sulfoxide (Dimethylsulfoxid, Tetramethylensulfon). Bevorzugt werden polare Lösungsmittel verwendet.

Die Reaktanden werden vorteilhaft in äquimolaren Mengen eingesetzt. Die Reaktionstemperatur kann zum Beispiel von 0 bis 200 °C betragen. Bei der Verwendung von Katalysatoren können die Temperaturen zweckmässig im Bereich von 0 bis 50 °C liegen, vorzugsweise bei Raumtemperatur. Geeignete Katalysatoren sind zum Beispiel Metallsalze wie Alkalimetallsalze von Carbonsäuren, tertiäre Amine, zum Beispiel ($C_1$-$C_6$-Alkyl)$_3$N (Triethylamin, Tri-n-butylamin), N-Methylpyrrolidin, N-Methylmorpholin, N,N-Dimethylpiperidin, Pyridin und 1,4-Diaza-bicyclooctan. Als besonders effektiv haben sich Zinnsalze erwiesen, besonders Alkylzinnsalze von Carbonsäuren wie zum Beispiel Dibutylzinndilaurat und Zinndioctoat. Sofern die Verbindungen der Formeln II und IIa mindestens zwei Hydroxylgruppen enthalten, wird die Reaktion aus Gründen der Selektivität zweckmässig bei Raumtemperatur durchgeführt.

Die Isolierung und Reinigung der hergestellten Verbindungen erfolgt nach bekannten Verfahren wie zum Beispiel Extraktion, Kristallisation, Umkristallisation oder chromatographische Reinigungsmethoden. Die Verbindungen werden in hohen Ausbeuten und Reinheiten erhalten. Die Ausbeuten bei nicht optimierten Verfahren können über 85 % der Theorie betragen.

Die Verbindungen der Formel I eignen sich hervorragend als Photoinitiatoren für ethylenisch ungesättigte radikalisch polymerisierbare Verbindungen, besonders solche, die zusätzlich H-aktive Gruppen wie zum Beispiel OH-, -COOH, -CONH- oder NH-Gruppen enthalten. In diesem Fall werden die Photoinitiatoren über die Isocyanatgruppe und die photochemischen Zerfallsprodukte (Radikalstarter beziehungsweise Kettenabbrecher) weitgehend an die gebildeten Polymeren kovalent gebunden und eine damit verbundene Verminderung der Gebrauchseigenschaften wirksam verhindert. Ein weiterer Gegenstand der Erfindung ist die Verwendung der Verbindungen der Formel I als Photoinitiatoren für ethylenisch ungesättigte radikalisch polymerisierbare Verbindungen, besonders solche, die zusätzlich H-aktive Gruppen enthalten.

Die Verbindungen der Formel I eignen sich auch hervorragend zur Herstellung von oligomeren oder polymeren Photoinitiatoren durch Umsetzung mit funktionellen Oligomeren oder Polymeren, die aktive H-Atome enthalten, zum Beispiel OH- oder NH-Gruppen. Diese makromeren Photoinitiatoren zeichnen sich durch eine gute Verträglichkeit und hohe Wirksamkeiten aus, wobei die photochemischen Zerfallsprodukte wie schon ausgeführt zum Beispiel als Kettenstarter oder -abbrecher kovalent in den gebildeten Polymeren eingebunden sind, so dass eine lange Gebrauchsdauer gewährleistet ist. Als weiterer Vorteil ist die besondere Struktur der Photopolymerisate anzugeben, da die Polymerketten auf dem makromeren Photoinitiator aufwachsen, wodurch sich weitere vorteilhafte Gebrauchseigenschaften ergeben. Mit der Wahl der Oligomeren beziehungsweise Polymeren lassen sich so gezielt im Photopolymer gewünschte Eigenschaften einstellen.

Ein weiterer Gegenstand der Erfindung sind Oligomere und Polymere mit an das Oligomer- oder Polymerrückgrat gegebenenfalls über eine Brückengruppe gebundenen H-aktiven Gruppen -OH und/oder -NH-, oder mit im Oligomer- oder Polymerrückgrat gebundenen H-aktiven -NH-Gruppen, deren H-Atome teilweise oder vollständig durch Reste der Formel IV

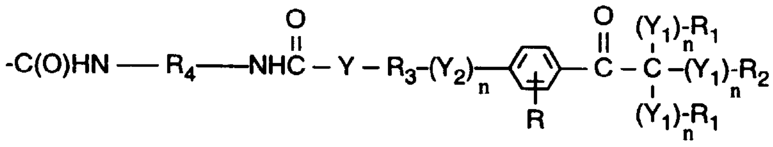 (IV),

substituiert sind, worin R, $R_1$, $R_2$, $R_3$, $R_4$, Y, $Y_1$, $Y_2$ und n die zuvor angegebenen Bedeutungen haben.

Bei den H-aktiven Gruppen handelt es sich vorzugsweise um -COOH, OH- oder -NH-Gruppen.

Die Oligomeren können ein mittleres Molekulargewicht von 300 bis 10 000 Dalton aufweisen und enthalten bevorzugt mindestens 3, bevorzugter 3 bis 50 und besonders bevorzugt 5 bis 20 Struktureinheiten. Der Übergang zwischen Oligomeren und Polymeren ist bekannterweise fliessend und nicht genau abzugrenzen. Die Polymeren können 50 bis 10 000, bevorzugter 50 bis 5 000 Struktureinheiten enthalten und ein mittleres Molekulargewicht von 10 000 bis 1 000 000, bevorzugt 10 000 bis 5000 000 aufweisen. Die Oligomeren und Polymeren können auch bis zu 95 Mol-%, bevorzugter 5 bis 90 Mol-% comonomere Struktureinheiten ohne H-aktive Gruppen enthalten, bezogen auf das Polymer.

Bei den Oligomeren und Polymeren mit H-aktiven Gruppen kann es sich um natürliche oder synthetische Oligomere oder Polymere handeln.

Natürliche Oligomere und Polymere sind zum Beispiel Oligo- und Polysaccharide oder deren Derivate, Proteine, Glycoproteine, Enzyme und Wachstumsfaktoren. Einige Beispiele sind Cyclodextrine, Stärke, Hyaluronsäure, deacetylierte Hyaluronsäure, Chitosan, Trehalose, Cellobiose, Maltotriose, Maltohexaose, Chitohexaose, Agarose, Chitin 50, Amylose, Glucane, Heparin, Xylan, Pectin, Galactan, Glycosaminoglycane, Dextran, aminiertes Dextran, Cellulose, Hydroxyalkylcellulosen, Carboxylalkylcellulosen, Heparin, Fucoidan, Chondroitinsulfat, sulfatierte Polysaccharide, Mucopolysaccharide, Gelatine, Zein, Collagen, Albumin, Globulin, Bilirubin, Ovalbumin, Keratin, Fibronectin und Vitronectin, Pepsin, Trypsin und Lysozyme.

Bei den synthetischen Oligomeren und Polymeren kann es sich um die Gruppen -COOH, -OH, $-NH_2$ oder $-NHR_7$ enthaltende Substanzen handeln, wobei $R_7$ $C_1$-$C_6$-Alkyl bedeutet. Es kann sich zum Beispiel um verseifte Polymerisate von Vinylestern oder Ethern (Polyvinylalkohol) hydroxylierte Polydiolefine wie z.B. Polybutadien, Polyisopren oder Chloropren; Polyacrylsäure und Polymethacrylsäure sowie Polyacrylate, Polymethacrylate, Polyacrylamide oder Polymethacrylamide mit Hydroxyalkyl- oder Aminoalkylresten in der Estergruppe oder Amidgruppe; Polysiloxane mit Hydroxyalkyl- oder Aminoalkylgruppen; Polyether aus Epoxiden oder Glycidylverbindungen und Diolen; Polyvinylphenole oder Copolymere von Vinylphenol und olefinischen Comonomeren; sowie Copolymerisate von mindestens einem Monomer aus der Gruppe Vinylalkohol, Vinylpyrrolidon, Acrylsäure, Methacrylsäure, oder Hydroxyalkyl oder Aminoalkyl enthaltenden Acrylaten, Methacrylaten, oder Acrylamid oder Methacrylamid, oder hydroxylierten Dioloefinen mit ethylenisch ungesättigten Comonomeren wie z.B. Acrylnitril, Olefinen, Diolefinen, Vinylchlorid, Vinylidenchlorid, Vinylfluorid, Vinylidenfluorid, Styrol, $\alpha$-Methylstyrol, Vinylethern und Vinylestern; Polyoxaalkylene mit endständigen OH- oder Aminoalkyloxygruppen handeln.

Bevorzugte Oligomere und Polymere sind zum Beispiel Cyclodextrine mit insgesamt 6 bis 8 einen Ring bildenden Glucosestruktureinheiten oder Hydroxyalkyl- oder Aminoalkylderivaten oder Glucose- oder Maltose-substituierten Derivaten, wovon mindestens eine Struktureinheit der Formel XVI entspricht,

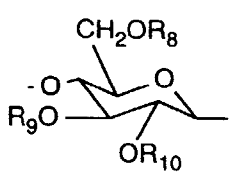 (XVI),

worin $R_8$, $R_9$ und $R_{10}$ unabhängig voneinander H, $C_1$-$C_4$-Alkyl, besonders Methyl, $C_2$-$C_6$-Acyl, besonders Acetyl, $C_1$-$C_4$-Hydroxyalkyl, besonders Hydroxymethyl oder 2-Hydroxyeth-1-yl, $C_2$-$C_{10}$-Aminoalkyl und besonders $C_2$-$C_4$-Aminoalkyl, zum Beispiel 2-Aminoeth-1-yl oder 3-Aminoprop-yl oder 4-Aminobut-1-yl bedeuten, und mindestens einer der Reste $R_8$, $R_9$ und $R_{10}$ einen Rest der Formel V

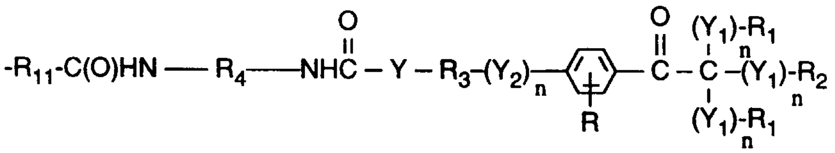

(V),

darstellen, worin R, $R_1$, $R_2$, $R_3$, $R_4$, Y, $Y_1$, $Y_2$ und n die zuvor angegebenen Bedeutungen haben, und $R_{11}$ eine direkte Bindung, -($C_1$-$C_4$-Alkylen-O)- oder -($C_2$-$C_{10}$-Alkylen-NH)-darstellt.

In einer bevorzugten Ausführungsform enthalten mindestens die Hälfte der Glucoseeinheiten bis alle 6 bis 8 der Glucoseeinheiten mindestens einen Rest der Formel V. Für R, $R_1$, $R_2$, $R_3$, $R_4$, Y, $Y_1$, $Y_2$ und n gelten die zuvor angegebenen Bevorzugungen. $R_{11}$ steht bevorzugt für eine direkte Bindung, $-CH_2-O-$, $-CH_2CH_2-O-$, $-CH_2CH_2-NH-$ oder $-CH_2CH_2CH_2-NH-$.

Andere bevorzugte Oligomere und Polymere sind zum Beispiel Oligo- und Polysiloxane mit OH- oder $NH_2$-Gruppen in Alkylendgruppen oder -seitenketten, deren H-Atome mit einem erfindungsgemässen Photoinitiator substituiert sind. Es kann sich um statistische oder Blockoligomere oder Blockpolymere handeln. Bevorzugtere Oligomere und Polymere sind solche, die

a) 5 bis 100 Mol-% Strukturelemente der Formel VI

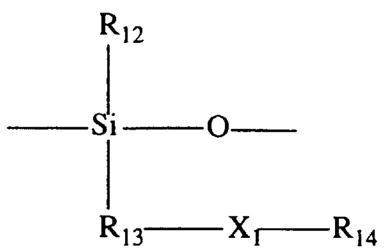

(VI),

und

b) 95 bis 0 Mol-% Strukturelemente der Formel VIa

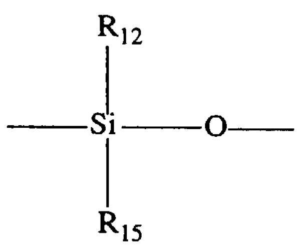

(VIa)

enthalten, bezogen auf das Oligomer oder Polymer, worin $R_{12}$ gegebenenfalls teilweise oder vollständig mit F substituiertes $C_1$-$C_4$-Alkyl, Vinyl, Allyl oder Phenyl, bevorzugt Methyl oder Trifluormethyl darstellt, $R_{13}$ $C_2$-$C_6$-Alkylen, bevorzugt 1,3-Propylen bedeutet, $R_{15}$ die Bedeutung von $R_{12}$ hat oder $-R_{13}-X_1-H$ oder $-R_{13}-X_1-R_{16}-H$ darstellt, $X_1$ für -O- oder -NH- steht, und $R_{14}$ für einen Rest der Formel VII steht,

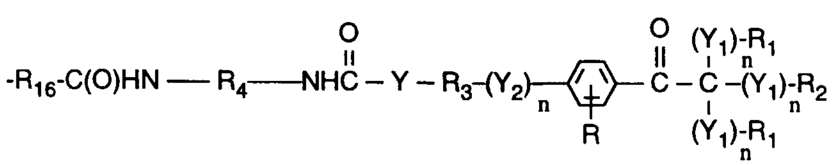

(VII),

worin R, $R_1$, $R_2$, $R_3$, $R_4$, Y, $Y_1$, $Y_2$ und n die zuvor angegebenen Bedeutungen haben, und $R_{16}$ eine direkte Bindung oder eine Gruppe $-C(O)-(CHOH)_r-CH_2-O-$ darstellt, worin r für 0 oder eine ganze Zahl von 1 bis 4 steht. Für R, $R_1$, $R_2$, $R_3$, $R_4$, Y, $Y_1$, $Y_2$ und n gelten die zuvor angegebenen Bevorzugungen. $X_1$ steht bevorzugt für -NH-.

Bevorzugte oligomere oder polymere Siloxane sind auch solche der Formel VIII

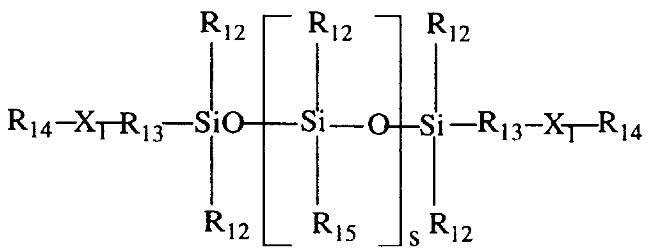 (VIII),

worin $R_{12}$ gegebenenfalls teilweise oder vollständig mit F substituiertes $C_1$-$C_4$-Alkyl, Vinyl, Allyl oder Phenyl, bevorzugt Methyl darstellt, $R_{13}$ $C_2$-$C_6$-Alkylen, bevorzugt 1,3-Propylen bedeutet, $R_{15}$ die Bedeutung von $R_{12}$ hat oder $-R_{13}-X_1-H$ oder $-R_{13}-X_1-R_{16}-H$ darstellt, $X_1$ für -O- oder -NH- steht, und $R_{14}$ für einen Rest der Formel VII steht,

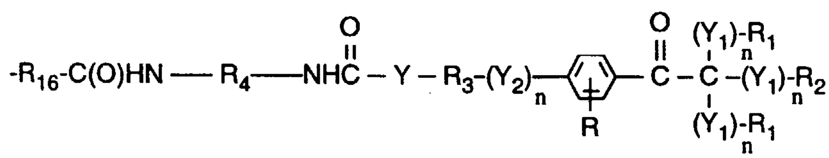 (VII),

worin R, $R_1$, $R_2$, $R_3$, $R_4$, Y, $Y_1$, $Y_2$ und n die zuvor angegebenen Bedeutungen haben, und $R_{16}$ eine direkte Bindung oder eine Gruppe $-C(O)-(CHOH)_r-CH_2-O-$ darstellt, worin r für 0 oder eine ganze Zahl von 1 bis 4 steht. Für R, $R_1$, $R_2$, $R_3$, $R_4$, Y, $Y_1$,
$Y_2$ und n gelten die zuvor angegebenen Bevorzugungen. $X_1$ steht bevorzugt für -NH-.

Andere bevorzugte Oligomere und Polymere sind solche auf der Basis von Oligo- und Polyvinylalkohol, in denen die H-Atome in den OH-Gruppen teilweise oder ganz mit einem Rest der Formel V substituiert sind. Es kann sich um Homopolymere mit $-CH_2CH(OH)-$Struktureinheiten oder um Copolymere mit anderen mono- oder bivalenten Struktureinheiten von Olefinen handeln.

Bevorzugter sind solche Oligomere und Polymere, welche

a) 5 bis 100 Mol-% Struktureinheiten der Formel IX

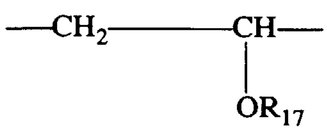 (IX),

und b) 95 bis 0 Mol-% Struktureinheiten der Formel X

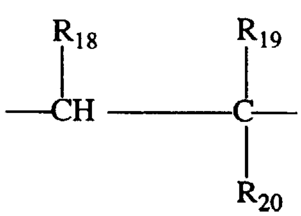 (X)

enthalten, worin $R_{17}$ einen Rest der Formel V

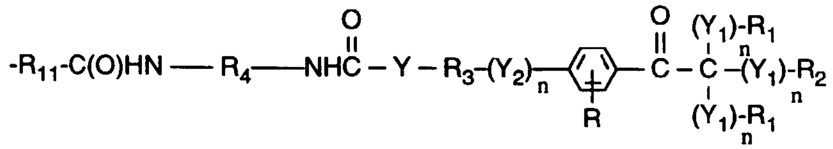 (V),

darstellt, worin R, $R_1$, $R_2$, $R_3$, $R_4$, Y, $Y_1$, $Y_2$ und n die zuvor angegebenen Bedeutungen haben, und $R_{11}$ eine direkte Bindung, -($C_1$-$C_4$-Alkylen-O)- oder -($C_2$-$C_{10}$-Alkylen-NH)-darstellt; $R_{18}$ für H, $C_1$-$C_6$-Alkyl, -$COOR_{21}$ oder -$COO^{\ominus}$ steht, $R_{19}$ H, F, Cl, CN oder $C_1$-$C_6$-Alkyl bedeutet, und $R_{20}$ H, OH, $R_{11}$-H, F, Cl, CN, $R_{21}$-O-, $C_1$-$C_{12}$-Alkyl, -$COO^{\ominus}$, -$COOR_{10}$, -OCO-$R_{10}$, Methylphenyl oder Phenyl darstellt, wobei $R_{21}$ $C_1$-$C_{18}$-Alkyl, $C_5$-$C_7$-Cycloalkyl, ($C_1$-$C_{12}$-Alkyl)-$C_5$-$C_7$-cycloalkyl, Phenyl, ($C_1$-$C_{12}$-Alkyl)phenyl, Benzyl oder ($C_1$-$C_{12}$-Alkyl)benzyl darstellt.

$R_{18}$ steht bevorzugt für H. Bedeutet $R_{18}$ Alkyl, so handelt es sich bevorzugt um Methyl oder Ethyl. Bedeutet $R_{18}$-$COOR_{21}$, so stellt $R_{21}$ bevorzugt $C_1$-$C_{12}$-, besonders $C_1$-$C_6$-Alkyl dar.

Bedeutet $R_{19}$ Alkyl, so handelt es sich bevorzugt um $C_1$-$C_4$-Alkyl, z.B. Methyl, Ethyl, n-Propyl und n-Butyl. $R_{19}$ steht bevorzugt für H, Cl oder $C_1$-$C_4$-Alkyl.

Bedeutet $R_{20}$ die Gruppe $R_{21}$-O-, so stellt $R_{21}$ bevorzugt $C_1$-$C_{12}$-, besonders $C_1$-$C_6$-Alkyl dar. Bedeutet $R_{20}$ Alkyl, so enthält es bevorzugt 1 bis 6, besonders 1 bis 4 C-Atome. Bedeutet $R_{20}$ die Gruppe -$COOR_{21}$, so stellt $R_{21}$ bevorzugt $C_1$-$C_{12}$-, besonders $C_1$-$C_6$-Alkyl, Cyclopentyl oder Cyclohexyl dar. Bedeutet $R_{20}$ die Gruppe -OCO-$R_{21}$, so stellt $R_{21}$ bevorzugt $C_1$-$C_{12}$-, besonders $C_1$-$C_6$-Alkyl, Phenyl oder Benzyl dar.

In einer bevorzugten Ausführungsform stehen $R_{18}$ für H, $R_{19}$ für H, F, Cl, Methyl oder Ethyl, und $R_{20}$ für H, OH, F, Cl, CN, $C_1$-$C_4$-Alkyl, $C_1$-$C_6$-Alkoxy, $C_1$-$C_6$-Hydroxyalkoxy, -COO-$C_1$-$C_6$-Alkyl, -OOC-$C_1$-$C_6$-Alkyl oder Phenyl.

Besonders bevorzugt sind solche Oligomeren und Polymeren, worin $R_{18}$ H bedeutet, $R_{19}$ H oder Methyl darstellt, und $R_{20}$ H, OH, CN, Methyl, $OCH_3$, $O(CH_2)_tOH$ oder -$COOCH_3$ bedeutet, und t für ganze Zahlen von 2 bis 6 steht.

Eine weitere bevorzugte Gruppe von Oligomeren und Polymeren sind teilweise oder vollständig hydroxyalkylierte Oligo- oder Polyacrylate oder -methacrylate beziehungsweise -acrylamide oder -methacrylamide, in denen die primäre Hydroxylgruppe beziehungsweise Aminogruppe durch Reste der Formel VII substituiert sind. Sie können zum Beispiel 5 bis 100 Mol-% Struktureinheiten der Formel XI

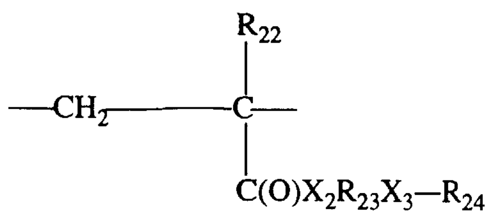 (XI),

und 95 bis 0 Mol-% Struktureinheiten der Formel XII

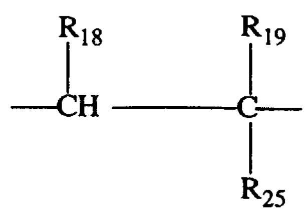 (XII)

enthalten, worin $R_{22}$ H oder Methyl bedeutet, $X_2$ und $X_3$ unabhängig voneinander -O- oder -NH- darstellen, $R_{23}$ für -$(CH_2)_c$- steht und c ganze Zahlen von 2 bis 12, vorzugsweise 2 bis 6 bedeutet, $R_{24}$ einen Rest der Formel VII darstellt, $R_{18}$ und $R_{19}$ die zuvor angegebenen Bedeutungen haben, und $R_{25}$ die gleiche Bedeutung wie $R_{20}$ hat oder -C(O)$X_2R_{23}X_3$H bedeutet. Für $R_{24}$, $R_{18}$, $R_{19}$ und $R_{20}$ gelten die zuvor angegebenen Bevorzugungen. Für $X_2$ und $X_3$ gelten die zuvor angegebenen Bevorzugungen.

Andere bevorzugte Oligomere und Polymere sind solche aus Polyalkylenoxiden, in denen die H-Atome der endständige OH- oder $-NH_2$-Gruppen teilweise oder vollständig mit Resten der Formel VII substituiert sind. Es kann sich zum Beispiel um solche der Formel XIII mit gleichen oder verschiedenen wiederkehrenden Struktureinheiten $-[CH_2CH(R_{27})-O]-$ handeln,

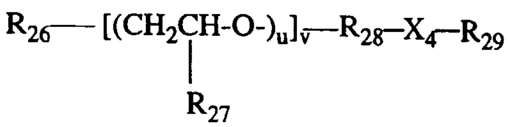 (XIII),

worin $R_{26}$ die Gruppe $R_{29}$-$X_4$- darstellt oder der v-wertige Rest eines Alkohols oder Polyols mit 1 bis 20 C-Atomen ist, $R_{27}$ H, $C_1$-$C_8$-Alkyl, vorzugsweise $C_1$-$C_4$-Alkyl und insbesondere bevorzugt Methyl bedeutet, $R_{28}$ zusammen mit $X_4$ eine direkte Bindung oder $R_{28}$ $C_2$-$C_6$-Alkylen, vorzugsweise $C_3$-$C_6$-Alkylen und insbesondere bevorzugt 1,3-Propylen darstellt, $X_4$ für -O- oder -NH- steht, $R_{29}$ einen Rest der Formel VII bedeutet, u für einen Zahlenwert von 3 bis 10 000, bevorzugt 5 bis 5 000, besonders bevorzugt 5 bis 1000 und insbesondere bevorzugt 5 bis 100 steht, und v eine ganze Zahl von 1 bis 6, bevorzugt 1 bis 4 bedeutet.

$R_{26}$ kann der ein- bis vierwertige Rest eines Alkohols oder Polyols sein. Wenn es sich bei $R_{26}$ um den Rest eines Alkohols handelt, so bedeutet $R_{26}$ vorzugsweise lineares oder verzweigtes $C_3$-$C_{20}$-Alkyl oder -Alkenyl, $C_3$-$C_8$- und besonders $C_5$-$C_6$-Cycloalkyl, $-CH_2$-($C_5$-$C_6$-Cycloalkyl), $C_6$-$C_{10}$-Aryl und besonders Phenyl und Naphthyl, $C_7$-$C_{16}$-Aralkyl und besonders Benzyl und 1-Phenyleth-2-yl. Die cyclischen beziehungsweise aromatischen Reste können mit $C_1$-$C_{18}$-Alkyl oder $C_1$-$C_{18}$-Alkoxy substituiert sein.

Wenn es sich bei $R_{26}$ um den Rest eines Diols handelt, so bedeutet $R_{26}$ vorzugsweise verzweigtes und besonders lineares $C_3$-$C_{20}$-Alkylen oder Alkenylen und bevorzugter $C_3$-$C_{12}$-Alkylen, $C_3$-$C_8$- und besonders $C_5$-$C_6$-Cycloalkylen, $-CH_2$-($C_5$-$C_6$-Cycloalkyl)-, $-CH_2$-($C_5$-$C_6$-Cycloalkyl)-$CH_2$-, $C_7$-$C_{16}$-Aralkylen und besonders Benzylen, $-CH_2$-($C_6$-$C_{10}$-Aryl)-$CH_2$- und besonders Xylylen. Die cyclischen beziehungsweise aromatischen Reste können mit $C_1$-$C_{12}$-Alkyl oder $C_1$-$C_{12}$-Alkoxy substituiert sein.

Wenn es sich bei $R_{26}$ um einen dreiwertigen Rest handelt, so leiten sich diese von aliphatischen oder aromatischen Triolen ab. $R_{26}$ ist bevorzugt ein dreiwertiger aliphatischer Rest mit 3 bis 12 C-Atomen, der sich besonders von Triolen mit vorzugsweise primären Hydroxylgruppen ableitet. Besonders bevorzugt stellt $R_{26}$ $-CH_2(CH-)CH_2-$, $HC(CH_2-)_3$ oder $CH_3C(CH_2-)_3$ dar.

Wenn es sich bei $R_{26}$ um einen vierwertigen Rest handelt, so leiten sich diese bevorzugt von aliphatischen Triolen ab. $R_{26}$ ist in diesem Fall bevorzugt $C(CH_2-)_4$.

Für $R_{29}$ gelten die zuvor angegebenen Bevorzugungen. Besonders bevorzugt sind Homo-und Blockoligomere und -polymere mit Struktureinheiten der Formeln $-[CH_2CH_2-O]-$ und $-[CH_2CH(CH_3)-O]-$.

Geeignet sind auch fluorierte Polyether, die der Formel XIIIa

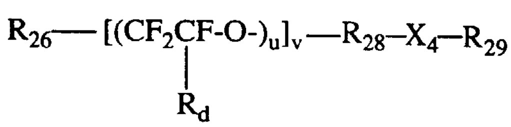 (XIIIa)

entsprechen, worin $R_{28}$, $R_{29}$, $X_4$, u und v die zuvor angegebenen Bedeutungen haben, $R_{26}$ die zuvor angegebene Bedeutung hat oder der einwertige Rest eines teil- oder perfluorierten Alkohols mit 1 bis 20, bevorzugt 1 bis 12 und besonders bevorzugt 1 bis 6 C-Atomen, oder der zweiwertige Rest eines teil- oder perfluorierten Diols mit 2 bis 6, bevorzugt 2 bis 4 und besonders bevorzugt 2 oder 3 C-Atomen ist, und $R_d$ F oder Perfluoralkyl mit 1 bis 12, bevorzugt 1 bis 6 und besonders bevorzugt 1 bis 4 C-Atomen bedeutet. $R_d$ steht besonders bevorzugt für $-CF_3$.

Weitere geeignete Oligomere und Polymere sind zum Beispiel Polyethylenimine, in denen H-Atome der NH-Gruppen durch Reste der Formel V substituiert sind, einschliesslich der schon erwähnten Bevorzugungen. Ebenfalls geeignet ist Poly-ε-lysin.

Die Herstellung der erfindungsgemässen Oligomeren und Polymeren kann einfach und in an sich bekannter Weise durch die Umsetzung von Verbindungen der Formel I mit HO-oder NH-funktionellen Oligomeren und Polymeren erfolgen. NH-funktionelle Oligomere und Polymere sind in grosser Vielzahl bekannt und käuflich erhältlich; deren Umsetzung mit Zuckersäuren ergibt die entsprechenden Ester und Amide mit einem endständigen Polyhydroxyalkylrest.

Die Verbindungen der Formeln I eignen sich hervorragend als Initiatoren für die strahlungsinduzierte Polymerisation von ethylenisch ungesättigten Verbindungen. Die erfindungsgemässen Oligomeren und Polymeren eignen sich ebenfalls ausgezeichnet als Initiatoren, wobei Pfropfpolymere gebildet werden oder auch je nach Gehalt an Initiatorgruppen im Makroinitiator sich durchdringende und nicht oder nur teilweise miteinander verbundene Polymernetzwerke gebildet werden können.

Die Verbindungen der Formel I können auch auf Oberflächen von anorganischen oder organischen Materialien (nachfolgend als Substrate bezeichnet) gebunden werden, die H-aktive -COOH, HO-, HS- oder -NH-Gruppen enthalten. Geeignete Verfahren hierfür sind bekannt, zum Beispiel Tauchen, Sprühen, Streichen, Rakeln, Giessen, Rollen und besonders Schleudergiessen oder Vakuumaufdampfverfahren. Die Verbindungen der Formel I, werden durch eine Reaktion mit den Isocyanatgruppen fest auf der Oberfläche verankert. Diese Reaktion kann zum Beispiel bei erhöhten Temperaturen durchgeführt werden, zum Beispiel bei 40 bis 100 °C. Nach der Reaktion können überschüssige Verbindungen zum Beispiel mit Lösungsmitteln entfernt werden. Auf den modifizierten Oberflächen können nun photopolymerisierbare Substanzen aufgetragen werden, die anschliessend unter Strahlungseinwirkung polymerisiert werden, und durch Pfropfpolymerisation über die Photoinitiatoren fest mit dem Substrat verbunden werden. Hierbei bildet sich eine tentakelartige oder bürstenartige Polymerstruktur auf der Substratoberfläche.

Geeignete Substrate sind zum Beispiel Gläser, silikatische Minerale (Kieselgele), Metalloxide und besonders natürliche oder synthetische Kunststoffe, die in grosser Vielzahl bekannt sind. Einige Beispiele für Kunststoffe sind Polyadditions- und Polykondensationskunststoffe (Polyurethane, Epoxidharze, Polyether, Polyester, Polyamide, Polyimide); Vinylpolymere (Polyacrylate, Polymethacrylate, Polystyrol, Polyethylen und seine halogenierten Derivate, Polyvinylacetat und Polyacrylnitril); Elastomere (Silicone, Polybutadien und Polyisopren); gegebenenfalls modifizierte Biopolymere (Collagen, Cellulose, Chitosan und zuvor genannte Biopolymere). Wenn Substrate zu wenig oder keine funktionellen Gruppen enthalten, kann die Substratoberfläche mit an sich bekannten Methoden, zum Beispiel Plasmaverfahren, modifiziert und funktionelle Gruppen wie -OH, $-NH_2$ oder $-CO_2H$ erzeugt werden. Besonders bevorzugte Substrate sind Kontaktlinsen.

Ein weiterer Gegenstand der Erfindung ist ein Material aus (a) einem anorganischen oder vorzugsweise organischen Substrat, auf dem (b) als Photoinitiator mindestens eine Verbindung der Formel I gebunden ist, die über O-Atome, S-Atome, N-$C_1$-$C_6$-Alkyl-Gruppen oder NH-Gruppen einerseits und die Isocyanatgruppe der Photoinitiatoren andererseits fest mit dem Substrat verbunden ist, und gegebenenfalls (c) einer dünnen Schicht aus einem Polymer auf der Photoinitiatorschicht, welches Polymer durch Aufbringen einer dünnen Schicht aus photopolymerisierbaren ethylenisch ungesättigten Substanzen auf der mit Photoinitiatorresten versehenen Substratoberfläche und Polymerisation der Schicht aus ethylenisch ungesättigten Substanzen durch Bestrahlen vorzugsweise mit UV-Strahlung erhältlich ist.

Bei diesem Material handelt es sich bevorzugt um einen ophthalmischen Formkörper aus einem transparenten organischen Basismaterial, wie z.B. eine Kontaktlinse oder eine Intraokularlinse, besonders bevorzugt um eine Kontaktlinse.

Die Schichtdicke der ethylenisch ungesättigten Substanzen richtet sich hauptsächlich nach den gewünschten Eigenschaften. Sie kann 0,001 µm bis 1000 µm, bevorzugt 0,01 µm bis 500 µm, besonders bevorzugt 0,01 bis 100 µm, insbesondere bevorzugt 0,5 bis 50 µm und ganz besonders bevorzugt 1 bis 20 µm betragen. Speziell für die Herstellung von Kontaktlinsen ist eine Schichtdicke von 0,5 bis 5 µm gewünscht. Die Herstellung der Schichten kann nach den zuvor erwähnten Beschichtungsverfahren erfolgen.

Ethylenisch ungesättigte photovernetzbare Verbindungen sind bekannt.
Photopolymerisierbare Substanzen sind z.B. Acryl- und besonders Methacrylsäureester von Alkoholen und Polyolen, oder Acryl- und besonders Methacrylsäureamide von Aminen und Polyaminen, z.B. $C_1$-$C_{18}$-Alkanolen, Ethylenglykol, Propandiol, Butandiol, Hexandiol, Di(hydroxymethyl)cyclohexan, Polyoxyalkylendiole wie z.B. Di-, Tri- oder Tetraethylenglykol, Di- oder Tri-1,2-propylenglykol, Trimethylolmethan, ethan oder -propan und Pentaerythrit, $C_1$-$C_{18}$-Alkylaminen, Ethylendiamin, Diethylentriamin und Triethylentetramin, die alleine, in Mischungen und in Abmischung mit Bindemitteln verwendet werden können. Ferner sind Mono-, Oligo- und Polysiloxane mit Acryl- und besonders Methacrylsäureresten, die an seiten- oder endständige Hydroxy($C_2$-$C_{12}$-Alkyl)- oder Amino($C_2$-$C_{12}$-Alkyl)-Gruppen gebunden sind, geeignet, zum Beispiel 1-Trimethylsilyl-3-methacroyloxypropan, 1-Pentamethyldisiloxanyl-3-methacryloxypropan und 3-[Tris(trimethylsiloxy)-silyl]-propyl-methacrylat. Ferner sind auch Perfluoralkylacrylate und -methacrylate geeignet.

Weitere geeignete ethylenisch ungesättigte Verbindungen sind nichtflüchtige substituierte Polyolefine, besonders Acrylsäure und Methacrylsäure sowie deren Ester und Amide, zum Beispiel Acrylsäure- oder Methacrylsäure-$C_1$-$C_{12}$-alkylester oder -oligooxaalkylenester oder -$C_1$-$C_{12}$-hydroxyalkylester oder -amide (2,3-Dihydroxypropyl-methacrylat, N,N-Dimethyl-acrylamid, Acrylamid, N,N-Diethylaminoethyl-methacrylat, Oligoethylenoxidacrylate und -methacrylate, 2-Hydroxyethylmethacrylsäureester), und N-Vinylpyrrolidon.

Ein weiterer Gegenstand der Erfindung ist ein Verfahren zur Modifizierung von Oberflächen anorganischer oder organischer Substrate, die H-aktive HO-, HS-, HN-$C_1$-$C_6$-Alkyl-Gruppen oder $-NH_2$-Gruppen enthalten, umfassend die Schritte

a) Aufbringen einer dünnen Schicht aus Photoinitiatoren mindestens einer Verbindung der Formel I auf dem Substrat, gegebenenfalls zusammen mit einem Katalysator, wie zum Beispiel Dibutylzinnlaurat,

b) gegebenenfalls Erwärmen des beschichteten Materials und Abwaschen des überschüssigen Photoinitiators,

c) Aufbringen einer dünnen Schicht aus photopolymerisierbaren ethylenisch ungesättigten Substanzen auf der mit Photoinitiatorresten versehenen Substratoberfläche, und

d) Polymerisation der Schicht aus ethylenisch ungesättigten Substanzen durch Bestrahlen vorzugsweise mit UV-Strahlung.

Eventuell gebildete nicht-kovalent gebundene Polymerisate können nach der Polymerisation entfernt werden, zum Beispiel durch Behandlung mit geeigneten Lösungsmitteln.

Mit dem erfindungsgemässen Verfahren können die Oberflächen auf vielfältige Weise modifiziert und den Oberflächen besondere Eigenschaften für unterschiedliche Verwendungszwecke verliehen werden. Je nach Wahl der ethylenisch ungesättigten Substanzen können zum Beispiel mechanische Eigenschaften, zum Beispiel die Oberflächenhärte, Kratzfestigkeit, Benetzbarkeit, Abriebfestigkeit und Beschreibbarkeit, sowie physikalische Eigenschaften wie zum Beispiel der Reibungskoeffizient, die Durchlässigkeit für Gase, Flüssigkeiten und gelöste anorganische oder organische Substanzen mit niederem bis hohen Molekulargewicht, sowie die Lichtdurchlässigkeit gezielt verbessert werden, wobei eine besonders feste Haftung der Polymerschichten einen besonderen Vorteil darstellt.

Die erfindungsgemässen Photoinitiatoren beziehungsweise mit den Photoinitiatoren modifizierten Substrate zeichnen sich durch eine hohe chemische und photochemische Reaktivität aus. Sie können zum Aufbau von photoreaktiven Materialien verwendet werden, die als Beschichtungsmaterialien, photostrukturierbare Materialien, für Verbundmaterialien und insbesondere als Werkstoffe für biomedizinische Anwendungen, zum Beispiel Kontaktlinsen und chirurgische Materialien, Anwendung finden können. Besonders eignen sich die Materialien zur Erzeugung von hydrophilen und biokompatiblen Oberflächen auf Kontaktlinsen durch Pfropfpolymerisation unter Bildung einer für geforderte Eigenschaften besonders günstigen Tentakelstruktur (Bürstenstruktur).

Von besonderer Bedeutung sind die hohe Benetzbarkeit und die Erhaltung eines stabilen Feuchtigkeitsfilms auf der Oberfläche, zum Beispiel eines Tränenfilms auf der Oberfläche einer Kontaktlinse. Ferner ist die Verbesserung des Verhaltens in biologischen Systemen von grosser Bedeutung, zum Beispiel eine verbesserte Biokompatibilität, Schutz gegen Bioerrosion, Verhinderung einer Plaque-Bildung und von Bio-Fouling, keine Blutgerinnung oder toxische und allergische Reaktionen.

Die erfindungsgemässen modifizierten Materialien eignen sich besonders zur Herstellung von Kontaktlinsen. Im Hinblick auf Kontaktlinsen sind folgende Eigenschaftsverbesserungen besonders wichtig: Hohe Benetzbarkeit (kleiner Kontaktwinkel), hohe Reissfestigkeit, guter Schmiereffekt, hohe Abriebfestigkeit, kein oder nur unwesentlicher enzymatischer Abbau, keine Ablagerung von Komponenten aus der Tränenflüssigkeit (Proteine, Lipide, Salze, Zellabbauprodukte), keine Affinität zu Kosmetika, flüchtigen Chemikalien wie zum Beispiel Lösungsmittel, Schmutz und Staub, kein Anheften beziehungsweise Einnisten von Mikroorganismen.

Die erfindungsgemäss modifizierten Materialien eignen sich auch zur Herstellung von künstlichen Blutgefässen und anderen biomedizinischen Werkstoffen für Prothesen, für die Chirurgie und für die Diagnostik, wobei besonders vorteilhaft ist, dass diese mit Endothelzellen überwachsen werden können.

Ein weiterer Gegenstand der Erfindung ist eine Kontaktlinse aus (a) einem transparenten organischen Basismaterial mit funktionellen Gruppen, insbesondere Hydroxy-, Mercapto-, Amino-, Alkylamino- oder Carboxygruppen, und (b) einer dünnen Schicht auf der Oberfläche aus (b1) mindestens einem Photoinitiator der Formel I und (b2) einem Pfropfpolymer, gebildet duch Photopolymerisation eines nichtflüchtigen oder schwerflüchtigen Olefins.

Geeignete Basismaterialien sind zum Beispiel gegebenenfalls modifizierte natürliche Polymere wie zum Beispiel Collagen, Chitosan, Hyaluronsäure und Celluloseester wie Celluloseacetat oder Cellulosebutyrat. Geeignete Basismaterialien sind zum Beispiel gegebenenfalls modifizierte syntehetische Polymere wie zum Beispiel Polyvinylalkohol, Polyhydroxyethylmethacrylat, Polyglycerylmethacrylat,und Copolymere auf der Basis dieser Polymeren. Geeignet sind auch natürliche und synthetische Polymere, zum Beispiel Polymere mit Silikon-, Perfluoralkyl- und/oder Alkylacrylatstruktureinheiten, bei denen mit geeigneten Verfahren wie zum Beispiel Plasmabehandlung, Ätzen oder Oxidation funktionelle Gruppen auf der Oberfläche erzeugt werden.

Geeignete nicht flüchtige oder schwerflüchtige Olefine sind zum Beispiel Acrylamid, N,N-Dimethylacrylamid, Methacrylamid, Hydroxyethylmethacrylat, Glycerylmethacrylat, Oligoethylenoxidmono- und -bisacrylate, Ethylenglykoldimethacrylat, Methylenbisacrylamid, Vinylcaprolactam, Acrylsäure, Methacrylsäure, Fumarsäuremonovinylester, Vinyltrifluoracetat und Vinylencarbonat.

Die nachfolgenden Beispiele erläutern die Erfindung näher.

A) Herstellungsbeispiele

Beispiel A1: Herstellung von

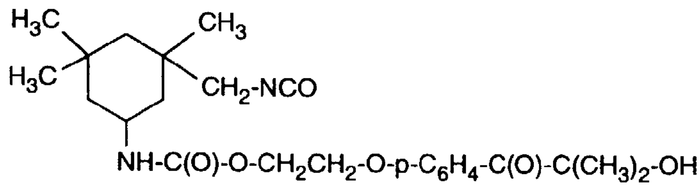

In einem 500 ml Kolben mit Rückflusskühler, Thermometer, Rührer und Stickstoffeinleitungsrohr wird unter Stickstoff eine Lösung von 11,125 g (0,05 Mol) frisch destilliertem Isophorondiisocyanat (IPDI) in 50 ml trockenem Methylenchlorid mit einer Lösung von 11,2 g (0,05 Mol) 4'-(β-Hydroxyethoxy)-2-hydroxyprop-2-yl-phenon (Darocure 2959®) in 300 ml trockenem Methylenchlorid gemischt und nach Zugabe von 20 mg Dibutyl-zinndilaurat als Katalysator 48 Stunden bei Raumtemperatur gerührt. Der Verlauf der Umsetzung wird mittels Dünnschichtchromatographie auf Kieselgelplatten (60 $F_{254}$, Art. 5719 Merck) verfolgt (Laufmittel: Toluol/Acetonitril 7:3). Das entstandene Produkt wird durch Säulenchromatographie an Kieselgel 60 (Eluent Toluol/Acetonitril 7:3) von geringen Mengen an nicht umgesetztem Darocure 2959 und zweifach substituiertem IPDI befreit. Nach Eindampfen der reinen Fraktionen am Rotationsverdampfer wird ein farbloses Oel erhalten, das beim Abkühlen auf -16°C langsam kristallisiert und anschliessend aus trockenem Diethylether umkristallisiert wird. Es werden 15,6 g eines weissen kristallinen Produktes erhalten (70 % d. Th.), das einen Schmelzpunkt von 76°C aufweist.

Der Isocyanatgehalt des Produktes wird durch Titration mit Dibutylamin in Toluol ermittelt: berechnet 2,242 mVal/g, gefunden 2,25 mVal/g.

Die Methode ist in "Analytical Chemistry of Polyurethanes" (High Polymer Series XVI/Part III, D.S. David + H.B. Staley editors, Interscience Publishers, New York 1969 p. 86) beschrieben.

Beispiel A2: Herstellung von

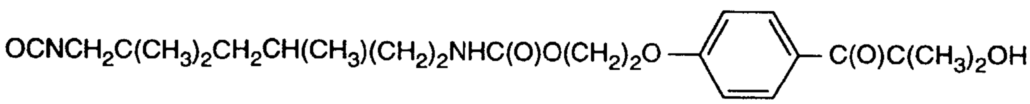

Analog Beispiel Al werden 10,5 g (0,05 Mol) 1,6-Diisocyanato-2,2,4-trimethyl-hexan (TMDI) mit 11,1 g (0,05 Mol) Darocure 2959® in 400 ml trockenem Methylenchlorid bei Raumtemperatur unter Stickstoff während 40 Stunden umgesetzt. Es werden 14,5 g (67% d. Th.) eines weissen kristallinen Produktes vom Schmelzpunkt 41-43°C erhalten. NCO Titration: berechnet 2,30 mVal/g, gefunden 2,36 mVal/g.

Beispiel A3: Herstellung von

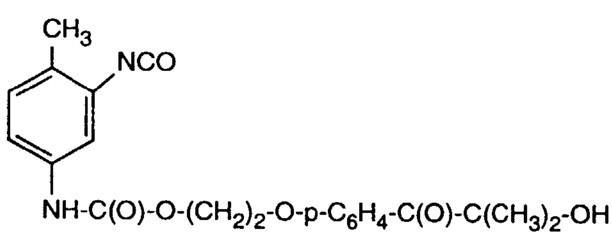

In der unter Beispiel A1 beschriebenen Apparatur werden 1,74 g (0,01 Mol) Toluylen2,4-diisocyanat (TDI) in 20 ml Dichlormethan mit 2,24 g (0,01 Mol) Darocure 2959® gelöst in 60 ml trockenem Dichlormethan umgesetzt. Die Reaktionsmischung wird ohne Zusatz eines Katalysators während 48 Stunden bei Raumtemperatur und 1 Stunde bei 40°C gerührt, bis im Dünnschichtchromatogramm kein unumgesetztes Darocure 2959 mehr nachgewiesen werden kann. Das Produkt wird durch Ausfällen der Reaktionslösung in 180 ml trockenem Petrolether (Sdp. 40-60°C) isoliert

und anschliessend zweimal aus Dichlormethan/Petrolether 1:3 umkristallisiert.

Es wird ein weisses kristallines Produkt vom Schmelzpunkt 124-125°C erhalten. Ausbeute 17,2 g entsprechend 87 % der Theorie. OCN-Titration: berechnet 2,50 mVal/g gefunden 2,39 mVal/g.

B) Herstellung von Makrophotoinitiatoren

Beispiel B1: Herstellung von

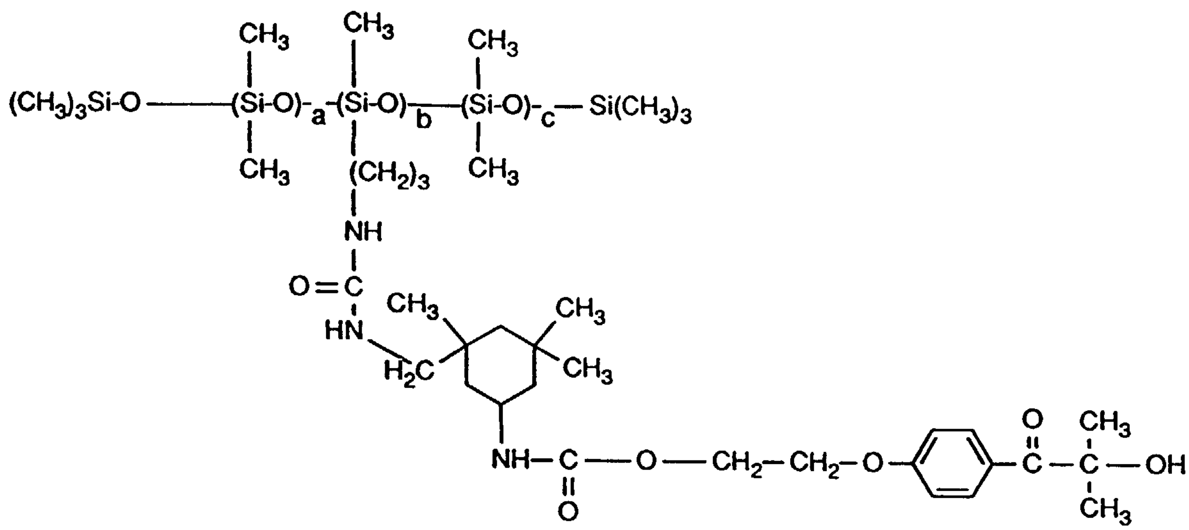

In einem wie in Beispiel A1 beschriebenen Kolben von 250 ml Inhalt wird eine Lösung von 1 g der Verbindung gemäss Beispiel A1 (0,00224 Mol) in 50 ml trockenem Dichlormethan mit 4,37 g Aminoalkyl-polysiloxan (0,515 m Val $NH_2$/g, Petrarch PS 813®: $\bar{M}n \sim 3000$, b = 3, a+c = 37) gelöst in 100 ml trockenem Dichlormethan umgesetzt. Die Reaktionsmischung wird 10 Stunden bei Raumtemperatur gerührt und anschliessend 1 Stunde auf 40°C erwärmt. Nach dem Abkühlen wird das Lösungsmittel durch Eindampfen am Rotationsverdampfer entfernt. Es wird ein hochviskoses, farbloses Oel erhalten, das zuletzt im Hochvakuum bei 40°C und 1,33 hP ($10^{-4}$Torr) von Spuren des Lösungsmittels befreit wird. Ausbeute 5,34 g entsprechend 99,5 % der Theorie. Das Produkt zeigt im IR-Spektrum keine OCN-Bande mehr.

Beispiele B2-B6

Analog Beispiel B1 werden weitere aminofunktionelle Makromere mit der in Beispiel A1 beschriebenen Verbindung umgesetzt. Die Resultate sind in der Tabelle 3 zusammengefasst.

Tabelle 3:

| Beispiel | aminofunktionelles Makromer | Verbindung nach Bsp. A1 | Struktur (Produkt) | Ausbeute | % N (Berechnet/Gefunden) |
|---|---|---|---|---|---|
| B2 | X-22-161c (Shin Etsu, JP) 7,8 g (0,43 mVal $NH_2$/g) $\overline{M}$ ~ 4600 | 1,5 g (3,36 m Mol) | a | 9,2 g (99,6%) | 1,52/1,42 |
| B3 | Jeffamin® T 403 (Texaco, USA) 2,8 g (6,38 mVal $NH_2$/g) | 2,84 g (6,36 m Mol) | b | 5,62g (99,7%) | 7,08/7,11 |
| B4 | Jeffamin® D2000 (Texaco, USA 4,0 g (1 mVal $NH_2$/g) | 1,786 g (2,0 m Mol) | c | 5,78g (99,9%) | 2,90/2,89 |
| B5 | KF-8003 (Skin Etsu, JP) 4,6 g (0,49 mVal $NH_2$/g) | 1,0 g (2,29 m Mol) | d | 4,55g (98,9%) | 1,63/1,58 |
| B6 | X-22-161B (Shin Etsu, JP) 3,23 g (0,699 mVal $NH_2$/g) $\overline{M}$~ 2900 | 1,0 g (2,29 m Mol) | e | 4,2g (99,3%) | 2,23/2,09 |

a = 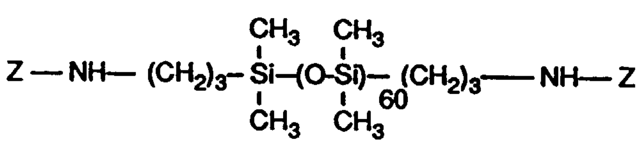

b = 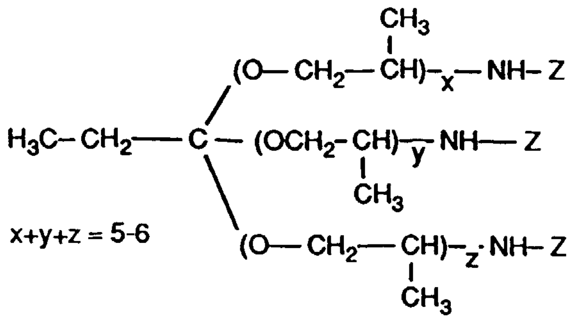

x+y+z = 5-6

c = 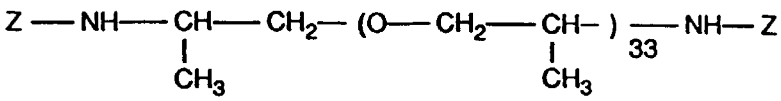

d = 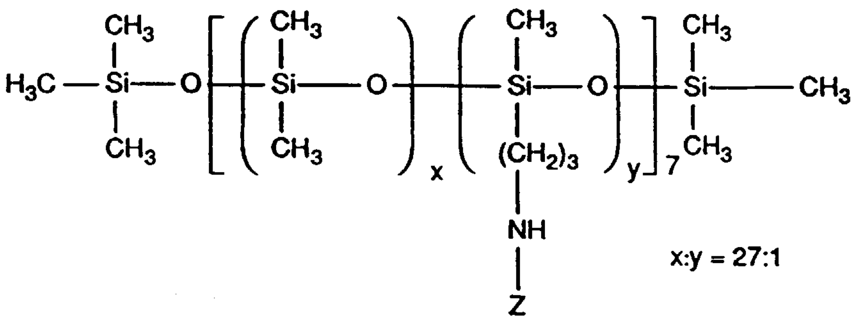

x:y = 27:1

e = 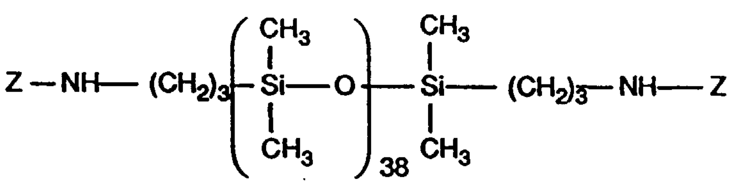

Z= 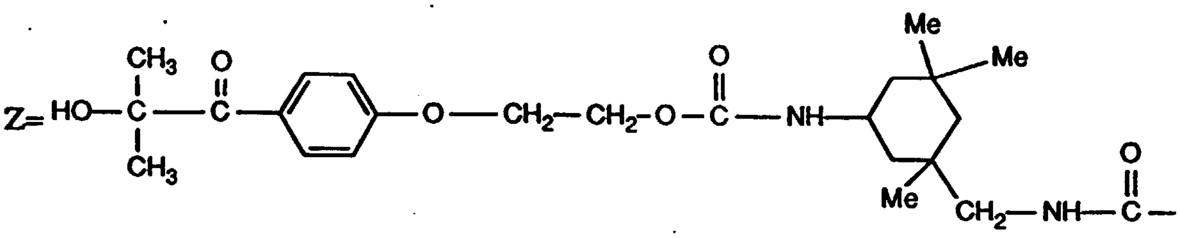

Beispiel B7: Herstellung von

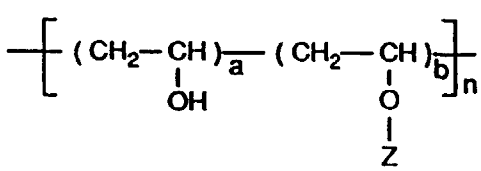

a:b≈30:1
n≈10

In der unter Beispiel Al beschriebenen Apparatur werden unter Stickstoff 2,1 g Polyvinylalkohol (PVA) (Serva®

03/20 $\overline{M}n$ ~ 13'000) bei 90°C in 50 ml trockenem N-Methyl-2-pyrrolidon (NMP) gelöst. Die Lösung wird auf Raumtemperatur abgekühlt und nach dem Filtrieren durch eine G4 Glasfritte mit einer Lösung von 0,7 g (1,567 m Mol) der Verbindung gemäss Beispiel Al in 10 ml trockenem NMP versetzt. Nach Zugabe von 10 mg Dibutyl-zinn-dilaurat wird die Reaktionsmischung während 48 Stunden bei 50°C gerührt. Nach dieser Reaktionszeit kann IR-spektroskopisch kein unumgesetztes Isocyanat mehr nachgewiesen werden (OCN bei 2280 $cm^{-1}$). Nach Abkühlen auf Raumtemperatur wird das Produkt in 400 ml trockenem Diethylether gefällt, filtriert, mit trockenem Diethylether gewaschen und im Vakuum getrocknet. Es werden 2,6 g eines weissen Produktes erhalten, das 1,38 % Stickstoff enthält. $^1H$-chemische Verschiebungen von aromatischen Protonen der Photoinitiatoren, die an PVA gebunden sind: δ 7,00-7,10 (d, 2H); δ 8,15-8,25 (d, 2H).

Beispiel B8: Umsetzung von Hyaluronsäure mit dem reaktiven Photoinitiator gemäss Beispiel A1.

Analog Beispiel B7 werden 444 mg Hyaluronsäure (Denki Kagaku Kogyo, $\overline{M}n$ ~ $1,2x10^6$) gelöst in 100 ml trockenem Dimethylsulfoxid (DMSO) mit einer Lösung von 200 mg der in Beispiel 1 beschriebenen Verbindung in 10 ml trockenem DMSO bei 50°C umgesetzt. Es werden 534 mg (82,7 % d. Th.) eines weissen Produktes erhalten, das an ca. 30 % der Zuckerreste in der Polymerhauptkette eine als Urethan oder Carbonsäureamid gebundene Photoinitiatorgruppe trägt, wie die Auswertung des $^1H$ NMR Spektrums zeigt.
$^1H$-chemische Verschiebungen von aromatischen Protonen der Photoinitiatoren, die an Hyaluronsäure gebunden sind: δ 7,00-7,10 (d, 2H); δ 8,15-8,25 (d, 2H).

Beispiele B9-B11:

Analog Beispiel B8 wird der in Beispiel A1 beschriebene reaktive Photoinitiator mit einigen hydroxyalkyl-substituierten Poly-dimethylsiloxanen in Dichlormethan als Lösungsmittel umgesetzt. Die Resultate sind in der Tabelle 4 angegeben.

Tabelle 4:

| Beispiel | Photoinitiator nach Bsp. 1 | Polysiloxan | Ausbeute | Elementaranalyse % Berechnet/Gefunden |
|---|---|---|---|---|
| B9 | 1,0 g (2,25 m Mol) | KF-6002 (Shin Etsu, JP) 3,6g (0,625 mVal OH/g) | 4,55 g (98,9%) | C 39,87/39,86<br>H 7,96/8,29<br>N 1,36/1,04 |
| B10 | 1,0 g (2,23 m Mol) | KF-6001 (Shin Etsu, JP) 2,05g (1,1 mVal OH/g) | 3,0 g (98,3%) | C 23,49/24,11<br>H 8,12/8,54<br>N 2,03/1,79 |
| B11 | 1,0 g (2,25 m Mol) | Gluconamidopropyl-methyl-dimethylsiloxan copolymer 4,55g (6,495 mVal OH/g) | 4,8 g (86,5%) | C -/36,18<br>H -/8,08<br>N -/1,03 |

Produkt B9 = 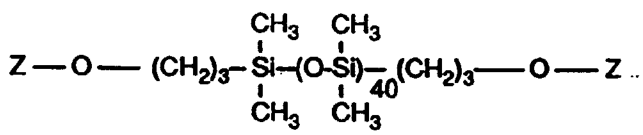

Produkt B10 = 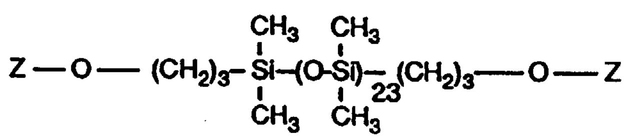

Produkt B11 = 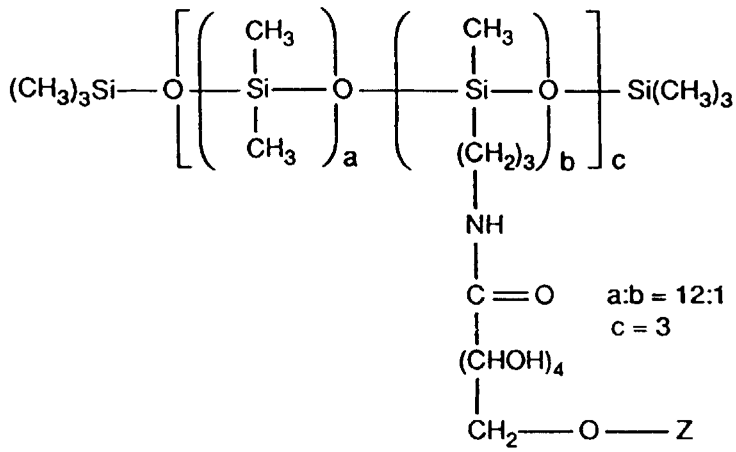

Beispiel B12: Cyclodextrinmakroinitiator

Cyclodextrine sind cyclische Oligosaccharide der Formel

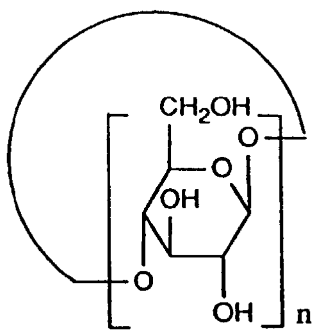

worin n für eine Zahl von 6 bis 8 steht. Sie sind käuflich wie auch hydroxyalkylierte Derivate mit einem Substitutionsgrad von 0,6 und 1,6 pro Dextrineinheit.

Die Umsetzung mit den erfindungsgemässen Photoinitiatoren ergibt im allgemeinen Gemische, die Derivate mit verschiedenartigen Substitutionsmustern und unterschiedlichem Substitutionsgrad umfassen. Bevorzugte Stelle der Substitution ist die primäre Hydroxylgruppe. Die Gemische können chromatographisch getrennt werden, wobei mit 6 bis 8 Photoinitiatoren am $C_6$ monosubstituierte Derivate leicht abgetrennt werden können. In einem 250 ml Kolben aus braunem Glas mit Rückflusskühler, Rührer, Innenthermometer und Tropftrichter werden unter trockenem Stickstoff 5 g (4,4053 mMol) trockenes β-Cyclodextrin und 0,094 g Dibutylzinnlaurat in 50 ml trockenem Dimethylsulfoxid gelöst. Zu dieser Lösung wird bei Raumtemperatur eine Lösung von 13,77 g (3,084 mMol) der Verbindung gemäss Beispiel A1 in 50 ml trockenem Dimethylsulfoxid zugetropft. Die Mischung wird zunächst 3 Stunden bei Raumtemperatur und anschliessend 15,5 Stunden bei 50 °C gerührt. Danach ist chromatographisch kein unumgesetztes β-Cyclodextrin mehr nachzuweisen. Das Reaktionsgemisch wird abgekühlt und das Produkt durch Zugabe von 1000 ml trockenem Diethylether ausgefällt. Das isolierte viskose Produkt wird in 25 ml Aceton gelöst und nochmals mit 500 ml Diethylether ausgefällt, wobei eine weisse Suspension entsteht Das Produkt wird abfiltriert und das erhaltene weisse Pulver zweimal mit 100 ml Diethylether gewaschen und anschliessend im Vakuum unter Lichtausschluss getrocknet Man erhält 13,04 g (53,5 % der Theorie) Produkt. Der Stickstoffgehalt von 3,73 % entspricht einem mittleren Substitutionsgrad von 5,6 pro Cyclodextrinring. Das Produkt wird durch Flash-Chromatographie (Säule 60 cm Länge, 5 cm Durchmesser) an Kieselgel (Merck 60 F, Körnung 0,04 bis 0,063 mm) mit Methano/Toluol (2:8) als Eluent fraktioniert. Mit 13 g Rohprodukt werden folgende Fraktionen erhalten, wobei die Fraktion 2 mit reinem Methanol und die Fraktion 3 mit Methanol/Wasser (1:1) eluiert werden:

| Fraktion | Menge (g) | N-Gehalt (%) | Mittlerer Substitutionsgrad |
|---|---|---|---|
| 1 | 1,3 | 4,25 | 6,4 |
| 2 | 3,59 | 3,59 | 5,4 |
| 3 | 1,36 | 1,36 | 2,0 |

C) Oberflächenreaktion von Polymerfilmen mit dem in Beispiel A1 beschriebenen reaktiven Photoinitiator.

Beispiele C1-C5:

Filme aus verschiedenen Polymermaterialien, die reaktive Gruppen aufweisen, werden auf der Oberflache mit der Lösung des gemäss Beispiel A1 hergestellten Photoinitiators in einem geeigneten Lösungsmittel (Konz.~ 20 Gew.%) benetzt, wobei als Technik Tauchen, Sprühen und Bestreichen angewendet wird. Die so behandelten Filme werden unter trockenem Sückstoff 24 Stunden auf 60°C erhitzt und anschliessend durch Waschen mit Aceton von nicht umgesetztem Photoinitiator befreit. Nach dem Trocknen unter Lichtausschluss werden die Filme mittels FTIR-Mikroskopie analysiert

| Beispiel | Polymerfilm | $\overline{M}n$ | Lösungsmittel | IR-Banden ($cm^{-1}$) |
|---|---|---|---|---|
| C1 | Polyvinyl-alkohol | ~70'000 | DMSO | (Ar C=C) 1600, 1510 (C=O) 1695 |
| C2 | Chitosan | ~145'000 | DMSO | (Ar C=C) 1600, 1510 (C=O) 1690 |
| C3 | Collagen | ~80'000 | DMSO | (Ar C=C) 1600, 1510 (C=O) 1695 |
| C4 | Polyvinyl-alkohol versetzt mit 1 % TMDI | - | MEK+1 % DMSO | (Ar C=C) 1600<br>(C=O) 1705 |
| C5 | Gluconamidopropyl-methyl-dimethylsiloxan-Copolymer, vernetzt mit IPDI (20% OH Gruppen) | ~4'000 | MEK+1 % DMSO | (Ar C=C) 1600, 1510<br>(C=O) 1700 |
| MEK = Methylethylketon | | | | |

Beispiel C6: Oberflächenreaktion einer Kontaktlinse

Kontaktlinsen aus vernetzten Polyhydroxyethylmethacrylaten (Poly-HEMA) werden auf der Oberfläche mit einer Lösung der Verbindung Al in Tetrahydrofuran (Konzentration 5 %) beziehungsweise Diethylether benetzt. Die behandelten Kontaktlinsen werden unter trockenem Stickstoff 16 Stunden bei Raumtemperatur gelagert. Danach werden die Kontaktlinsen 8 Stunden mit Aceton gewaschen und dann im Hochvakuum getrocknet.

D) Oberflächen - Pfropfpolymerisation mit den nach den Beispielen C1-C4 hergestellten modifizierten Polymerfilmen und N-Vinyl-2-pyrrolidon.

Beispiele D1-D4:

Die Polymerfilme gemäss den Beispielen C1-C4 werden durch Tauchen, Besprühen oder Bestreichen mit frisch destilliertem N-Vinyl-2-pyrrolidon benetzt, durch mehrfaches Evakuieren und Entlasten mit $N_2$ Gas von Sauerstoff befreit und in einer $N_2$-Atmosphäre der UV-Strahlung einer Hg Hochdrucklampe ausgesetzt (Photoresist Belichter 82420, Oriel). Anschliessend werden die Filme mehrmals mit Methanol gewaschen, um nicht polymerisiertes N-Vinyl-2-pyrrolidon und nicht gebundenes Homopolymerisat zu entfernen. Die Filme werden im Vakuum getrocknet und mittels FTIR-Spektroskopie analysiert (IR Banden von NVP).

| Beispiel | Dauer der UV-Bestrahlung | FTIR Banden ($cm^{-1}$) |
|---|---|---|
| D1 | 20 Minuten | 1510, 1600 (C=C Ar); 1660 (C=O), 1440-1470 NVP |
| D2 | 30 Minuten | 1510, 1600 (C=C Ar); 1660 (C=O) NVP |
| D3 | 15 Minuten | 1600 (C=C Ar); 1660 (C=O) NVP |
| D4 | 40 Minuten | 1600 (C=C Ar); 1675 (C=O), 1400-1450 NVP |

**Ar = aromatisch, NVP = N-Vinylpyrrolidon**

Beispiel D6: Modifikation der Oberfläche einer Kontaktlinse.

Gemäss Beispiel C6 behandelte Kontaktlinsen werden in eine wässrige Lösung von Acrylamid getaucht und dann durch mehrfaches Evakuieren und Entlasten mit Stickstoff von Sauerstoff befreit. Dann wird mit einer Hg-Hochdrucklampe (Photoresistbelichter 82420, Oniel, 2000 W) unter Stickstoff 2 mal 2 Minuten bestrahlt. Die Kontaktlinsen werden darauf mit destilliertem Wasser gewaschen und im Hochvakuum getrocknet. Die Kontaktlinsen zeigen vor (Poly-HEMA) und nach der Behandlung folgende Werte für die Kontaktwinkel und die Kontaktwinkel-Hysterese. Die Zahlenwerte zeigen die gute Reproduzierbarkeit.

| Produkt | Advancing angle | Receding angle | Hysterese |
|---|---|---|---|
| Poly-HEMA | 78° | 33° | 44° |
| Linse 1 nach Beispiel D6 | 54° | 49° | 5° |
| Linse 2 nach Beispiel D6 | 49° | 41° | 8° |
| Linse 3 nach Beispiel D6 | 53° | 48° | 5° |

## Patentansprüche

1. Verbindungen der Formel

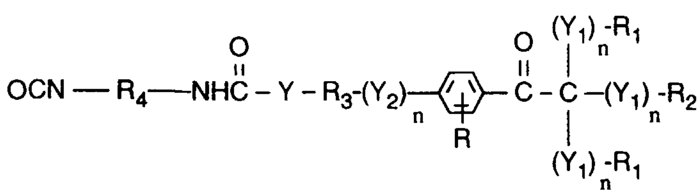

(I)

in reiner Form, worin Y O, NH oder $NR_6$ bedeutet; $Y_1$ O darstellt; $Y_2$ für -O-, -O-(O)C-, -C(O)-O- oder -O-C(O)-O- steht; die n unabhängig voneinander für 0 oder 1 stehen; R H, $C_1$-$C_{12}$-Alkyl, $C_1$-$C_{12}$-Alkoxy oder $C_1$-$C_{12}$-AlkylNH- darstellt; die $R_1$ und $R_2$ unabhängig voneinander H, lineares oder verzweigtes $C_1$-$C_8$-Alkyl, $C_1$-$C_8$-Hydroxyalkyl oder $C_6$-$C_{10}$-Aryl darstellen, oder zwei Gruppen $R_1$-$(Y_1)_n$- zusammen $-(CH_2)_x$- bedeuten, oder die Gruppen $R_1$-

$(Y_1)_n$- und $R_2$-$(Y_1)_n$- zusammen einen Rest der Formel

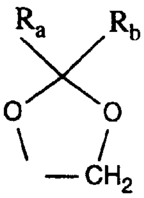

bilden; $R_3$ eine direkte Bindung oder lineares oder verzweigtes $C_1$-$C_8$-Alkylen darstellt, das unsubstituiert oder mit -OH substituiert ist und/oder gegebenenfalls mit ein oder mehreren Gruppen -O-, -O-C(O)- oder -O-C(O)-O- unterbrochen ist; $R_4$ verzweigtes $C_3$-$C_{18}$-Alkylen, mit $C_1$-$C_4$-Alkyl oder $C_1$-$C_4$-Alkoxy substituiertes $C_6$-$C_{10}$-Arylen, unsubstituiertes oder mit $C_1$-$C_4$-Alkyl oder $C_1$-$C_4$-Alkoxy substituiertes $C_7$-$C_{13}$-Aralkylen, mit $C_1$-$C_4$-Alkyl oder $C_1$-$C_4$-Alkoxy substituiertes $C_3$-$C_8$-Cycloalkylen, unsubstituiertes oder mit $C_1$-$C_4$-Alkyl oder $C_1$-$C_4$-Alkoxy substituiertes $C_3$-$C_8$-Cycloalkylen-$C_yH_{2y}$- oder mit $C_1$-$C_4$-Alkyl oder $C_1$-$C_4$-Alkoxy substituiertes -$C_yH_{2y}$-($C_3$-$C_8$-Cycloalkylen)-$C_yH_{2y}$-bedeutet; $R_6$ für lineares oder verzweigtes $C_1$-$C_6$-Alkyl steht; x ganze Zahlen von 3 bis 5 bedeutet; y ganze Zahlen von 1 bis 6 bedeutet; $R_a$ und $R_b$ unabhängig voneinander H, $C_1$-$C_8$-Alkyl, $C_3$-$C_8$-Cycloalkyl, Benzyl oder Phenyl darstellen; mit den Massgaben, dass n in den Gruppen -$(Y_1)_n$-$R_1$ für 0 steht, wenn $R_2$ H bedeutet; dass höchstens zwei $Y_1$ der -$(Y_1)_n$-Gruppen O bedeuten sowie n in den anderen -$(Y_1)_n$-Gruppen für 0 steht; dass n in der Gruppe -$(Y_2)_n$- für 0 steht, wenn $R_3$ eine direkte bindung bedeutet; und das der Rest $R_4$ den beiden NCO-Gruppen eine unterschiedliche Reaktivität verleiht.

**2.** Verbindungen gemäss Anspruch 1, dadurch gekennzeichnet, dass Y für O steht.

**3.** Verbindungen gemäss Anspruch 1, dadurch gekennzeichnet, dass $Y_2$ für O steht.

**4.** Verbindungen gemäss Anspruch 1, dadurch gekennzeichnet, dass es sich bei R um $C_1$-$C_6$-Alkyl oder $C_1$-$C_6$-Alkoxy handelt.

**5.** Verbindungen gemäss Anspruch 1, dadurch gekennzeichnet, dass es sich bei R um $C_1$-$C_4$-Alkyl oder $C_1$-$C_4$-Alkoxy handelt.

**6.** Verbindungen gemäss Anspruch 1, dadurch gekennzeichnet, dass R für H steht.

**7.** Verbindungen gemäss Anspruch 1, dadurch gekennzeichnet, dass es sich bei den Substituenten Alkyl und Alkoxy um Methyl, Ethyl, Methoxy oder Ethoxy handelt.

**8.** Verbindungen gemäss Anspruch 1, dadurch gekennzeichnet, dass $R_1$ als Alkyl linares $C_1$-$C_4$-Alkyl ist.

**9.** Verbindungen gemäss Anspruch 8, dadurch gekennzeichnet, dass $R_1$ Methyl oder Ethyl ist.

**10.** Verbindungen gemäss Anspruch 1, dadurch gekennzeichnet, dass $R_1$ als Aryl Phenyl bedeutet.

**11.** Verbindungen gemäss Anspruch 1, dadurch gekennzeichnet, dass beide Gruppen $R_1$-$(Y_1)_n$- für -$(CH_2)_x$- stehen, worin x 4 oder 5 bedeutet.

**12.** Verbindungen gemäss Anspruch 1, dadurch gekennzeichnet, dass $R_2$ für H, Methyl oder Ethyl steht.

**13.** Verbindungen gemäss Anspruch 1, dadurch gekennzeichnet, dass $R_1$ Ethyl oder Methyl oder die beiden Gruppen $R_1$-$(Y_1)_n$- zusammen Pentamethylen bedeuten, n in der Gruppe -$(Y_1)_n$-$R_2$ für 0 steht, $R_2$ Methyl oder H darstellt und R für H steht.

**14.** Verbindungen gemäss Anspruch 1, dadurch gekennzeichnet, dass in der Gruppe -$(Y_1)_n$-$R_2$ $Y_1$ für O, n für 1 und $R_2$ für H stehen.

**15.** Verbindungen gemäss Anspruch 1, dadurch gekennzeichnet, dass in der Gruppe -$(Y_1)_n$-$R_2$ $Y_1$ für O, n für 1 und $R_2$ für H stehen, und n in den Gruppen $R_1$-$(Y_1)_n$- für 0 steht.

**16.** Verbindungen gemäss Anspruch 1, dadurch gekennzeichnet, dass $R_3$ als Alkylen 1 bis 4 C-Atome enthält.

**17.** Verbindungen gemäss Anspruch 1, dadurch gekennzeichnet, dass $R_3$ Ethylen darstellt, oder $R_3$ eine direkte Bindung ist und n in der Gruppe $-(Y_2)_n-$ für 0 steht.

**18.** Verbindungen gemäss Anspruch 1, dadurch gekennzeichnet, dass $R_4$ verzweigtes $C_4$-$C_{10}$-Alkylen darstellt.

**19.** Verbindungen gemäss Anspruch 16, dadurch gekennzeichnet, dass $R_4$ 2,2-Dimethyl1,4-butylen, 2,2-Dimethyl-1,5-pentylen, 2,2,3- oder 2,2,4-trimethyl- 1,5-pentylen, 2,2-Dimethyl-1,6-hexylen, 2,2,3- oder 2,2,4- oder 2,2,5-Trimethyl-1,6-hexylen, 2,2-Dimethyl1,7-heptylen, 2,2,3- oder 2,2,4- oder 2,2,5- oder 2,2,6-Trimethyl-1,7-heptylen, 2,2-Dimethyl-1,8-octylen, 2,2,3- oder 2,2,4- oder 2,2,5- oder 2,2,6- oder 2,2,7-Trimethyl-1,8-octy-len darstellt.

**20.** Verbindungen gemäss Anspruch 1, dadurch gekennzeichnet, dass $R_4$ als Arylen in Orthostellung zur OCN-Gruppe substituiertes Phenylen ist.

**21.** Verbindungen gemäss Anspruch 1, dadurch gekennzeichnet, dass $R_4$ als Arylen 1-Methyl-2,4-phenylen, 1,5-Dimethyl-2,4-phenylen, 1-Methoxy-2,4-phenylen oder 1 -Methyl-2,7-naphthylen darstellt.

**22.** Verbindungen gemäss Anspruch 1, dadurch gekennzeichnet, dass $R_4$ als Aralkylen Phenylalkylen mit 1 bis 6 C-Atomen in der Alkylengruppe darstellt.

**23.** Verbindungen gemäss Anspruch 1, dadurch gekennzeichnet, dass $R_4$ als Cycloalkylen mit Methyl substituiertes $C_5$- oder $C_6$-Cycloalkyl darstellt.

**24.** Verbindungen gemäss Anspruch 23, dadurch gekennzeichnet, dass $R_4$ 4-Methyl-1,3-cyclopentylen, 4-Methyl-1,3-cyclohexylen, 4,4-Dimethyl-1,3-cyclohexylen, 3-Methyloder 3,3-Dimethyl-1,4-cyclohexylen, 3,5-Dimethyl-1,3-cyclohexylen, oder 2,4-Dimethyl-1,4-cyclohexylen darstellt.

**25.** Verbindungen gemäss Anspruch 1, dadurch gekennzeichnet, dass $R_4$ als Cycloalkylen-$C_yH_{2y}$- Cyclohexylen-$C_yH_{2y}$- bedeutet, das mit 1 bis 3 $C_1$-$C_4$-Alkyl substituiert ist.

**26.** Verbindungen gemäss Anspruch 25, dadurch gekennzeichnet, dass die Gruppe -$C_yH_{2y}$-Methylen bedeutet.

**27.** Verbindungen gemäss Anspruch 1, dadurch gekennzeichnet, dass $R_4$ als Cycloalkylen-$C_yH_{2y}$- Cyclopent-1-yl-3-methylen, 3-Methyl-cyclopent-1-yl-3-methylen, 3,4-Dimethyl-cyclopent-1-yl-3-methylen, 3,4,4-Trimethyl-cyclopent- 1-yl-3-methylen, Cyclohex-1-yl-3- oder -4-methylen, 3- oder 4- oder 5-Methyl-cyclohex-1-yl-3- oder -4-methylen, 3,4- oder 3,5-Dimethyl-cyclohex-1-yl-3- oder -4-methylen, 3,4,5- oder 3,4,4-oder 3,5,5-Trimethyl-cyclohex-1-yl-3- oder -4-methylen darstellt.

**28.** Verbindungen gemäss Anspruch 1, dadurch gekennzeichnet, dass $R_4$ als -$C_yH_{2y}$-Cycloalkylen-$C_yH_{2y}$- -$C_yH_{2y}$-Cyclohexylen-$C_yH_{2y}$- bedeutet, das mit 1 bis 3 $C_1$-$C_4$-Alkyl substituiert ist.

**29.** Verbindungen gemäss Anspruch 28, dadurch gekennzeichnet, dass die Gruppe -$C_yH_{2y}$-für Methylen steht.

**30.** Verbindungen gemäss Anspruch 28, dadurch gekennzeichnet, dass $R_4$ 3- oder 4- oder 5-Methyl-cyclohexan-1,3-oder -1,4-dimethylen, 3,4- oder 3,5-Dimethyl-cyclohexan-1,3-oder -1,4-dimethylen, 3,4,5- oder 3,4,4- oder 3,5,5-Trimethyl-cyclohexan-1,3- oder -1,4-dimethylen bedeutet.

**31.** Verbindungen gemäss Anspruch 1, dadurch gekennzeichnet, dass $R_4$ in $\alpha$- oder besonders $\beta$-Stellung zur OCN-Gruppe verzweigtes Alkylen oder einen in mindestens einer $\alpha$-Stellung wie definiert substituierten cyclischen Kohlenwasserstoffrest darstellt.

**32.** Verbindungen gemäss Anspruch 1, dadurch gekennzeichnet, dass es sich bei den Verbindungen der Formel I um solche handelt, worin in den Gruppen $R_1$-$(Y_1)_n$- n für 0 steht, Y, $Y_2$ und $Y_1$ in der Gruppe $R_2$-$(Y_1)_n$- je O bedeuten, n in der Gruppe $R_2$-$(Y_1)_n$- für 0 oder 1 steht, $R_1$ $C_1$-$C_4$-Alkyl oder Phenyl bedeuten oder die Gruppen $R_1$-$(Y_1)_n$-zusammen Tetramethylen oder Pentamethylen darstellen, $R_2$ $C_1$-$C_4$-Alkyl oder H darstellt, R Wasserstoff bedeutet, n in der Gruppe $-(Y_2)-_n$ für 0 oder 1 steht, $R_3$ lineares oder verzweigtes $C_2$-$C_4$-Alkylen darstellt oder eine direkte Bindung bedeutet, wobei n in der Gruppe $-(Y_2)-_n$ für 0 steht, $R_4$ verzweigtes $C_5$-$C_{10}$-Alkylen, mit 1 bis 3 Methylgruppen substituiertes Phenylen, Benzylen oder mit 1 bis 3 Methylgruppen substituiertes Benzylen, mit 1 bis 3 Methylgruppen substituiertes Cyclohexylen, Cyclohexyl-$C_yH_{2y}$- oder mit 1 bis 3 Methylgruppen substituiertes Cyclohexyl-$C_yH_{2y}$- oder -$C_yH_{2y}$-Cyclohexyl-$C_yH_{2y}$-bedeutet, und y für 1 oder 2 steht.

**33.** Verbindungen gemäss Anspruch 1, dadurch gekennzeichnet, dass es sich bei den Verbindungen der Formel I um solche handelt, worin in den Gruppen $R_1$-$(Y_1)_n$- und -$(Y_2)$-$_n$ n für 0 steht, Y, $Y_2$ und $Y_1$ in der Gruppe $R_2$-$(Y_1)_n$- je O bedeuten, n in der Gruppe $R_2$-$(Y_1)_n$- für 0 oder 1 steht, $R_1$ Methyl oder Phenyl bedeutet oder die Gruppen $R_1$-$(Y_1)_n$-zusammen Pentamethylen darstellen, $R_2$ Methyl oder H darstellt, R Wasserstoff bedeutet, n in der Gruppe -$(Y_2)$-$_n$ für 1 steht und $R_3$ Ethylen darstellt oder n in der Gruppe -$(Y_2)$-$_n$ für 0 steht und $R_3$ eine direkte Bindung bedeutet, und $R_4$ verzweigtes $C_6$-$C_{10}$-Alkylen, mit 1 bis 3 Methylgruppen substituiertes Phenylen, Benzylen oder mit 1 bis 3 Methylgruppen substituiertes Benzylen, mit 1 bis 3 Methylgruppen substituiertes Cyclohexylen, Cyclohexyl-$CH_2$- oder mit 1 bis 3 Methylgruppen substituiertes Cyclohexyl-$CH_2$- bedeutet.

**34.** Verbindungen gemäss Anspruch 1, dadurch gekennzeichnet, dass es sich um solche der Formeln

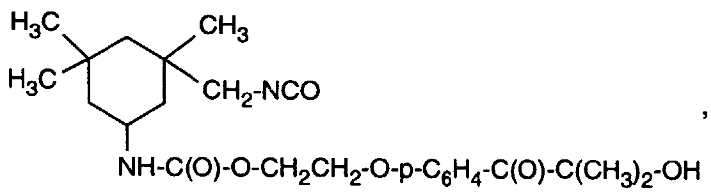

,

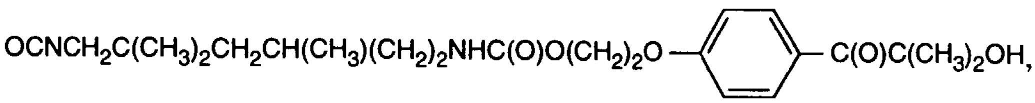

oder

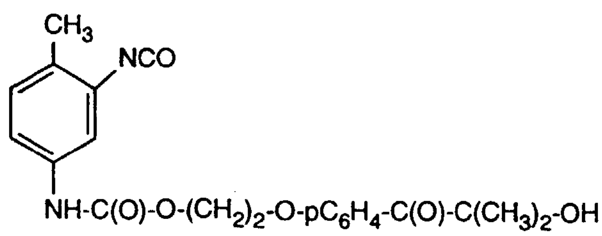

handelt.

**35.** Verfahren zur Herstellung der Verbindungen der Formel I, dadurch gekennzeichnet, dass man eine Verbindung der Formel II

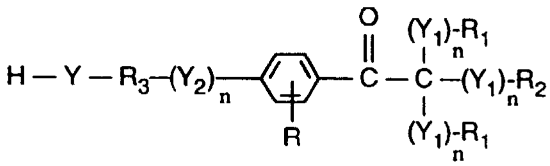

(II),

worin Y, $Y_1$, $Y_2$, R, $R_1$, $R_2$, $R_3$ und n die in Anspruch 1 angebenen Bedeutungen haben, bei einer Temperatur von bis zu 40°C in einem inerten organischen Lösungsmittel mit einem Diisocyanat der Formel III oder einem solchen

gegebenenfalls monoverkappten Diisocyanat,

$$OCN\text{-}R_4\text{-}NCO \quad (III),$$

worin $R_4$ die in Anspruch 1 angegebenen Bedeutungen hat, umsetzt.

**36.** Oligomere oder Polymere mit an das Oligomer- oder Polymerrückgrat gegebenenfalls über eine Brückengruppe gebundenen H-aktiven Gruppen -OH und/oder -NH-, oder mit im Oligomer- oder Polymerrückgrat gebundenen H-aktiven -NH-Gruppen, deren H-Atome teilweise oder vollständig durch Reste der Formel IV

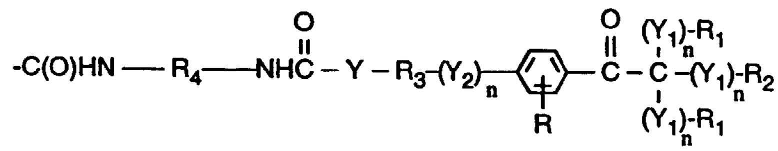

(IV),

substituiert sind, worin R, $R_1$, $R_2$, $R_3$, $R_4$, Y, $Y_1$, $Y_2$ und n die in Anspruch 1 angegebenen Bedeutungen haben, dadurch gekennzeichnet, dass es sich bei den Oligomeren oder Polymeren um (i) Cyclodextrine, Stärke, Hyaluronsäure, deacetylierte Hyaluronsäure, Chitosan, Trehalose, Cellobiose, Maltotriose, Maltohexaose, Chitohexaose, Agarose, Chitin 50, Amylose, Glucane, Heparin, Xylan, Pectin, Galactan, Glyosaminoglycane, Dextran, aminiertes Dextran, Cellulose, Hydroxyalkylcellulosen, Carboxylalkylcellulosen, Heparin, Fucoidan, Chondroinsulfat, sulfatierte Polysaccharide, Mucopolysaccharide, Gelatine, Collagen, Albumin, Globulin, Bilirubin, Ovalbumin, Keratin, Fibronectin oder Vitronectin, Pepsin, Trypsin oder Lysozym; (ii) Oligo- und Polysiloxane mit OH- oder $NH_2$-Gruppen in Endgruppen oder Seitenketten, deren H-Atome mit einem Photoinitiator gemäss Anspruch 1 substituiert sind; oder (iii) um Polyoxaalkylenoxide der Formel XIII mit gleichen oder verschiedenen wiederkehrenden Struktureinheiten $-[CH_2CH(R_{27})\text{-}O]$-handelt,

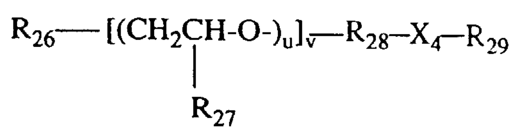

(XIII),

worin $R_{26}$ die Gruppe $R_{29}$-$X_4$- darstellt oder der v-wertige Rest eines Alkohols oder Polyols mit 1 bis 20 C-Atomen ist, $R_{27}$ H, $C_1$-$C_8$-Alkyl, vorzugsweise $C_1$-$C_4$-Alkyl und insbesondere bevorzugt Methyl bedeutet, $R_{28}$ zusammen mit $X_4$ eine direkte Bindung oder $R_{28}$ $C_2$-$C_6$-Alkylen, vorzugsweise $C_3$-$C_6$-Alkylen und insbesondere bevorzugt 1,3-Propylen darstellt, $X_4$ für -O- oder -NH- steht, $R_{29}$ einen Rest der Formel VII bedeutet, u für einen Zahlenwert von 3 bis 10 000 steht, und v eine ganze Zahl von 1 bis 6 bedeutet.

**37.** Oligomere oder Polymere gemäss Anspruch 36, dadurch gekennzeichnet, dass die Oligomeren ein mittleres Molekulargewicht von 300 bis 10 000 Dalton und die Polymeren ein mittleres Molekulargewicht von mehr als 10 000 bis 1 000 000 aufweisen.

**38.** Oligomere oder Polymere gemäss Anspruch 36, dadurch gekennzeichnet, dass dass es sich bei den Oligomeren oder Polymeren um Cyclodextrine, Stärke, Hyaluronsäure, deacetylierte Hyaluronsäure, Chitosan, Trehalose, Cellobiose, Maltotriose, Maltohexaose, Chitohexaose, Agarose, Chitin 50, Amylose, Glucane, Heparin, Xylan, Pectin, Galactan, Glyosaminoglycane, Dextran, aminiertes Dextran, Cellulose, Hydroxyalkylcellulosen, Carboxylalkylcellulosen, Heparin, Fucoidan, Chondroinsulfat, sulfatierte Polysaccharide, Mucopolysaccharide, Gelatine, Collagen, Albumin, Globulin, Bilirubin, Ovalbumin, Keratin, Fibronectin oder Vitronectin, Pepsin, Trypsin oder Lysozym handelt.

**39.** Oligomere oder Polymere gemäss Anspruch 38, dadurch gekennzeichnet, dass es sich um Cyclodextrine mit insgesamt 6 bis 8 einen Ring bildenden Glucosestruktureinheiten oder Hydroxyalkyl- oder Aminoalkylderivaten

oder Glucose- oder Maltose-substituierten Derivaten handelt, wovon mindestens eine Struktureinheit der Formel XVI entspricht,

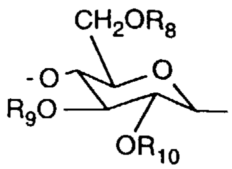

(XVI),

worin $R_8$, $R_9$ und $R_{10}$ unabhängig voneinander H, $C_1$-$C_4$-Alkyl, $C_2$-$C_6$-Acyl, $C_1$-$C_4$-Hydroxyalkyl oder $C_2$-$C_{10}$-Aminoalkyl bedeuten, und mindestens einer der Reste $R_8$, $R_9$ und $R_{10}$ einen Rest der Formel V

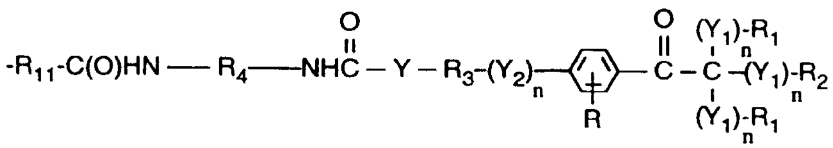

(V),

darstellen, worin R, $R_1$, $R_2$, $R_3$, $R_4$, Y, $Y_1$, $Y_2$ und n die in Anspruch 1 angegebenen Bedeutungen haben, und $R_{11}$ eine direkte Bindung, -($C_1$-$C_4$-Alkylen-O)- oder -($C_2$-$C_{10}$-Alkylen-NH)- darstellt.

**40.** Oligomere oder Polymere gemäss Anspruch 36, dadurch gekennzeichnet, dass es sich bei den Oligomeren oder Polymeren um Oligo- und Polysiloxane mit OH- oder $NH_2$-Gruppen in Endgruppen oder Seitenketten handelt, deren H-Atome mit einem Photoinitiator gemäss Anspruch 1 substituiert sind.

**41.** Oligomere oder Polymere gemäss Anspruch 40, dadurch gekennzeichnet, dass es sich um Oligomere und Polymere handelt, die

a) 5 bis 100 Mol-% Strukturelemente der Formel VI

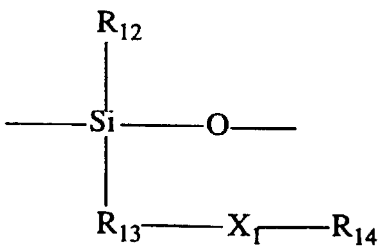

(VI),

und
b) 95 bis 0 Mol-% Strukturelemente der Formel VIa

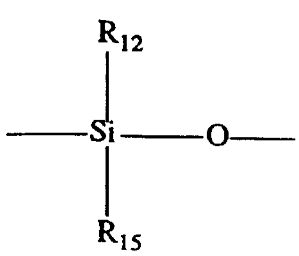

(VIa)

enthalten, bezogen auf das Oligomer oder Polymer, worin $R_{12}$ gegebenenfalls mit F substituiertes $C_1$-$C_4$-Alkyl,

Vinyl, Allyl oder Phenyl, bevorzugt Methyl darstellt, $R_{13}$ $C_2$-$C_6$-Alkylen, bevorzugt 1,3-Propylen bedeutet, $R_{15}$ die Bedeutung von $R_{12}$ hat oder -$R_{13}$-$X_1$-H oder -$R_{13}$-$X_1$-$R_{16}$-H darstellt, $X_1$ für -O- oder -NH- steht, und $R_{14}$ für einen Rest der Formel VII,

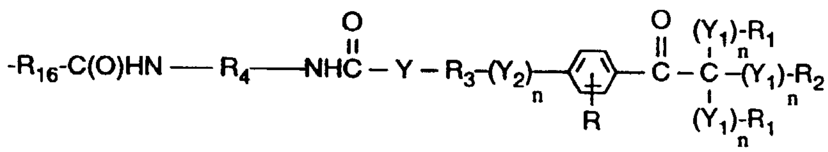

(VII),

worin R, $R_1$, $R_2$, $R_3$, $R_4$, Y, $Y_1$, $Y_2$ und n die in Anspruch 1 angegebenen Bedeutungen haben, und $R_{16}$ eine direkte Bindung oder eine Gruppe -C(O)-(CHOH)$_r$-$CH_2$-O- darstellt, worin r für 0 oder eine ganze Zahl von 1 bis 4 steht.

**42.** Oligomere oder Polymere gemäss Anspruch 40, dadurch gekennzeichnet, dass es sich um oligomere oder polymere Siloxane der Formel VIII handelt,

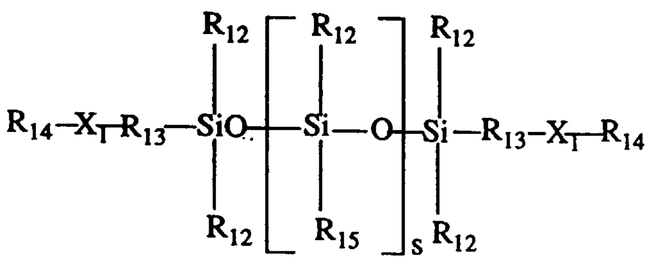

(VIII),

worin $R_{12}$ gegebenenfalls mit F substituiertes $C_1$-$C_4$-Alkyl, Vinyl, Allyl oder Phenyl, darstellt, $R_{13}$ $C_2$-$C_6$-Alkylen bedeutet, $R_{15}$ die Bedeutung von $R_{12}$ hat oder -$R_{13}$-$X_1$-H oder -$R_{13}$-$X_1$-$R_{16}$-H darstellt, $X_1$ für -O- oder -NH- steht, und $R_{14}$ für einen Rest der Formel VII

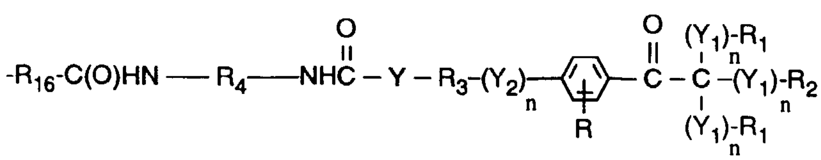

(VII),

steht, worin R, $R_1$, $R_2$, $R_3$, $R_4$, Y, $Y_1$, $Y_2$ und n die in Anspruch 1 angegebenen Bedeutungen haben, und $R_{16}$ eine direkte Bindung oder eine Gruppe -C(O)-(CHOH)$_r$-$CH_2$-O-darstellt, worin r für 0 oder eine ganze Zahl von 1 bis 4 steht.

**43.** Oligomere oder Polymere gemäss Anspruch 36, dadurch gekennzeichnet, dass es sich um Polyoxaalkylenoxide der Formel XIII mit gleichen oder verschiedenen wiederkehrenden Struktureinheiten -[$CH_2CH(R_{27})$-O]- handelt,

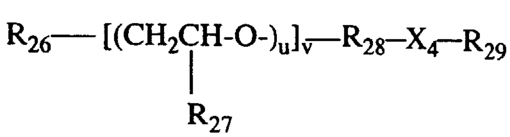

(XIII),

worin $R_{26}$ die Gruppe $R_{29}$-$X_4$- darstellt oder der v-wertige Rest eines Alkohols oder Polyols mit 1 bis 20 C-Atomen

ist, $R_{27}$ H, $C_1$-$C_8$-Alkyl, vorzugsweise $C_1$-$C_4$-Alkyl und insbesondere bevorzugt Methyl bedeutet, $R_{28}$ zusammen mit $X_4$ eine direkte Bindung oder $R_{28}$ $C_2$-$C_6$-Alkylen, vorzugsweise $C_3$-$C_6$-Alkylen und insbesondere bevorzugt 1,3-Propylen darstellt, $X_4$ für -O- oder -NH- steht, $R_{29}$ einen Rest der Formel VII bedeutet, u für einen Zahlenwert von 3 bis 10 000 steht, und v eine ganze Zahl von 1 bis 6 bedeutet.

**44.** Material aus (a) einem anorganischen oder organischen Substrat, auf dessen Oberfläche (b) als Photoinitiator mindestens eine Verbindung der Formel I, wie in Anspruch 1 definiert, gebunden ist, die über O-Atome, S-Atome, N-$C_1$-$C_6$Alkyl-Gruppen oder NH-Gruppen und die Isocyanatgruppe der Photoinitiatoren fest mit dem Substrat verbunden ist.

**45.** Material gemäss Anspruch 44 aus einem transparenten organischen Basismaterial, bei dem es sich um einen ophthalmischen Formkörper, insbesondere um eine Kontaktlinse handelt.

**46.** Material gemäss Anspruch 44, dadurch gekennzeichnet, dass es auf der Photoinitiatorschicht zusätzlich (c) eine dünne äussere Polymerschicht aufweist, die durch Aufbringen einer dünnen Schicht aus photopolymerisierbaren ethylenisch ungesättigten Substanzen auf der mit Photoinitiatorresten versehenen Substratoberfläche und Polymerisation der Schicht aus ethylenisch ungesättigten Substanzen durch Bestrahlen vorzugweise mit UV-Strahlung erhältlich ist.

**47.** Material gemäss Anspruch 46, bei dem es sich um eine Kontaktlinse handelt.

**48.** Verfahren zur Modifizierung von Oberflächen anorganischer oder organischer Substrate, die H-aktive HO-, HS-, -HN-$C_1$-$C_6$Alkyl-Gruppen oder -$NH_2$-Gruppen enthalten, umfassend die Schritte

a) Aufbringen einer dünnen Schicht aus Photoinitiatoren mindestens einer Verbindung der Formel I auf dem Substrat,
b) gegenenfalls unter Erwärmen des beschichteten Materials Binden der Photoinitiatoren und Abwaschen des überschüssigen Photoinitiators,
c) Aufbringen einer dünnen Schicht aus photopolymerisierbaren ethylenisch ungesättigten Substanzen auf der mit Photoinitiatorresten versehenen Substratoberfläche, und
d) Polymerisation der Schicht aus ethylenisch ungesättigten Substanzen durch Bestrahlen vorzugweise mit UV-Strahlung.

**49.** Kontaktlinse aus (a) einem transparenten organischen Basismaterial mit funktionellen Gruppen, und (b) einer dünnen Schicht auf der Oberfläche aus (b1) mindestens einem Photoinitiator der Formel I und (b2) einem Pfropfpolymer, gebildet durch Photopolymerisation eines nichtflüchtigen oder schwerflüchtigen Olefins.

**50.** Kontaktlinse enthaltend ein Oligomer oder Polymer mit an das Oligomer- oder Polymerrückgrat gegebenenfalls über eine Brückengruppe gebundenen H-aktiven Gruppen -OH und/oder -NH-, oder mit im Oligomer- oder Polymerrückgrat gebundenen H-aktiven -NH-Gruppen, deren H-Atome teilweise oder vollständig durch Reste der Formel IV

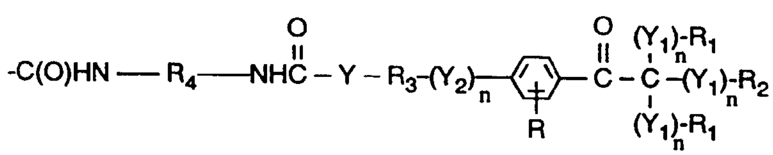

(IV),

substituiert sind, worin R, $R_1$, $R_2$, $R_3$, $R_4$, Y, $Y_1$, $Y_2$ und n die in Anspruch 1 angegebenen Bedeutungen haben, und eine dünne äussere Schicht auf mindestens einem Teil der Oberfläche aus einem Pfropfpolymer, gebildet durch Photopolymerisation eines nichtflüchtigen oder schwerflüchtigen Olefins.

**51.** Kontaktlinse gemäss Anspruch 50, dadurch gekennzeichnet, dass die Oligomeren ein mittleres Molekulargewicht von 300 bis 10 000 Dalton und die Polymeren ein mittleres Molekulargewicht von mehr als 10 000 bis 1 000 000 aufweisen.

**52.** Kontaktlinse gemäss Anspruch 50, dadurch gekennzeichnet, dass es sich bei dem Oligomer oder Polymer um ein

natürliches oder synthetisches Oligomer handelt.

**53.** Kontaktlinse gemäss Anspruch 50, dadurch gekennzeichnet, dass es sich bei dem Oligomer oder Polymer um ein Oligomer oder Polymer gemäss Anspruch 38 handelt.

**54.** Kontaktlinse gemäss Anspruch 50, dadurch gekennzeichnet, dass es sich bei dem Oligomer oder Polymer um verseifte Polymerisate von Vinylestern oder -ethern (Polyvinylalkohol) hydroxylierte Polydiolefine; Polyacrylsäure, Polymethacrylsäure, Polyacrylate, Polymethacrylate, Polyacrylamide oder Polymethacrylamide mit Hydroxyalkyl- oder Aminoalkylresten in der Estergruppe oder Amidgruppe; Polysiloxane mit Hydroxyalkyl- oder Aminoalkylgruppen; Polyether aus Epoxiden oder Glycidylverbindungen und Diolen; Polyvinylphenole oder Copolymere von Vinylphenol und olefinischen Comonomeren; Copolymerisate von mindestens einem Monomer aus der Gruppe Vinylalkohol, Hydroxyalkyl oder Aminoalkyl enthaltenden Acrylaten, Methacrylaten, Acrylamid, Methacrylamid, Acrylsäure, Methacrylsäure, hydroxylierten Diolefinen mit ethylenisch ungesättigten Comonomeren; oder um Polyoxaalkylene mit endständigen OH- oder Aminoalkyloxygruppen handelt.

**55.** Kontaktlinse gemäss Anspruch 50, dadurch gekennzeichnet, dass es sich bei dem Oligomer oder Polymer um ein Oligomer oder Polymer gemäss Anspruch 39 handelt.

**56.** Kontaktlinse gemäss Anspruch 50, dadurch gekennzeichnet, dass es sich bei dem Oligomer oder Polymer um ein Oligomer oder Polymer gemäss Anspruch 40 handelt.

**57.** Kontaktlinse gemäss Anspruch 50, dadurch gekennzeichnet, dass es sich bei dem Oligomer oder Polymer um ein Oligomer oder Polymer gemäss Anspruch 41 handelt.

**58.** Kontaktlinse gemäss Anspruch 50, dadurch gekennzeichnet, dass es sich bei dem Oligomer oder Polymer um ein Oligomer oder Polymer gemäss Anspruch 42 handelt.

**59.** Kontaktlinse gemäss Anspruch 54, dadurch gekennzeichnet, dass es sich bei dem Oligomer oder Polymer um Oligomere und Polymere handelt, welche

a) 5 bis 100 Mol-% Struktureinheiten der Formel IX

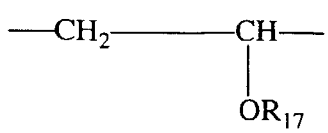

(IX),

und
b) 95 bis 0 Mol-% Struktureinheiten der Formel X

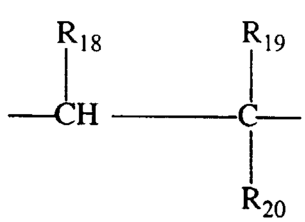

(X)

enthalten, worin $R_{17}$ einen Rest der Formel V

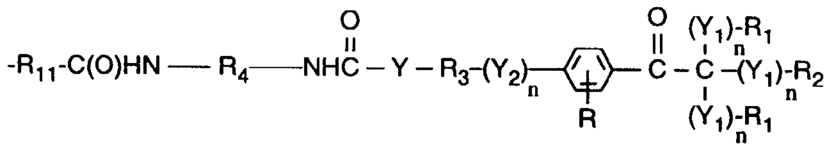 (V),

darstellt, worin R, $R_1$, $R_2$, $R_3$, $R_4$, Y, $Y_1$, $Y_2$ und n die in Anspruch 1 angegebenen Bedeutungen haben, und $R_{11}$ eine direkte Bindung, -($C_1$-$C_4$-Alkylen-O)- oder -($C_2$-$C_{10}$-Alkylen-NH)- darstellt; $R_{18}$ für H, $C_1$-$C_6$-Alkyl, -$COOR_{21}$ oder -$COO^{\ominus}$ steht, $R_{19}$ H, F, Cl, CN oder $C_1$-$C_6$-Alkyl bedeutet, und $R_{20}$ H, OH, $R_{11}$-H, F, Cl, CN, $R_{21}$-O-, $C_1$-$C_{12}$-Alkyl, -$COO^{\ominus}$, -$COOR_{21}$, -OCO-$R_{21}$, Methylphenyl oder Phenyl darstellt, wobei $R_{21}$ $C_1$-$C_{18}$-Alkyl, $C_5$-$C_7$-Cycloalkyl, ($C_1$-$C_{12}$-Alkyl)-$C_5$-$C_7$-cycloalkyl, Phenyl, ($C_1$-$C_{12}$-Alkyl)phenyl, Benzyl oder ($C_1$-$C_{12}$-Alkyl)benzyl darstellt.

**60.** Kontaktlinse gemäss Anspruch 54, dadurch gekennzeichnet, dass es sich bei dem Oligomer oder Polymer um Oligomere und Polymere handelt, welche

a) 5 bis 100 Mol-% Struktureinheiten der Formel XI

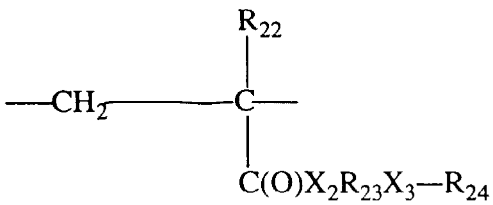 (XI),

und

b) 95 bis 0 Mol-% Struktureinheiten der Formel XII

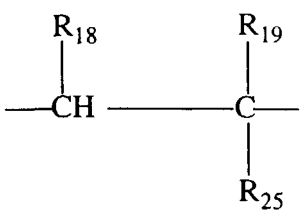 (XII)

enthalten, worin $R_{22}$ H oder Methyl bedeutet, $X_2$ und $X_3$ unabhängig voneinander -O- oder -NH- darstellen, $R_{23}$ für -$(CH_2)_c$- steht und c ganze Zahlen von 2 bis 12, vorzugsweise 2 bis 6 bedeutet, $R_{24}$ einen Rest der Formel VII darstellt, $R_{18}$ und $R_{19}$ die in Anspruch 59 angegebenen Bedeutungen haben, und $R_{25}$ die gleiche Bedeutung wie $R_{20}$ hat oder -$C(O)X_2R_{23}X_3H$ bedeutet.

**61.** Kontaktlinse gemäss Anspruch 50, dadurch gekennzeichnet, dass es sich bei dem Oligomer oder Polymer um ein Oligomer oder Polymer gemäss Anspruch 43 handelt.

## Claims

**1.** A compound of the formula

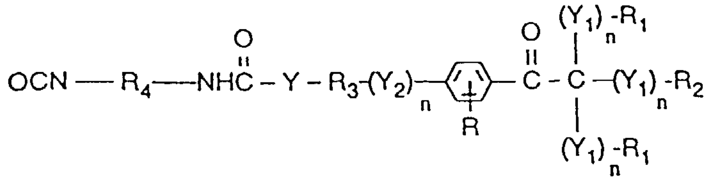 (I)

in pure form, in which

Y is O, NH or $NR_6$;
$Y_1$ is O;
$Y_2$ is -O-, -O-(O)C-, -C(O)-O- or -O-C(O)-O-;
the n indices, independently of one another, are 0 or 1;
R is H, $C_1$-$C_{12}$alkyl, $C_1$-$C_{12}$alkoxy or $C_1$-$C_{12}$alkyl-NH-;
the $R_1$ and $R_2$ radicals, independently of one another, are H, linear or branched $C_1$-$C_8$alkyl, $C_1$-$C_8$hydroxyalkyl or $C_6$-$C_{10}$aryl, or two groups $R_1$-$(Y_1)_n$- together are $-(CH_2)_x-$, or the $R_1$-$(Y_1)_n$- and $R_2$-$(Y_1)_n$- groups together form a radical of the formula

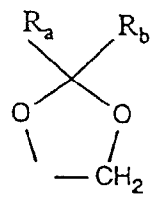 ;

$R_3$ is a direct bond or linear or branched $C_1$-$C_8$alkylene, which is unsubstituted or substituted by -OH and/or is uninterrupted or interrupted by one or more -O-, -O-C(O)- or -O-C(O)-O-groups;
$R_4$ is branched $C_3$-$C_{18}$alkylene, $C_6$-$C_{10}$arylene substituted by $C_1$-$C_4$alkyl or by $C_1$-$C_4$alkoxy, $C_7$-$C_{18}$aralkylene which is unsubstituted or substituted by $C_1$-$C_4$alkyl or by $C_1$-$C_4$alkoxy, $C_3$-$C_8$cycloalkylene substituted by $C_1$-$C_4$alkyl or by $C_1$-$C_4$alkoxy, $C_3$-$C_8$cycloalkylene-$C_yH_{2y}$-which is unsubstituted or substituted by $C_1$-$C_4$alkyl or by $C_1$-$C_4$alkoxy, or $-CyH_{2y}-(C_3$-$C_8$-cycloalkylene)-$C_yH_{2y}$- substituted by $C_1$-$C_4$alkyl or by $C_1$-$C_4$alkoxy;
$R_6$ is linear or branched $C_1$-$C_6$alkyl;
x is an integer from 3 to 5;
y is an integer from 1 to 6; and
$R_a$ and $R_b$, independently of one another, are H, $C_1$-$C_8$alkyl, $C_3$-$C_8$cycloalkyl, benzyl or phenyl;

with the provisos that n in the $-(Y_1)_n-R_1$ groups is 0 if $R_2$ is H; that at most two $Y_1$ radicals in the $-(Y_1)_n-$ groups are O and n in the other $-(Y_1)_n-$ groups is 0; that n in the $-(Y_2)_n-$ group is 0 if $R_3$ is a direct bond; and that the $R_4$ radical gives the two NCO groups different reactivities.

**2.** A compound according to claim 1, wherein Y is O.

**3.** A compound according to claim 1, wherein $Y_2$ is O.

**4.** A compound according to claim 1, wherein R is $C_1$-$C_6$alkyl or $C_1$-$C_6$alkoxy.

**5.** A compound according to claim 1, wherein R is $C_1$-$C_4$alkyl or $C_1$-$C_4$alkoxy.

**6.** A compound according to claim 1, wherein R is H.

**7.** A compound according to claim 1, wherein the alkyl and alkoxy substituents are methyl, ethyl, methoxy or ethoxy.

**8.** A compound according to claim 1, wherein alkyl $R_1$ is linear $C_1$-$C_4$alkyl.

**9.** A compound according to claim 8, wherein $R_1$ is methyl or ethyl.

**10.** A compound according to claim 1, wherein aryl $R_1$ is phenyl.

**11.** A compound according to claim 1, wherein both $R_1$-$(Y_1)_n$- groups are $-(CH_2)_x-$, in which x is 4 or 5.

**12.** A compound according to claim 1, wherein $R_2$ is H, methyl or ethyl.

**13.** A compound according to claim 1, wherein $R_1$ is ethyl or methyl or the two $R_1$-$(Y_1)_n$-groups together are pentamethylene, n in the -$(Y_1)_n$-$R_2$ group is 0, $R_2$ is methyl or H, and R is H.

**14.** A compound according to claim 1, wherein, in the -$(Y_1)_n$-$R_2$ group, $Y_1$ is O, n is 1 and $R_2$ is H.

**15.** A compound according to claim 1, wherein, in the -$(Y_1)_n$-$R_2$ group, $Y_1$ is O, n is 1 and $R_2$ is H, and n in the $R_1$-$(Y_1)_n$- groups is 0.

**16.** A compound according to claim 1, wherein alkylene $R_3$ contains from 1 to 4 carbon atoms.

**17.** A compound according to claim 1, wherein $R_3$ is ethylene, or $R_3$ is a direct bond and n in the -$(Y_2)_n$- group is 0.

**18.** A compound according to claim 1, wherein $R_4$ is branched $C_4$-$C_{10}$alkylene.

**19.** A compound according to claim 16, wherein $R_4$ is 2,2-dimethyl-1,4-butylene, 2,2-dimethyl-1,5-pentylene, 2,2,3- or 2,2,4-trimethyl-1,5-pentylene, 2,2-dimethyl-1,6-hexylene, 2,2,3- or 2,2,4- or 2,2,5-trimethyl-1,6-hexylene, 2,2-dimethyl-1,7-heptylene, 2,2,3- or 2,2,4-or 2,2,5- or 2,2,6-trimethyl-1,7-heptylene, 2,2-dimethyl-1,8-octylene, or 2,2,3- or 2,2,4- or 2,2,5- or 2,2,6- or 2,2,7-trimethyl-1,8-octylene.

**20.** A compound according to claim 1, wherein arylene $R_4$ is phenylene substituted in the ortho-position to the OCN group.

**21.** A compound according to claim 1, wherein arylene $R_4$ is 1-methyl-2,4-phenylene, 1,5-dimethyl-2,4-phenylene, 1-methoxy-2,4-phenylene or 1-methyl-2,7-naphthylene.

**22.** A compound according to claim 1, wherein aralkylene $R_4$ is phenylalkylene having from 1 to 6 carbon atoms in the alkylene group.

**23.** A compound according to claim 1, wherein cycloalkylene $R_4$ is methyl-substituted $C_5$- or $C_6$-cycloalkyl.

**24.** A compound according to claim 23, wherein $R_4$ is 4-methyl-1,3-cyclopentylene, 4-methyl-1,3-cyclohexylene, 4,4-dimethyl-1,3-cyclohexylene, 3-methyl- or 3,3-dimethyl-1,4-cyclohexylene, 3,5-dimethyl-1,3-cyclohexylene or 2,4-dimethyl-1,4-cyclohexylene.

**25.** A compound according to claim 1, wherein cycloalkylene-$C_yH_{2y}$- $R_4$ is cyclohexylene-$C_yH_{2y}$- substituted by from 1 to 3 $C_1$-$C_4$alkyl groups.

**26.** A compound according to claim 25, wherein the -$C_yH_{2y}$- group is methylene.

**27.** A compound according to claim 1, wherein cycloalkylene-$C_yH_{2y}$- $R_4$ is cyclopent-1-yl-3-methylene, 3-methylcyclopent-1-yl-3-methylene, 3,4-dimethylcyclopent-1-yl-3-methylene, 3,4,4-tnmethylcyclopent-1-yl-3-methylene, cyclohex-1-yl-3- or -4-methylene, 3- or 4- or 5-methylcyclohex-1-yl-3- or -4-methylene, 3,4- or 3,5-dimethylcyclohex-1-yl-3- or -4-methylene, or 3,4,5- or 3,4,4- or 3,5,5-trimethylcyclohex-1-yl-3- or -4-methylene.

**28.** A compound according to claim 1, wherein -$C_yH_{2y}$-cycloalkylene-$C_yH_{2y}$- $R_4$ is -$C_yH_{2y}$-cyclohexylene-$C_yH_{2y}$- substituted by from 1 to 3 $C_1$-$C_4$alkyl groups.

**29.** A compound according to claim 28, wherein the -$C_yH_{2y}$- group is methylene.

**30.** A compound according to claim 28, wherein $R_4$ is 3- or 4- or 5-methylcyclohexane-1,3-or -1,4-dimethylene, 3,4- or 3,5-dimethylcyclohexane-1,3- or -1,4-dimethylene, or 3,4,5- or 3,4,4- or 3,5,5-trimethylcyclohexane-1,3- or -1,4-dimethylene.

**31.** A compound according to claim 1, wherein $R_4$ is alkylene which is branched in the α-position or, especially, in the β-position to the OCN group, or is a cyclic hydrocarbon radical substituted in at least one α-position as defined.

**32.** A compound according to claim 1, wherein the compound of formula I is one in which n in the $R_1$-$(Y_1)_n$- groups is 0, Y, $Y_2$ and $Y_1$ in the $R_2$-$(Y_1)_n$- group are each O, n in the $R_2$-$(Y_1)_n$-group is 0 or 1, $R_1$ is $C_1$-$C_4$alkyl or phenyl, or the $R_1$-$(Y_1)_n$- groups together are tetramethylene or pentamethylene, $R_2$ is $C_1$-$C_4$alkyl or H, R is hydrogen, n in the -$(Y_2)$-$_n$ group is 0 or 1, $R_3$ is linear or branched $C_2$-$C_4$alkylene or a direct bond, in which case n in the -$(Y_2)$-$_n$ group is 0, $R_4$ is branched $C_5$-$C_{10}$alkylene, phenylene substituted by from 1 to 3 methyl groups, benzylene or benzylene substituted by from 1 to 3 methyl groups, cyclohexylene substituted by from 1 to 3 methyl groups, cyclohexyl-$C_yH_{2y}$-, or cyclohexyl-$C_yH_{2y}$- or -$C_yH_{2y}$-cyclohexyl-$C_yH_{2y}$- each substituted by from 1 to 3 methyl groups, and y is 1 or 2.

**33.** A compound according to claim 1, wherein the compound of formula I is one in which n in the $R_1$-$(Y_1)_n$- and -$(Y_2)$-$_n$ groups is 0, Y, $Y_2$ and $Y_1$ in the $R_2$-$(Y_1)_n$- group are each O, n in the $R_2$-$(Y_1)_n$- group is 0 or 1, $R_1$ is methyl or phenyl, or the $R_1$-$(Y_1)_n$- groups together are pentamethylene, $R_2$ is methyl or H, R is hydrogen, n in the -$(Y_2)$-$_n$ group is 1 and $R_3$ is ethylene, or n in the -$(Y_2)$-$_n$ group is 0 and $R_3$ is a direct bond, and $R_4$ is branched $C_6$-$C_{10}$-alkylene, phenylene substituted by from 1 to 3 methyl groups, benzylene or benzylene substituted by from 1 to 3 methyl groups, cyclohexylene substituted by from 1 to 3 methyl groups, or cyclohexyl-$CH_2$- or cyclohexyl-$CH_2$- substituted by from 1 to 3 methyl groups.

**34.** A compound according to claim 1 having the formula

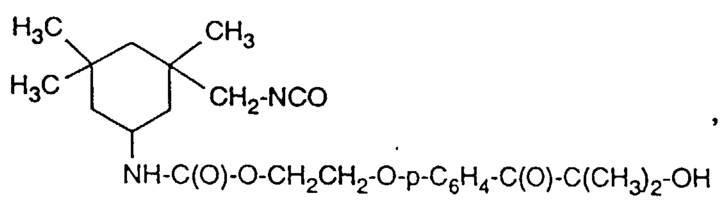

,

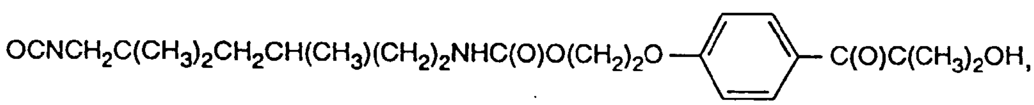

or

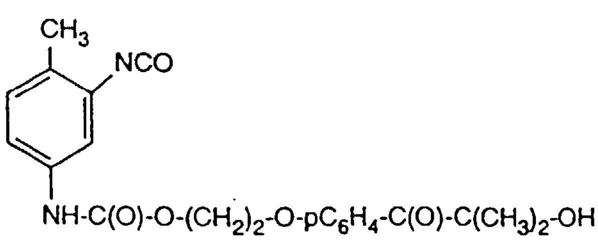

**35.** A process for the preparation of a compound of formula I, which comprises reacting a compound of formula II

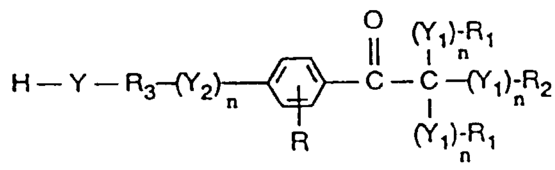 (II),

in which Y, $Y_1$, $Y_2$, R, $R_1$, $R_2$, $R_3$ and n are as defined in claim 1, with a diisocyanate of formula III

$$OCN\text{-}R_4\text{-}NCO \qquad (III),$$

in which $R_4$ is as defined in claim 1, or with such a diisocyanate mono-masked where necessary, in an inert organic solvent, at a temperature of up to 40°C.

**36.** An oligomer or polymer containing H-active groups -OH and/or -NH- bonded to the oligomer or polymer backbone, optionally via a bridge group, or containing H-active -NH-groups bonded in the oligomer or polymer backbone, some or all of the hydrogen atoms of which groups have been replaced by radicals of formula IV

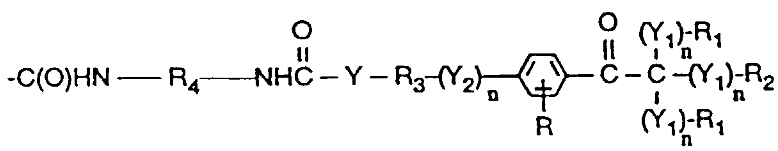 (IV)

in which R, $R_1$, $R_2$, $R_3$, $R_4$, Y, $Y_1$, $Y_2$ and n are as defined in claim 1, wherein the oligomer or polymer is (i) a cyclodextrin, starch, hyaluronic acid, deacetylated hyaluronic acid, chitosan, trehalose, cellobiose, maltotriose, maltohexaose, chitohexaose, agarose, chitin 50, amylose, glucan, heparin, xylan, pectin, galactan, glycosaminoglycan, dextran, aminated dextran, cellulose, a hydroxyalkylcellulose, a carboxyalkylcellulose, heparin, fucoidan, chondroin sulfate, a sulfated polysaccharide, a mucopolysaccharide, gelatin, collagen, albumin, globulin, bilirubin, ovalbumin, keratin, fibronectin or vitronectin, pepsin, trypsin or lysozyme; (ii) an oligo- or poly-siloxane containing, in terminal groups or side chains, OH or $NH_2$ groups whose hydrogen atoms have been replaced by a photoinitiator according to claim 1; or (iii) a polyoxaalkylene oxide of formula XIII containing identical or different structural repeating units $-[CH_2CH(R_{27})\text{-}O]-$

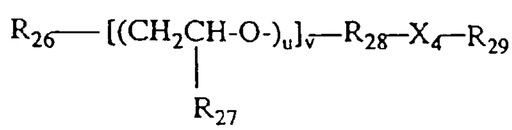 (XIII)

in which

$R_{26}$ is a $R_{29}$-$X_4$- group or the v-valent radical of an alcohol or polyol having from 1 to 20 carbon atoms,
$R_{27}$ is H, $C_1$-$C_8$alkyl, preferably $C_1$-$C_4$alkyl and especially methyl,
$R_{28}$ together with $X_4$ is a direct bond, or
$R_{28}$ is $C_2$-$C_6$alkylene, preferably $C_3$-$C_6$alkylene and especially 1,3-propylene, and $X_4$ is -O- or -NH-,
$R_{29}$ is a radical of formula VII,
u has a numerical value of from 3 to 10 000, and
v is an integer from 1 to 6.

**37.** An oligomer or polymer according to claim 36, wherein the oligomer has a mean molecular weight of from 300 to 10 000 daltons and the polymer has a mean molecular weight of from more than 10 000 to 1 000 000.

**38.** An oligomer or polymer according to claim 36, which is a cyclodextrin, starch, hyaluronic acid, deacetylated hyaluronic acid, chitosan, trehalose, cellobiose, maltotriose, maltohexaose, chitohexaose, agarose, chitin 50, amylose, glucan, heparin, xylan, pectin, galactan, glycosaminoglycan, dextran, aminated dextran, cellulose, a hydroxyalkylcellulose, a carboxyalkylcellulose, heparin, fucoidan, chondroin sulfate, a sulfated polysaccharide, a mucopolysaccharide, gelatin, collagen, albumin, globulin, bilirubin, ovalbumin, keratin, fibronectin or vitronectin, pepsin, trypsin or lysozyme.

**39.** An oligomer or polymer according to claim 38, which is a cyclodextrin containing a total of from 6 to 8 ring-configured glucose structural units or a hydroxyalkyl or aminoalkyl derivative or a glucose- or maltose-substituted derivative in which at least one structural unit corresponds to formula XVI

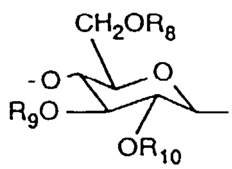

(XVI)

in which

$R_8$, $R_9$ and $R_{10}$, independently of one another, are H, $C_1$-$C_4$alkyl, $C_2$-$C_6$acyl, $C_1$-$C_4$hydroxyalkyl or $C_2$-$C_{10}$aminoalkyl and at least one of the radicals $R_8$, $R_9$ and $R_{10}$ is a radical of formula V

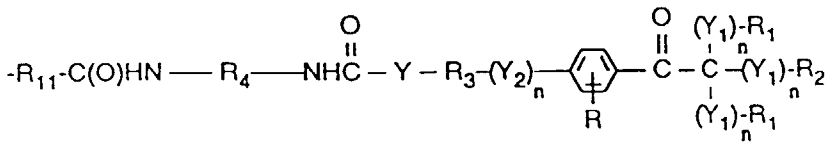

(V)

in which

R, $R_1$, $R_2$, $R_3$, $R_4$, Y, $Y_1$, $Y_2$ and n are as defined in claim 1, and
$R_{11}$ is a direct bond, -($C_1$-$C_4$alkylene-O)- or -($C_2$-$C_{10}$alkylene-NH)-.

**40.** An oligomer or polymer according to claim 36, which is an oligo- or poly-siloxane containing, in terminal groups or side chains, OH or $NH_2$ groups whose hydrogen atoms have been replaced by a photoinitiator according to claim 1.

**41.** An oligomer or polymer according to claim 40, which is an oligomer or polymer comprising

a) from 5 to 100 mol % of structural units of formula VI

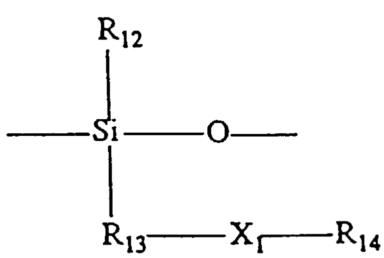

(VI)

and
b) from 95 to 0 mol % of structural units of formula VIa

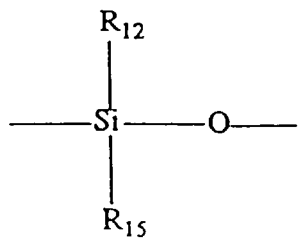

(VIa),

based on the oligomer or polymer, in which

$R_{12}$ is unsubstituted or F-substituted $C_1$-$C_4$alkyl, vinyl, allyl or phenyl, preferably methyl,
$R_{13}$ is $C_2$-$C_6$alkylene, preferably 1,3-propylene,
$R_{15}$ has the meaning of $R_{12}$ or is $-R_{13}-X_1-H$ or $-R_{13}-X_1-R_{16}-H$,
$X_1$ is -O- or -NH-, and
$R_{14}$ is a radical of formula VII

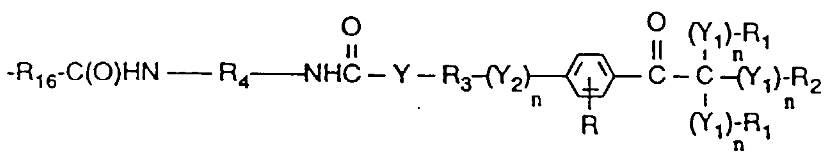

(VII)

in which

R, $R_1$, $R_2$, $R_3$, $R_4$, Y, $Y_1$, $Y_2$ and n are as defined in claim 1, and
$R_{16}$ is a direct bond or a $-C(O)-(CHOH)_r-CH_2-O-$ group in which r is 0 or an integer from 1 to 4.

**42.** An oligomer or polymer according to claim 40, which is an oligomeric or polymeric siloxane of formula VIII

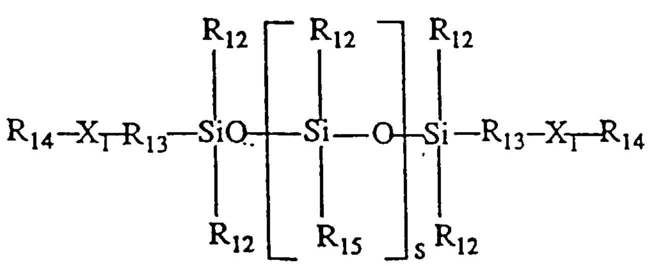

(VIII)

in which

$R_{12}$ is unsubstituted or F-substituted $C_1$-$C_4$alkyl, vinyl, allyl or phenyl,
$R_{13}$ is $C_2$-$C_6$alkylene,
$R_{15}$ has the meaning of $R_{12}$ or is $-R_{13}-X_1-H$ or $-R_{13}-X_1-R_{16}-H$,
$X_1$ is -O- or -NH-, and
$R_{14}$ is a radical of formula VII

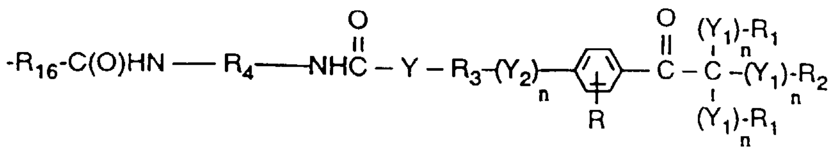 (VII)

in which

R, $R_1$, $R_2$, $R_3$, $R_4$, Y, $Y_1$, $Y_2$ and n are as defined in claim 1, and
$R_{16}$ is a direct bond or a -C(O)-(CHOH)$_r$-$CH_2$-O- group in which r is 0 or an integer from 1 to 4.

**43.** An oligomer or polymer according to claim 36, which is a polyoxaalkylene oxide of formula XIII containing identical or different structural repeating units -[$CH_2CH(R_{27})$-O]-

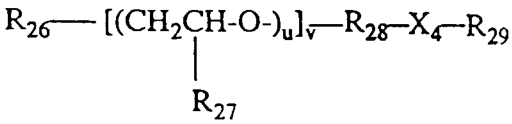 (XIII)

in which

$R_{26}$ is a $R_{29}$-$X_4$- group or the v-valent radical of an alcohol or polyol having from 1 to 20 carbon atoms,
$R_{27}$ is H, $C_1$-$C_8$alkyl, preferably $C_1$-$C_4$alkyl and especially methyl,
$R_{28}$ together with $X_4$ is a direct bond, or
$R_{28}$ is $C_2$-$C_6$alkylene, preferably $C_3$-$C_6$alkylene and especially 1,3-propylene, and $X_4$ is -O- or -NH-,
$R_{29}$ is a radical of formula VII,
u has a numerical value of from 3 to 10 000, and
v is an integer from 1 to 6.

**44.** A material comprising (a) an inorganic or organic substrate to whose surface there is bonded (b) as photoinitiator, at least one compound of formula I as defined in claim 1, which is firmly bonded to the substrate *via* oxygen atoms, sulfur atoms, N-$C_1$-$C_6$alkyl groups or NH groups and the isocyanate group of the photoinitiator.

**45.** A material according to claim 44 comprising a transparent, organic base material, which is an ophthalmic moulding, in particular a contact lens.

**46.** A material according to claim 44, wherein the photoinitiator coating is additionally coated by (c) a thin, extemal polymer coating obtainable by applying a thin coating of photopolymerisable, ethylenically unsaturated substances to the substrate surface provided with photoinitiator radicals, and polymerising the coating of ethylenically unsaturated substances by irradiation, preferably with UV radiation.

**47.** A material according to claim 46, which is a contact lens.

**48.** A process for modifying surfaces of inorganic or organic substrates containing H-active HO-, HS-, -HN-$C_1$-$C_6$alkyl or -$NH_2$ groups, comprising the steps

a) application of a thin coating of photoinitiators of at least one compound of formula I to the substrate,
b) binding the photoinitiators, if necessary with warming of the coated material, and washing off the excess photoinitiator,
c) application of a thin coating of photopolymerisable, ethylenically unsaturated substances to the substrate surface provided with photoinitiator radicals, and
d) polymerisation of the coating of ethylenically unsaturated substances by irradiation, preferably with UV radiation.

**49.** A contact lens comprising (a) a transparent, organic base material containing functional groups, and (b) a thin surface coating comprising (b1) at least one photoinitiator of formula I and (b2) a graft polymer formed by photopolymerisation of a non-volatile or low-volatility olefin.

**50.** A contact lens comprising an oligomer or polymer containing H-active groups -OH and/or -NH- bonded to the oligomer or polymer backbone, optionally *via* a bridge group, or containing H-active -NH- groups bonded in the oligomer or polymer backbone, some or all of the hydrogen atoms of which groups have been replaced by radicals of formula IV

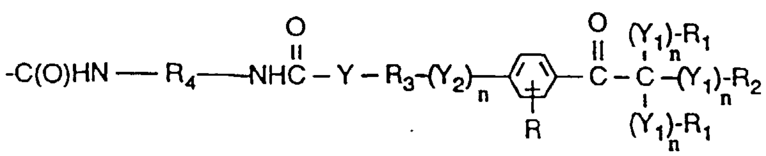 (IV)

in which R, $R_1$, $R_2$, $R_3$, $R_4$, Y, $Y_1$, $Y_2$ and n are as defined in claim 1, and a thin outer coating, on at least part of the surface, of a graft polymer formed by photopolymerisation of a non-volatile or low-volatility olefin.

**51.** A contact lens according to claim 50, wherein the oligomer has a mean molecular weight of from 300 to 10 000 daltons and the polymer has a mean molecular weight of from more than 10 000 to 1 000 000.

**52.** A contact lens according to claim 50, wherein the oligomer or polymer is a natural or synthetic oligomer.

**53.** A contact lens according to claim 50, wherein the oligomer or polymer is an oligomer or polymer according to claim 38.

**54.** A contact lens according to claim 50, wherein the oligomer or polymer is a hydrolysed polymer of a vinyl ester or ether (polyvinyl alcohol), a hydroxylated polydiolefin; polyacrylic acid, polymethacrylic acid or a polyacrylate, polymethacrylate, a polyacrylamide or a polymethacrylamide containing hydroxyalkyl or aminoalkyl radicals in the ester group or amide group; a polysiloxane containing hydroxyalkyl or aminoalkyl groups; a polyether made from an epoxide or glycidyl compound and a diol; a polyvinylphenol or a copolymer of vinylphenol and an olefinic comonomer; or a copolymer of at least one monomer from the group vinyl alcohol, hydroxyalkyl- or aminoalkyl-containing acrylates and methacrylates, acrylamide, methacrylamide, acrylic acid, methacrylic acid, and hydroxylated diolefins with an ethylenically unsaturated comonomer; or a polyoxaalkylene containing terminal OH or aminoalkoxy groups.

**55.** A contact lens according to claim 50, wherein the oligomer or polymer is an oligomer or polymer according to claim 39.

**56.** A contact lens according to claim 50, wherein the oligomer or polymer is an oligomer or polymer according to claim 40.

**57.** A contact lens according to claim 50, wherein the oligomer or polymer is an oligomer or polymer according to claim 41.

**58.** A contact lens according to claim 50, wherein the oligomer or polymer is an oligomer or polymer according to claim 42.

**59.** A contact lens according to claim 54, wherein the oligomer or polymer is an oligomer or polymer comprising

a) from 5 to 100 mol % of structural units of formula IX

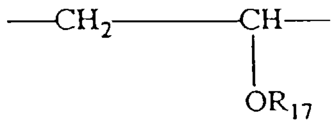

(IX)

and

b) from 95 to 0 mol % of structural units of formula X

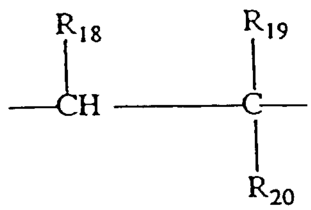

(X),

in which $R_{17}$ is a radical of formula V

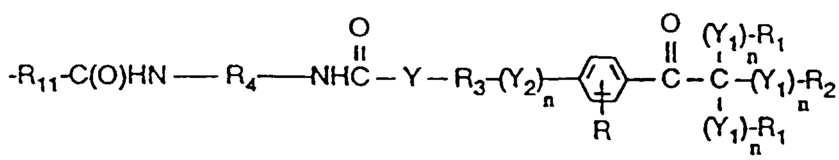

(V)

in which

$R$, $R_1$, $R_2$, $R_3$, $R_4$, $Y$, $Y_1$, $Y_2$ and n are as defined in claim 1,
$R_{11}$ is a direct bond, -($C_1$-$C_4$alkylene-O)- or -($C_2$-$C_{10}$alkylene-NH)-,
$R_{18}$ is H, $C_1$-$C_6$alkyl, -$COOR_{21}$ or -$COO^{\ominus}$ ,
$R_{19}$ is H, F, Cl, CN or $C_1$-$C_6$alkyl, and
$R_{20}$ is H, OH, $R_{11}$-H, F, Cl, CN, $R_{21}$-O-, $C_1$-$C_{12}$alkyl, -$COO^{\ominus}$, -$COOR_{21}$, -OCO-$R_{21}$, methylphenyl or phenyl, wherein $R_{21}$ is $C_1$-$C_{18}$alkyl, $C_5$-$C_7$cycloalkyl, ($C_1$-$C_{12}$alkyl)-$C_5$-$C_7$cycloalkyl, phenyl, ($C_1$-$C_{12}$alkyl)phenyl, benzyl or ($C_1$-$C_{12}$alkyl)benzyl.

**60.** A contact lens according to claim 54, wherein the oligomer or polymer is an oligomer or polymer comprising

a) from 5 to 100 mol % of structural units of formula XI

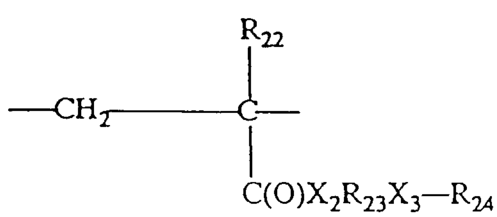

(XI)

and

b) from 95 to 0 mol % of structural units of formula XII

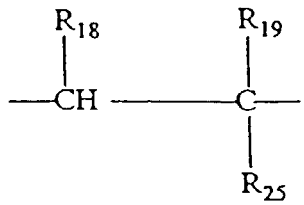

(XII),

in which

$R_{22}$ is H or methyl,
$X_2$ and $X_3$, independently of one another, are -O- or -NH-,
$R_{23}$ is $-(CH_2)_c-$ and c is an integer from 2 to 12, preferably from 2 to 6,
$R_{24}$ is a radical of formula VII,
$R_{18}$ and $R_{19}$ are as defined in claim 59, and
$R_{25}$ has the same meaning as $R_{20}$ or is $-C(O)X_2R_{23}X_3H$.

**61.** A contact lens according to claim 50, wherein the oligomer or polymer is an oligomer or polymer according to claim 43.

**Revendications**

**1.** Composé de formule

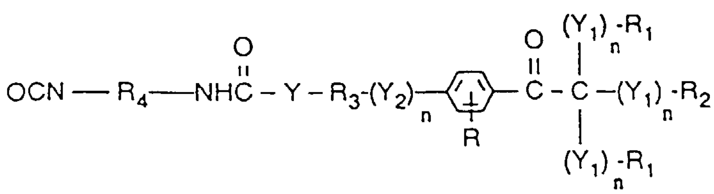

(I)

sous une forme pure, où Y représente O, NH ou $NR_6$ ; $Y_1$ représente 0 ; $Y_2$ représente -O-, -O-(O)C-, -C(O)-O- ou -O-C(O)-O- ; les n sont, indépendamment les uns des autres, égaux à 0 ou 1 ; R représente H, un alkyle en $C_1$-$C_{12}$, un alcoxy en $C_1$-$C_{12}$ ou un alkyle en $C_1$-$C_{12}$NH- ; les $R_1$ et $R_2$ représentent, indépendamment les uns des autres, H, un alkyle en $C_1$-$C_8$ linéaire ou ramifié, un hydroxyalkyle en $C_1$-$C_8$ ou un aryle en $C_6$-$C_{10}$ ou des deux groupes $R_1$-$(Y_1)_n$- représentent ensemble $-(CH_2)_x-$ ou les groupes $R_1$-$(Y_1)_n$- et $R_2$-$(Y_1)_n$- forment ensemble un reste de formule

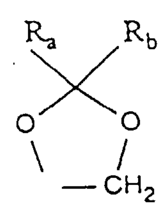

$R_3$ représente une liaison directe ou un alkylène en $C_1$-$C_8$ linéaire ou ramifié, non substitué ou substitué par -OH et/ou éventuellement interrompu par un ou plusieurs groupes -O-, -O-C(O)- ou -O-C(O)-O- ; $R_4$ représente un alkylène en $C_3$-$C_{18}$ ramifié, un arylène en $C_6$-$C_{10}$ substitué par un alkyle en $C_1$-$C_4$ ou par un alcoxy en $C_1$-$C_4$, un aralkylène en $C_7$-$C_{18}$ non substitué ou substitué par un alkyle en $C_1$-$C_4$ ou un alcoxy en $C_1$-$C_4$, un cycloalkylène en $C_3$-$C_8$ substitué par un alkyle en $C_1$-$C_4$ ou par un alcoxy en $C_1$-$C_4$, un cycloalkylène en $C_3$-$C_8$-$C_yH_{2y}$- non substitué ou substitué par un alkyle en $C_1$-$C_4$ ou par un alcoxy en $C_1$-$C_4$ ou un $-C_yH_{2y}$-(cycloalkylène en $C_3$-$C_8$)-

$C_yH_{2y}$- substitué par un alkyle en $C_1$-$C_4$ ou par un alcoxy en $C_1$-$C_4$ ; $R_6$ représente un alkyle en $C_1$-$C_6$ linéaire ou ramifié ; x est un nombre entier allant de 3 à 5 ; y est un nombre entier allant de 1 à 6 ; $R_a$ et $R_b$ représentent, indépendamment l'un de l'autre, H, un alkyle en $C_1$-$C_8$, un cycloalkyle en $C_3$-$C_8$, le benzyle ou le phényle ; sous réserve que n dans les groupes -$(Y_1)_n$-$R_1$ est égal à 0 lorsque $R_2$ représente H ; qu'au plus deux $Y_1$ dans les groupes -$(Y_1)_n$- représentent 0 et que n dans les autres groupes -$(Y_1)_n$- est égal à 0 ; que n dans le groupe -$(Y_2)_n$- est égal à 0, lorsque $R_3$ représente une liaison directe ; et que le reste $R_4$ confère aux deux groupes NCO une réactivité différente.

**2.** Composés selon la revendication 1, caractérisés en ce que Y représente 0.

**3.** Composés selon la revendication 1, caractérisés en ce que $Y_2$ représente 0.

**4.** Composés selon la revendication 1, caractérisés en ce que R est un alkyle en $C_1$-$C_6$ ou un alcoxy en $C_1$-$C_6$.

**5.** Composés selon la revendication 1, caractérisés en ce que R est un alkyle en $C_1$-$C_4$ ou un alcoxy en $C_1$-$C_4$.

**6.** Composés selon la revendication 1, caractérisés en ce que R représente H.

**7.** Composés selon la revendication 1, caractérisés en ce que les substituants alkyles et alcoxy sont le méthyle, l'éthyle, le méthoxy ou l'éthoxy.

**8.** Composés selon la revendication 1, caractérisés en ce que $R_1$, comme aryle, est un alkyle en $C_1$-$C_4$ linéaire.

**9.** Composés selon la revendication 8, caractérisés en ce que $R_1$ est le méthyle ou l'éthyle.

**10.** Composés selon la revendication 1, caractérisés en ce que $R_1$, comme aryle, représente le phényle.

**11.** Composés selon la revendication 1, caractérisés en ce que les deux groupes $R_1$-$(Y_1)_n$-représentent -$(CH_2)_x$-, x étant égal à 4 ou 5.

**12.** Composés selon la revendication 1, caractérisés en ce que $R_2$ représente H, le méthyle ou l'éthyle.

**13.** Composés selon la revendication 1, caractérisés en ce que $R_1$ représente l'éthyle ou le méthyle ou les deux groupes $R_1$-$(Y_1)_n$- représentent ensemble le pentaméthylène, n dans le groupe -$(Y_1)_n$-$R_2$ est égal à 0, $R_2$ représente le méthyle ou H et R représente H.

**14.** Composés selon la revendication 1, caractérisés en ce dans le groupe -$(Y_1)_n$-$R_2$ $Y_1$ représente 0, n est égal à 1 et $R_2$ représente H.

**15.** Composés selon la revendication 1, caractérisés en ce dans le groupe -$(Y_1)_n$-$R_2$ $Y_1$ représente 0, n est égal à 1 et $R_2$ représente H, et n dans les groupes $R_1$-$(Y_1)_n$- est égal à 0.

**16.** Composés selon la revendication 1, caractérisés en ce que $R_3$, comme alkylène, contient 1 à 4 atomes de C.

**17.** Composés selon la revendication 1, caractérisés en ce que $R_3$ représente l'éthylène ou $R_3$ est une liaison directe et n dans le groupe -$(Y_2)_n$- est égal à 0.

**18.** Composés selon la revendication 1, caractérisés en ce que $R_4$ représente un alkylène en $C_4$-$C_{10}$ ramifié.

**19.** Composés selon la revendication 16, caractérisés en ce que $R_4$ représente le 2,2-diméthyl-1,4-butylène, le 2,2-diméthyl-1,5-pentylène, le 2,2,3 ou 2,2,4-triméthyi-1,5-pentylène, le 2,2-diméthyl-1,6-hexylène, le 2,2,3- ou 2,2,4- ou 2,2,5-triméthyl-1,6-hexylène, le 2,2-diméthyl-1,7-heptylène, le 2,2,3- ou 2,2,4- ou 2,2,5- ou 2,2,6-triméthyl-1,7-heptylène, le 2,2-diméthyl-1,8-octylène, le 2,2,3- ou 2,2,4- ou 2,2,5- ou 2,2,6- ou 2,2,7-triméthyl-1,8-octylène.

**20.** Composés selon la revendication 1, caractérisés en ce que $R_4$, comme arylène, est un phénylène substitué en position ortho par rapport au groupe OCN.

**21.** Composés selon la revendication 1, caractérisés en ce que $R_4$, comme arylène, représente le 1-méthyl-2,4-phénylène, le 1,5-diméthyl-2,4-phénylène, le 1-méthoxy-2,4-phénylène ou le 1-méthyl-2,7-naphtylène.

**22.** Composés selon la revendication 1, caractérisés en ce que $R_4$, comme aralkylène, représente un phénylalkylène comportant 1 à 6 atomes de C dans le groupe alkylène.

**23.** Composés selon la revendication 1, caractérisés en ce que $R_4$, comme cycloalkylène, représente un cycloalkyle en $C_5$ ou un cycloalkyle en $C_6$ substitué par le méthyle.

**24.** Composés selon la revendication 23, caractérisés en ce que $R_4$ représente le 4-méthyl-1,3-cyclopentylène, le 4-méthyl-1,3-cyclohexylène, le 4,4-diméthyl-1,3-cyclohexylène, le 3-méthyl- ou 3,3-diméthyl-1,4-cyclohexylène, le 3,5-diméthyl-1,3-cyclohexylène ou le 2,4-diméthyl-1,4-cyclohexylène.

**25.** Composés selon la revendication 1, caractérisés en ce que $R_4$, comme cycloalkylène-$C_yH_{2y}$-, représente un cyclohexylène-$C_yH_{2y}$- substitué par 1 à 3 alkyles en $C_1$-$C_4$.

**26.** Composés selon la revendication 25, caractérisés en ce que le groupe -$C_yH_{2y}$- représente le méthylène.

**27.** Composés selon la revendication 1, caractérisés en ce que $R_4$, comme cycloalkylène-$C_yH_{2y}$-, représente le cyclopent-1-yl-3-méthylène, le 3-méthylcyclopent-1-yl-3-méthylène, le 3,4-diméthylcyclcpent-1-yl-3-méthylène, le 3,4,4-triméthylcyclopent-1-yl-3-méthylène, le cyclohex-1-yl-3-méthylène ou le cyclohex-1-yl-4-méthylène, le 3- ou 4- ou 5-méthylcyclohex-1-yl-3-méthylène ou le 3- ou 4- ou 5-méthylcyclohex-1-yl-4-méthylène, le 3,4- ou 3,5-diméthylcyclohex-1-yl-3-méthylène ou le 3,4- ou 3,5-diméthylcyclohex-1-yl-4-méthylène, le 3,4,5- ou 3,4,4- ou 3,5,5-triméthylcyclohex-1-yl-3-méthylène ou le 3,4,5- ou 3,4,4- ou 3,5,5-triméthylcyclohex-1-yl-4-méthylène.

**28.** Composés selon la revendication 1, caractérisés en ce que $R_4$, comme -$C_yH_{2y}$-cycloalkylène-$C_yH_{2y}$-, représente un -$C_yH_{2y}$-cycloalkylène-$C_yH_{2y}$- substitué par 1 à 3 alkyles en $C_1$-$C_4$.

**29.** Composés selon la revendication 28, caractérisés en ce que le groupe -$C_yH_{2y}$- représente le méthylène.

**30.** Composés selon la revendication 28, caractérisés en ce que $R_4$ représente le 3- ou 4- ou 5-méthylcyclohexane-1,3-diméthylène ou le 3- ou 4- ou 5-méthylcyclohexane-1,4-diméthylène, le 3,4- ou 3,5-diméthylcyclohexane-1,3-diméthylène ou le 3,4- ou 3,5-diméthylcyclohexane-1,4-diméthylène, le 3,4,5- ou 3,4,4- ou 3,5,5-triméthylcyclohexane-1,3-diméthylène ou le 3,4,5- ou 3,4,4- ou 3,5,5-triméthylcyclohexane-1,4-diméthylène.

**31.** Composés selon la revendication 1, caractérisés en ce que $R_4$ représente un alkylène ramifié en position a ou, en particulier, en position β par rapport au groupe OCN ou un reste hydrocarboné cyclique substitué en au moins une position a, comme il a été défini.

**32.** Composés selon la revendication 1, caractérisés en ce que les composés de formule I sont des composés où dans les groupes $R_1$-$(Y_1)_n$- n est égal à 0, Y, $Y_2$ et $Y_1$ dans le groupe $R_2$-$(Y_1)_n$- représente 0, n dans le groupe $R_2$-$(Y_1)_n$- est égal à 0 ou 1, $R_1$ représente un alkyle en $C_1$-$C_4$ ou le phényle ou les groupes $R_1$-$(Y_1)_n$-représentent ensemble le tétraméthylène ou le pentaméthylène, $R_2$ représente un alkyle en $C_1$-$C_4$ ou H, R représente l'hydrogène, n dans le groupe -$(Y_2)_n$- est égal à 0 ou 1, $R_3$ représente un alkylène en $C_2$-$C_4$ linéaire ou ramifié ou une liaison directe, n dans le groupe -$(Y_2)_n$- étant égal à 0, $R_4$ représente un alkylène en $C_5$-$C_{10}$ ramifié, un phénylène substitué par 1 à 3 groupes méthyles, le benzylène ou le benzylène substitué par 1 à 3 groupes méthyles, le cyclohexylène substitué par 1 à 3 groupes méthyls, un cyclohexyl-$C_yH_{2y}$-ou un cyclohexyl-$C_yH_{2y}$- substitué par 1 à 3 groupes méthyles ou un -$C_yH_{2y}$-cyclohexyl-$C_yH_{2y}$- et y est égal à 1 ou 2.

**33.** Composés selon la revendication 1, caractérisés en ce que les composés de formule I sont des composés où dans les groupes $R_1$-$(Y_1)_n$- et -$(Y_2)$- n est égal à 0, Y, $Y_2$ et $Y_1$ dans le groupe $R_2$-$(Y_1)_n$-représente 0, n dans le groupe $R_2$-$(Y_1)_n$- est égal à 0 ou 1, $R_1$ représente le méthyle ou le phényle ou les groupes $R_1$-$(Y_1)_n$- représentent ensemble le pentaméthylène, $R_2$ représente le méthyle ou H, R représente l'hydrogène, n dans le groupe -$(Y_2)_n$- est égal à 1 et $R_3$ représente l'éthylène ou n dans le groupe -$(Y_2)_n$- est égal à 0, $R_3$ représente une liaison directe et $R_4$ représente un alkylène en $C_6$-$C_{10}$ ramifié, le phénylène substitué par 1 à 3 groupes méthyles, le benzylène ou le benzylène substitué par 1 à 3 groupes méthyles, le cyclohexylène substitué par 1 à 3 groupes méthyles, un cyclohexyl-$CH_2$-ou un cyclohexyl-$CH_2$- substitué par 1 à 3 groupes méthyles.

**34.** Composés selon la revendication 1, caractérisés en ce qu'ils sont des composés de formules

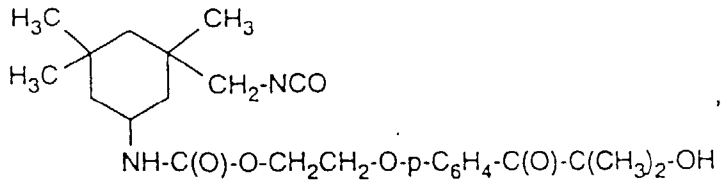

,

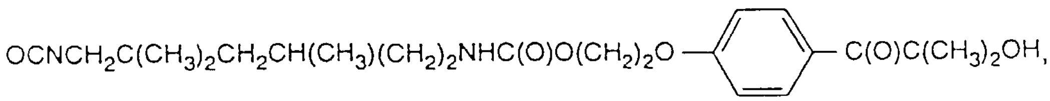

,

ou

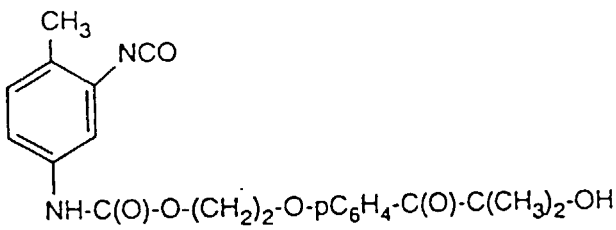

**35.** Procédé de préparation des composés de formule I, caractérisé en ce que l'on fait réagir un composé de formule II

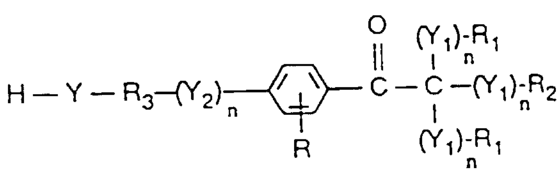

(II),

où Y, $Y_1$, $Y_2$, R, $R_1$, $R_2$, $R_3$ et n ont les significations indiquées dans la revendication 1, à une température allant jusqu'à 40°C dans un solvant organique inerte avec un diisocyanate de formule III ou avec un tel diisocyanate éventuellement monoprotégé

$$OCN\text{-}R4\text{-}NCO \qquad (III),$$

où $R_4$ a les significations indiquées dans la revendication 1.

**36.** Oligomères ou polymères comportant des groupes OH et/ou -NH- H actifs liés à la chaîne principale de l'oligomère ou du polymère éventuellement par un groupe pontal ou comportant des groupes -NH- H actifs liés à la chaîne principale de l'oligomère ou du polymère, dont les atomes d'hydrogène sont, en partie ou en totalité, substitués par des restes de formule IV

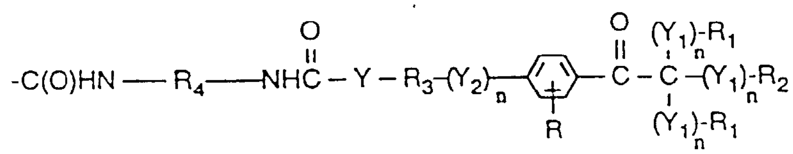 (IV),

où R, $R_1$, $R_2$, $R_3$, $R_4$, y, $Y_1$, $Y_2$ et n ont les significations indiquées dans la revendication 1, caractérisés en ce que les oligomères ou polymères sont (i) les cyclodextrines, l'amidon, l'acide hyaluronique, l'acide hyaluronique désacétylé, le chitosane, le trehalose, le cellobiose, le maltotriose, le maltohexaose, le chitohexaose, l'agarose, la chitine 50, l'amylose, le glycose, l'héparine, le xylane, la pectine, le galactane, le glycosaminoglycane, le dextrane, le dextrane aminé, la cellulose, les hydroxyalkylcelluloses, les carboxyalkylcelluloses, le fucoïdane, le sulfate de chondroïtine, les polysaccharides sulfatés, les mucopolysaccharides, la gélatine, la zeïne, le collagène, l'albumine, la globuline, la bilirubine, l'ovalbumine, la kératine, la fibronectine et la vitronectine, la pepsine, la trypsine et les lysozymes ; (ii) des oligo- et polysiloxanes comportant des groupes OH ou $NH_2$ dans les groupes terminaux ou les chaînes latérales, dont les atomes d'hydrogène sont substitués par un photoinitiateur selon la revendication 1 ; ou (iii) des oxydes de polyoxaalkylènes de formule XIII comportant des unités structurales de récurrence identiques ou différents $-[CH_2CH(R_{27})-O]-$

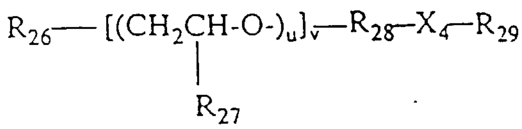 (XIII),

où $R_{26}$ représente le groupe $R_{29}$-$X_4$- ou est le reste de valence v d'un alcool ou d'un polyol comportant 1 à 20 atomes de C, $R_{27}$ représente H, un alkyle en $C_1$-$C_8$, de préférence un alkyle en $C_1$-$C_4$ et, en particulier, de préférence le méthyle, $R_{28}$ représente ensemble avec $X_4$ une liaison directe ou $R_{28}$ représente un alkylène en $C_2$-$C_6$, de préférence un alkylène en $C_3$-$C_6$ et, en particulier, de préférence le 1,3-propylène, $X_4$ représente -O- ou -NH-, $R_{29}$ représente un reste de formule VII, u est un nombre compris entre 3 et 10 000 et v est un nombre entier compris entre 1 et 6.

**37.** Oligomères ou polymères selon la revendication 36, caractérisés en ce que les oligomères présentent un poids moléculaire moyen compris entre 300 et 10 000 Daltons et les polymères présentent un poids moléculaire moyen supérieur à 10 000 et allant jusqu'à 1 000 000.

**38.** Oligomères ou polymères selon la revendication 32, caractérisé en ce que les oligomères ou polymères sont les cyclodextrines, l'amidon, l'acide hyaluronique, l'acide hyaluronique désacétylé, le chitosane, le tréhalose, le cellobiose, le maltotriose, le maltohexaose, le chitohexaose, l'agarose, la chitine 50, l'amylose, le glucane, l'héparine, le xylane, la pectine, le galactane, le glycosaminoglycane, le dextrane, le dextrane aminé, la cellulose, les hydroxyalkylcelluloses, les carboxyalkylcelluloses, le fucoïdane, le sulfate de chondroïtine, les polysaccharides sulfatés, les mucopolysaccharides, la gélatine, le collagène, l'albumine, la globuline, la bilirubine, l'ovalbumine, la kératine, la fibronectine ou la vitronectine, la pepsine, la trypsine et les lysozymes.

**39.** Oligomères ou polymères selon la revendication 38, caractérisés en ce qu'il s'agit de cyclodextrines comportant 6 à 8 unités structurales glucose formant un cycle ou des dérivés hydroxyalkyles ou aminoalkyles ou des dérivés substitués par le glucose ou maltose dont au moins une unité structurale correspond à la formule XVI

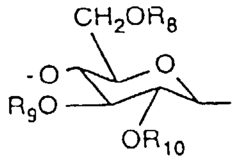

(XVI),

où $R_8$, $R_9$ et $R_{10}$ représentent, indépendamment les uns des autres, H, un alkyle en $C_1$-$C_4$, un acyle en $C_2$-$C_6$, un hydroxyalkyle en $C_1$-$C_4$ ou un aminoalkyle en $C_2$-$C_{10}$, et au moins l'un des restes $R_8$, $R_9$ et $R_{10}$ représente un reste de formule V

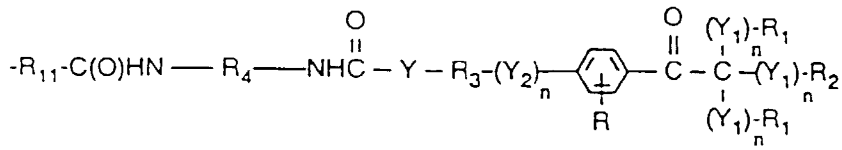

(V),

où R, $R_1$, $R_2$, $R_3$, $R_4$, y, $Y_1$, $Y_2$ et n ont les significations indiquées dans la revendication 1 et $R_{11}$ représente une liaison directe, un -(alkylène en $C_1$-$C_4$ O)- ou un -(alkylène en $C_2$-$C_{10}$ -NH)-.

**40.** Oligomères et polymères selon la revendication 36, caractérisés en ce que les oligomères ou polymères sont des oligo- ou polysiloxanes comportant des groupes OH ou $NH_2$ dans les groupes terminaux ou les chaînes latérales, dont les atomes d'hydrogène sont substitués par un photoinitiateur selon la revendication 1.

**41.** Oligomères et polymères selon la revendication 40, caractérisés en ce qu'il s'agit d'oligomères ou de polymères contenant

a) 5 à 100 % en mole d'éléments structuraux de formule VI

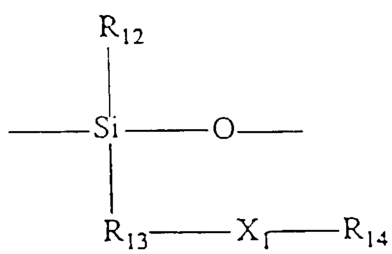

(VI),

et
b) 95 à 0 % en mole d'éléments structuraux de formule VIa

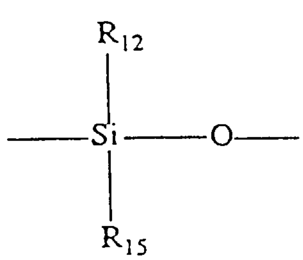

(VIa),

rapporté à l'oligomère ou au polymère, où $R_{12}$ représente un alkyle en $C_1$-$C_4$ éventuellement substitué par F,

le vinyle, l'allyle ou le phényle, de préférence le méthyle $R_{13}$ représente un alkylène en $C_2C_6$, de préférence le 1,3-propylène, $R_{15}$ a la signification de $R_{12}$ ou représente $-R_{13}-X_1-H$ ou $-R_{13}-X_1-R_{16}-H$, $X_1$ représentant -O- ou -NH-, et $R_{14}$ représente un reste de formule VII

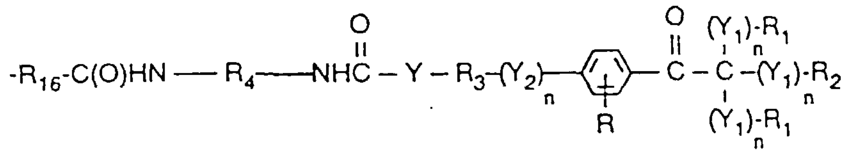

(VII),

où R, $R_1$, $R_2$, $R_3$, $R_4$, y, $Y_1$, $Y_2$ et n ont les significations indiquées dans la revendication 1 et $R_{16}$ représente une liaison directe ou un groupe $-C(O)-(CHOH)_r-CH_2-O-$, r étant égal à 0 ou est un nombre entier compris entre 1 et 4.

**42.** Oligomères ou polymères selon la revendication 40, caractérisés en ce qu'il s'agit de siloxanes oligomères ou polymères de formule VIII

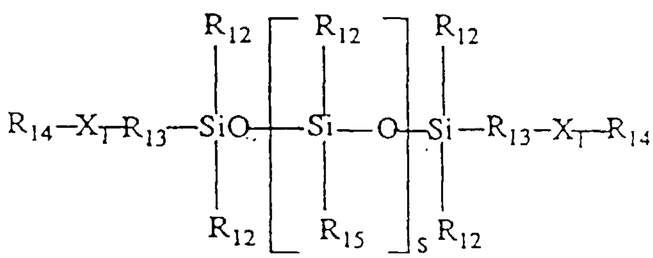

(VIII),

où $R_{12}$ représente un alkyle en $C_1$-$C_4$ éventuellement substitué par F, le vinyle, l'allyle ou le phényle, $R_{13}$ représente un alkylène en $C_2$-$C_6$, $R_{15}$ a la signification de $R_{12}$ ou représente $-R_{13}-X_1-H$ ou $-R_{13}-X_1-R_{16}-H$, $X_1$ représentant -O- ou -NH-, et $R_{14}$ représente un reste de formule VII

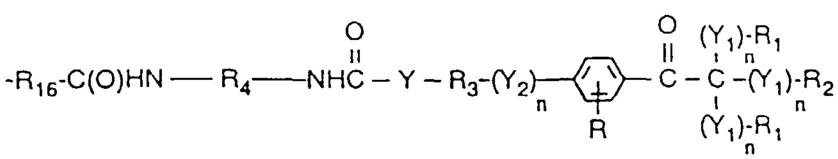

(VII),

où R, $R_1$, $R_2$, $R_3$, $R_4$, y, $Y_1$, $Y_2$ et n ont les significations indiquées dans la revendication 1 et $R_{16}$ représente une liaison directe ou un groupe $-C(O)-(CHOH)_r-CH_2-O-$, r étant égal à 0 ou est un nombre entier compris entre 1 et 4.

**43.** Oligomères ou polymères selon la revendication 36, caractérisés en ce qu'il s'agit d'oxydes de polyoxaalkylène de formule XIII comportant des unités structurales de récurrence identiques ou différents $-[CH_2CH(R_{27})-O]-$

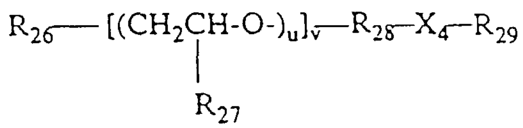

(XIII),

où $R_{26}$ représente le groupe $R_{29}$-$X_4$- ou est le reste de valence v d'un alcool ou d'un polyol comportant 1 à 20 atomes de C, $R_{27}$ représente H, un alkyle en $C_1$-$C_8$, de préférence un alkyle en $C_1$-$C_4$ et, en particulier, de préférence le méthyle, $R_{28}$ représente ensemble avec $X_4$ une liaison directe ou $R_{28}$ représente un alkylène en $C_2$-$C_6$, de préférence un alkylène en $C_3$-$C_6$ et, en particulier, de préférence le 1,3-propylène, $X_4$ représente -O- ou -NH-, $R_{29}$ représente un reste de formule VII, u est un nombre compris entre 3 et 10 000 et v est un nombre entier compris entre 1 et 6.

**44.** Matériau constitué (a) d'un substrat anorganique ou organique auquel est lié (b), comme photoinitiateur, au moins un composé de formule I tel que défini dans la revendication 1, solidement lié au substrat par des atomes d'oxygène, des atomes de soufre, des groupes N-alkyles en $C_1$-$C_6$ ou des groupes NH et les groupes isocyanates des photoinitiateurs.

**45.** Matériau selon la revendication 44, constitué d'un matériau de base organique, transparent, ledit matériau de base étant une pièce moulée ophtalmique, en particulier une lentille de contact.

**46.** Matériau selon la revendication 44, caractérisé en ce qu'il présente sur la couche du photoinitiateur en plus (c) une mince couche de polymère externe, obtenue par application d'une mince couche de substances à insaturation éthylénique photopolymérisables sur la surface du substrat munie des restes du photoinitiateur et par polymérisation de la couche de substances à insaturation éthylénique par rayonnement, de préférence par rayonnement UV.

**47.** Matériau selon la revendication 46, ledit matériau étant une lentille de contact.

**48.** Procédé pour modifier les surfaces de substrats anorganiques ou organiques contenant des groupes HO, HS, -NH-alkyle en $C_1$-$C_6$ ou des groupes -$NH_2$ H actifs comprenant les étapes

a) d'application sur le substrat d'une mince couche d'au moins un composé de formule I, comme photoinitiateur,
b) de fixation du photoinitiateur éventuellement en chauffant le matériau revêtu et de lavage du photoinitiateur en excès,
c) d'application d'une mince couche de substances à insaturation éthylénique photopolymérisables sur la surface du substrat munie des restes du photoinitiateur, et
d) de polymérisation de la couche de substances à insaturation éthylénique par rayonnement, de préférence par rayonnement UV.

**49.** Lentille de contact constituée (a) d'un matériau de base organique, transparent, comportant des groupes fonctionnels et (b) d'une mince couche sur la surface du matériau, constituée (b1) d'au moins un photoinitiateur de formule I et (b2) d'un polymère greffé, formé par photopolymérisation d'une oléfine non volatile ou difficilement volatile.

**50.** Lentille de contact contenant un oligomère ou un polymère comportant des groupes -OH et/ou -NH- H actifs liés à la chaîne principale de l'oligomère ou du polymère éventuellement par un groupe pontal ou comportant des groupes -NH- H actifs liés à la chaîne principale de l'oligomère ou du polymère, dont les atomes d'hydrogène sont, en partie ou en totalité, substitués par les restes de formule IV

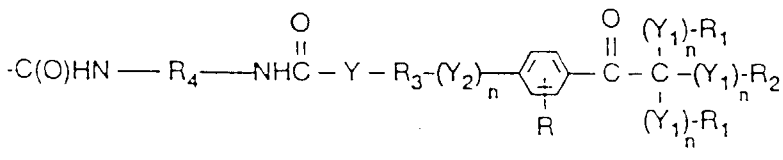

(IV) ,

où R, $R_1$, $R_2$, $R_3$, $R_4$, y, $Y_1$, $Y_2$ et n ont les significations indiquées dans la revendication 1, et une mince couche externe sur au moins une partie de la surface, constituée d'un polymère greffé, formé par photopolymérisation d'une oléfine non volatile ou difficilement volatile.

**51.** Lentille de contact selon la revendication 50, caractérisée en ce que les oligomères présentent un poids moléculaire moyen compris entre 300 et 10 000 Daltons et les polymères présentent un poids moléculaire moyen supérieur à 10 000 et allant jusqu'à 1 000 000 Daltons.

**52.** Lentille de contact selon la revendication 50, caractérisée en ce que l'oligomère ou le polymère est un oligomère ou un polymère naturel ou synthétique.

**53.** Lentille de contact selon la revendication 50, caractérisée en ce que l'oligomère ou le polymère est un oligomère ou un polymère selon la revendication 38.

**54.** Lentille de contact selon la revendication 50, caractérisée en ce que l'oligomère ou le polymère est un polymérisat saponifié d'esters ou d'éthers vinyliques (alcool polyvinylique) polydioléfines hydroxylées ; de l'acide polyacrylique, de l'acide polyméthacrylique, des polyacrylates, des polyméthacrylates, des polyacrylamides ou des polyméthacrylamides comportant des restes hydroxyalkyles ou aminoalkyles dans le groupe ester ou le groupe amide ; des polysiloxanes comportant des groupes hydroxyalkyles ou des groupes aminoalkyles ; des polyéthers d'époxydes ou de composés glycidyliques et de diols ; des phénols polyvinyliques ou des copolymères de phénol vinylique et de comonomères oléfiniques ; un copolymérisat d'au moins un monomère provenant du groupe constitué par l'alcool vinylique, les acrylates contenant des hydroxyalkyles ou aminoalkyles, les méthacrylates, l'acrylamide, le méthacrylamide, l'acide acrylique, l'acide méthacrylique, les dioléfines hydroxylées avec des comonomères à insaturation éthylénique ; ou des polyoxaalkylènes comportant des groupes OH ou aminoalkyloxy terminaux.

**55.** Lentille de contact selon la revendication 50, caractérisée en ce que l'oligomère ou le polymère est un oligomère ou un polymère selon la revendication 39.

**56.** Lentille de contact selon la revendication 50, caractérisée en ce que l'oligomère ou le polymère est un oligomère ou un polymère selon la revendication 40.

**57.** Lentille de contact selon la revendication 50, caractérisée en ce que l'oligomère ou le polymère est un oligomère ou un polymère selon la revendication 41.

**58.** Lentille de contact selon la revendication 50, caractérisée en ce que l'oligomère ou le polymère est un oligomère ou un polymère selon la revendication 42.

**59.** Lentille de contact selon la revendication 50, caractérisée en ce que l'oligomère ou le polymère est un oligomère ou un polymère contenant

a) 5 à 100 % en mole d'unités structurales de formule IX

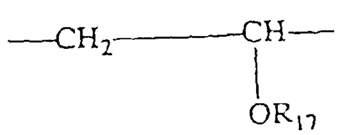

(IX) ,

b) 95 à 0 % en mole d'unités structurales de formule X

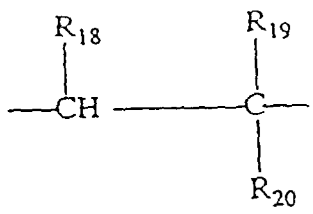

(X),

où $R_{17}$ représente un reste de formule V

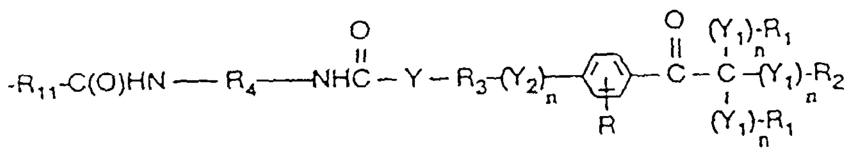

(V),

où R, $R_1$, $R_2$, $R_3$, $R_4$, y, $Y_1$, $Y_2$ et n ont les significations indiquées dans la revendication 1 et $R_{11}$ représente une liaison directe, un -(alkylène en $C_1$-$C_4$-O)- ou un -(alkylène en $C_2$-$C_{10}$-NH) ; $R_{18}$ représente H, un alkyle en $C_1$-$C_6$, -$COOR_{21}$ ou -$COO^-$, $R_{19}$ représente H, F, Cl, CN ou un alkyle en $C_1$-$C_6$ et $R_{20}$ représente H, OH, $R_{11}$-H, F, Cl, CN, $R_{21}$-O-, un alkyle en $C_1$-$C_{12}$, -$COO^-$, -$COOR_{21}$, -OCO-$R_{21}$, le méthylphényle ou le phényle, $R_{21}$ représentant un alkyle en $C_1$-$C_{18}$, un cycloalkyle en $C_5$-$C_7$, un (alkyle en $C_1$-$C_{12}$) cycloalkyle en $C_5$-$C_7$, le phényle, un (alkyle en $C_1$-$C_{12}$)phényle, le benzyle ou un (alkyle en $C_1$-$C_{12}$)benzyle.

**60.** Lentille de contact selon la revendication 54, caractérisée en ce que l'oligomère ou le polymère est un oligomère ou un polymère contenant

a) 5 à 100 % en mole d'unités structurales de formule XI.

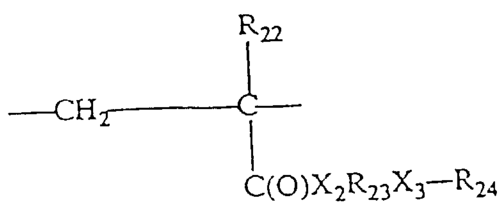

(XI),

et 95 à 0 % en mole d'unités structurales de formule XII

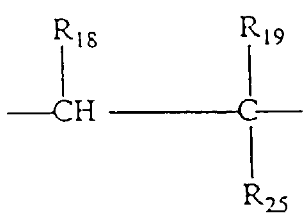

(XII),

où R22 représente H ou le méthyle, $X_2$ et $X_3$ représentent, indépendamment l'un de l'autre, -O- ou -NH-, $R_{23}$ représente -$(CH_2)$c et c est un nombre entier compris entre 2 et 12, de préférence, entre 2 et 6, $R_{24}$ représente un reste de formule VII, $R_{18}$ et $R_{19}$ ont les significations données dans la revendication 59 et $R_{25}$ a la même

signification que $R_{20}$ ou représente $-C(O)X_2R_{23}X_3H$.

**61.** Lentille de contact selon la revendication 50, caractérisée en ce que l'oligomère ou le polymère est un oligomère ou un polymère selon la revendication 43.